(19) Europäisches Patentamt
European Patent Office
Office européen des brevets

(11) EP 3 216 863 A1

(12) EUROPEAN PATENT APPLICATION

(43) Date of publication:
**13.09.2017 Bulletin 2017/37**

(51) Int Cl.:
***C12N 9/10*** *(2006.01)* ***C07B 53/00*** *(2006.01)*
***C07C 229/08*** *(2006.01)*

(21) Application number: **17162642.7**

(22) Date of filing: **25.03.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.03.2013 US 201361805786 P**

(62) Document number(s) of the earlier application(s) in accordance with Art. 76 EPC:
**14717080.7 / 2 978 748**

(71) Applicant: **PFIZER IRELAND PHARMACEUTICALS**
**Ringaskiddy**
**Cork (IE)**

(72) Inventors:
- **DEBARGE, Sebastien**
  **County Cork (IE)**
- **ERDMAN, David, Thomas**
  **Kalamazoo, MI 49001 (US)**
- **O'NEILL, Padraig, Mary**
  **County Cork (IE)**
- **KUMAR, Rajesh**
  **Groton, CT 06340 (US)**
- **KARMILOWICZ, Michael John**
  **Groton, CT 06340 (US)**

(74) Representative: **Pfizer**
**European Patent Department**
**23-25 avenue du Docteur Lannelongue**
**75668 Paris Cedex 14 (FR)**

Remarks:
This application was filed on 23-03-2017 as a divisional application to the application mentioned under INID code 62.

(54) **PROCESS AND INTERMEDIATES FOR THE PREPARATION OF PREGABALIN**

(57) The invention further provides improved methods for the conversion of the compound of formula **($I^A$)** into pregabalin.

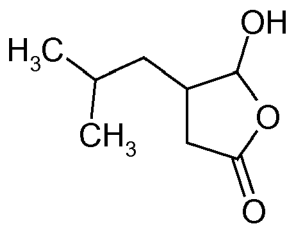

($I^A$)

EP 3 216 863 A1

## Description

### Field of the Invention

The present invention relates to the manufacture of 5-hydroxy-4-(2-methylpropyl)-3,4-dihydro-5*H*-2-furanone, and derivatives thereof, for use in the manufacture of (S)-(+)-3-aminomethyl-5-methylhexanoic acid (Pregabalin). The invention further relates to an improved process for the conversion of 5-hydroxy-4-(2-methylpropyl)-3,4-dihydro-5*H*-2-furanone into Pregabalin. Pregabalin is a $\gamma$-amino acid that exhibits binding affinity to the human $\alpha_2\delta$ calcium channel subunit.

### Background to the Invention

Pregabalin, or (*S*)-(+)-3-aminomethyl-5-methyl-hexanoic acid ((S)-**II**),

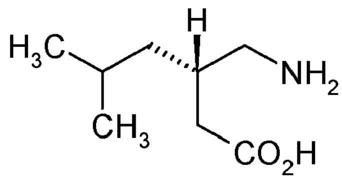

Pregabalin ((*S*)-**II**)

is the active agent in Lyrica®, which is approved for the treatment of epilepsy, neuropathic pain, fibromyalgia and generalized anxiety disorder. It exhibits anti-seizure activity, as discussed in U.S. Patent US 5,563,175, and anti-nociceptive activity, as discussed in U.S. Patent US 6,001,876. It is hypothesised that the pharmacological activity of Pregabalin (**II**) is the result of binding to the alpha-2-delta ($\alpha_2\delta$) subunit of a calcium channel. Pregabalin (**II**) is also described as having utility in other conditions, such as physiological conditions associated with psychomotor stimulants, inflammation, gastrointestinal damage, alcoholism, insomnia, and various psychiatric disorders, including anxiety, depression, mania, and bipolar disorder.

A number of methods for manufacturing Pregabalin have been disclosed. 5-Hydroxy-4-(2-methylpropyl)-3,4-dihydro-5*H*-2-furanone (**I^A^**) has been identified as a useful precursor.

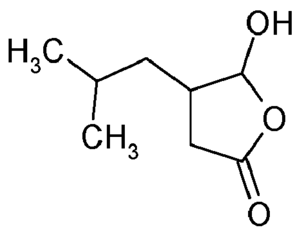

(**I$^A$**)

It will be appreciated that compound (**I$^A$**), in common with a number of the other compounds discussed herein, can be considered to be the cyclized isomer of a 4-oxobutanoic acid derivative. Derivatives of 4-oxobutanoic acid can exist as either the open-chain form, or as the cyclic form.

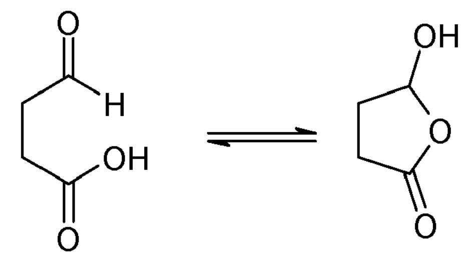

"Open chain" isomer

"Cyclic" isomer

These two isomeric forms may co-exist in equilibrium, and the relative contributions of the two forms will depend on the precise chemical nature of the species.

International Patent Application PCT/US2008/004699 (published as WO2008/127646A2) proposes the conversion of 5-hydroxy-4-(2-methylpropyl)-3,4-dihydro-5*H*-2-furanone (**I**$^A$) or an ester of the ring-opened isomeric form (**XIII**, wherein $R^A$ is alkyl) to (**II**) using a chemical or an enzyme-mediated reductive amination. It is suggested that the use of a transaminase enzyme will provide selectively the preferred ((*S*)-**II**) enantiomer.

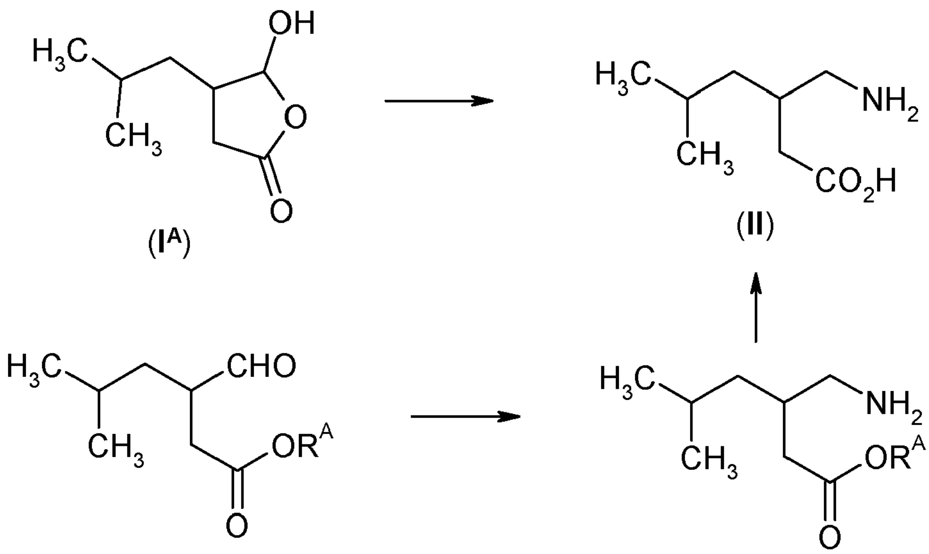

(**I**$^A$)

(**II**)

(**XIII**)

The ester (**XIII**) is prepared from 4-methylpentanal (**III**) by alkylation with an appropriate haloacetate in the presence of diisobutylamine. The precursor (**I**$^A$) is made either by ester hydrolysis of ester (**XIII**) or by condensation of 4-methylpentanal (**III**) with glyoxylic acid (**IV**) and subsequent reduction of the double bond. The use of an enzyme-mediated reduction is also suggested as a way of introducing the desired stereochemistry.

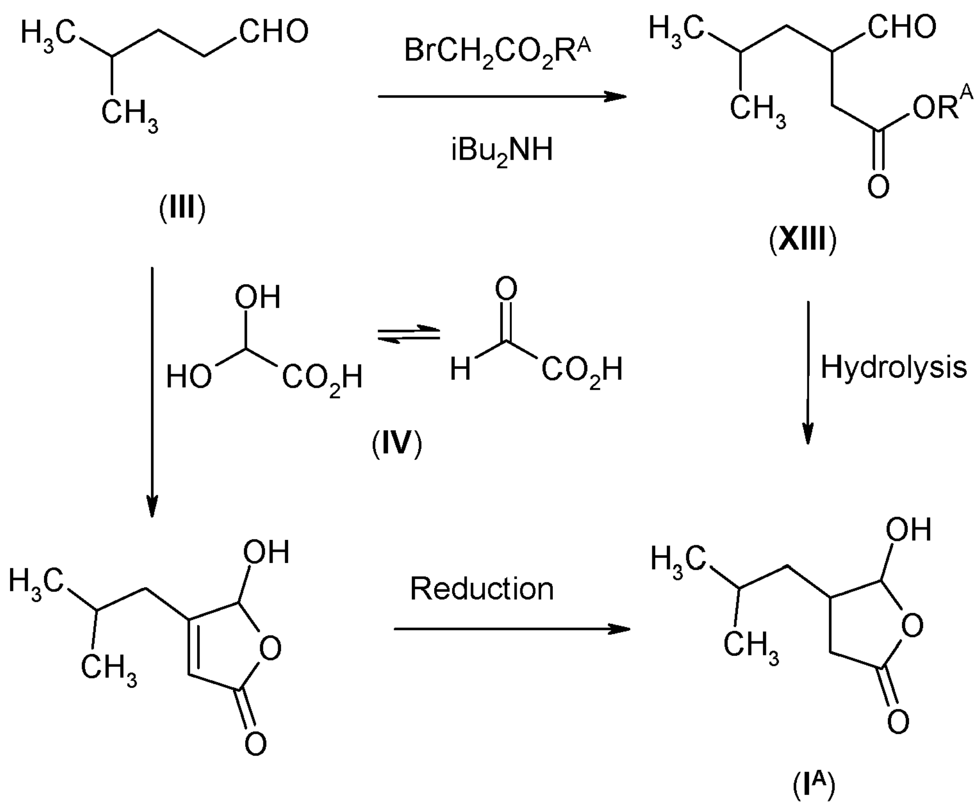


(III) (XIII) (IV) ($I^A$)

International Patent Application PCT/IN2010/000140 (published as WO2011/086565) discloses a related process. The condensation product of 4-methylpentanal (**III**) and glyoxylic acid (**IV**) is reacted with a chiral amine such as $\alpha$-methylbenzylamine to give a pyrrolone (**V**). Hydrogenation gives some degree of stereoselectivity, and deprotection gives Pregabalin (**II**) in chiral form.

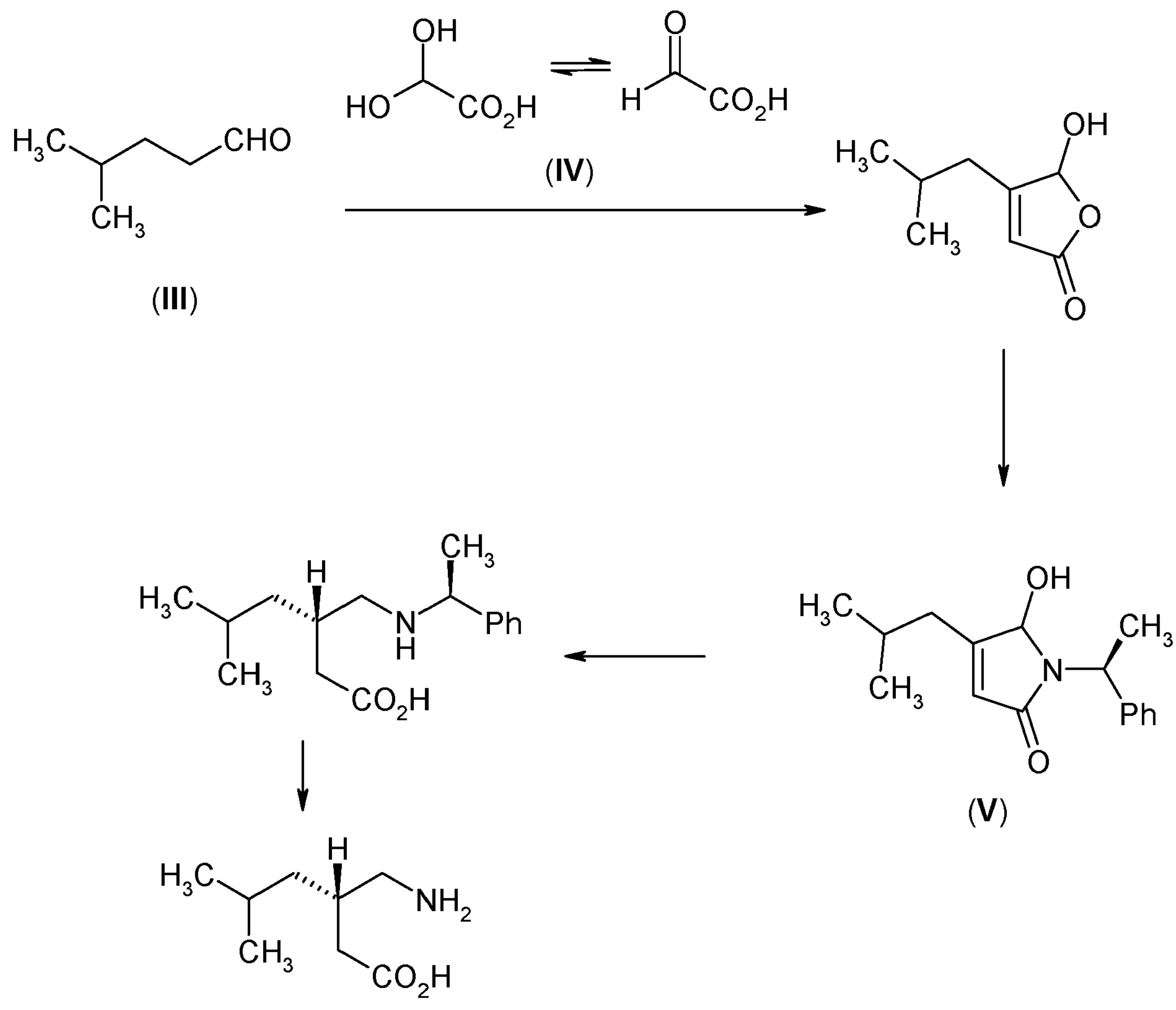

(III) (IV) (V)

((*S*)-II)

Still further improved syntheses of Pregabalin (**II**) are sought. It is especially desirable to provide a process which is cost effective and safe. In particular, it is important to provide a synthesis of Pregabalin (**II**) which can be carried out on a commercial scale, which uses readily available, inexpensive and safe starting materials and reagents, and which avoids the need for difficult separations.

<u>Summary of the Invention</u>

The present invention provides improved methods for the manufacture of 5-hydroxy-4-(2-methylpropyl)-3,4-dihydro-5*H*-2-furanone ($I^A$), and intermediates that are useful in these improved methods.
In a first aspect **A1,** the invention provides a compound according to formula (**VI**)

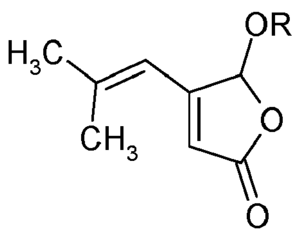

(**VI**)

In a first embodiment **A1E1,** the invention provides a compound according to formula (**VI**)

wherein R is selected from:

hydrogen,
$(C_1-C_6)$alkyl,
$(C_1-C_6)$haloalkyl,
$(C_1-C_3)$alkoxy$(C_2-C_6)$alkyl,
$(C_2-C_6)$alkenyl,
$(C_3-C_{10})$cycloalkyl, which may optionally be substituted with 1, 2 or 3 groups independently selected from halo, $(C_1-C_3)$alkyl, and $(C_1-C_3)$alkyloxy,
$(C_3-C_{10})$cycloalkyl$(C_1-C_6)$alkyl, wherein the cycloalkyl group may optionally be substituted with 1, 2 or 3 groups independently selected from halo, $(C_1-C_3)$alkyl, and $(C_1-C_3)$alkyloxy,
aryl, which may optionally be substituted with 1, 2 or 3 groups independently selected from halo, $(C_1-C_3)$alkyl, and $(C_1-C_3)$alkyloxy,
aryl$(C_1-C_6)$alkyl, wherein the aryl group may optionally be substituted with 1, 2 or 3 groups independently selected from halo, $(C_1-C_3)$alkyl, and $(C_1-C_3)$alkyloxy,
$R^1$-C(O)-, and
$R^2$-$SO_2$-;

$R^1$ is selected from:

hydrogen,
$(C_1-C_6)$alkyl,
$(C_1-C_6)$haloalkyl,
$(C_1-C_3)$alkoxy$(C_2-C_6)$alkyl,
$(C_2-C_6)$alkenyl,
$(C_3-C_{10})$cycloalkyl, which may optionally be substituted with 1, 2 or 3 groups independently selected from halo, $(C_1-C_3)$alkyl, and $(C_1-C_3)$alkyloxy,
$(C_3-C_{10})$cycloalkyl$(C_1-C_6)$alkyl, wherein the cycloalkyl group may optionally be substituted with 1, 2 or 3 groups independently selected from halo, $(C_1-C_3)$alkyl, and $(C_1-C_3)$alkyloxy,
aryl, which may optionally be substituted with 1, 2 or 3 groups independently selected from halo, $(C_1-C_3)$alkyl, and $(C_1-C_3)$alkyloxy, and
aryl$(C_1-C_6)$alkyl, wherein the aryl group may optionally be substituted with 1, 2 or 3 groups independently selected from halo, $(C_1-C_3)$alkyl, and $(C_1-C_3)$alkyloxy,

and $R^2$ is selected from:

$(C_1-C_6)$alkyl,
$(C_1-C_6)$haloalkyl,
$(C_1-C_3)$alkoxy$(C_2-C_6)$alkyl,
$(C_2-C_6)$alkenyl,
$(C_3-C_{10})$cycloalkyl, which may optionally be substituted with 1, 2 or 3 groups independently selected from halo, $(C_1-C_3)$alkyl, and $(C_1-C_3)$alkyloxy,
$(C_3-C_{10})$cycloalkyl$(C_1-C_6)$alkyl, wherein the cycloalkyl group may optionally be substituted with 1, 2 or 3 groups independently selected from halo, $(C_1-C_3)$alkyl, and $(C_1-C_3)$alkyloxy,
aryl, which may optionally be substituted with 1, 2 or 3 groups independently selected from halo, $(C_1-C_3)$alkyl, and $(C_1-C_3)$alkyloxy, and
aryl$(C_1-C_6)$alkyl, wherein the aryl group may optionally be substituted with 1, 2 or 3 groups independently selected from halo, $(C_1-C_3)$alkyl, and $(C_1-C_3)$alkyloxy.

In a further embodiment **A1 E2,** the invention provides a compound according to embodiment **A1 E1** wherein R is hydrogen such that the compound of formula (**VI**) is 5-hydroxy-4-(2-methyl-1-propenyl)-5H-2-furanone according to formula (**VIA**).

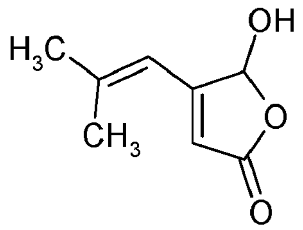

(**VI^A**)

In a further embodiment **A1E3,** the invention provides a compound according to embodiment **A1E1** of formula (**VI^B**)

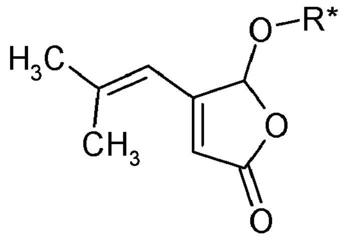

(**VI^B**)

wherein R* is a chiral ($C_5$-$C_{15}$) hydrocarbon group.

In a further embodiment **A1E4,** the invention provides a compound according to embodiment **A1E3** wherein R* is selected from (*R*)- or (*S*)-$\alpha$-methylbenzyl, (*R*)- or (*S*)-1-(1-naphthyl)ethyl, (*R*)- or (*S*)-1-(2-naphthyl)ethyl, menthyl and bornyl, such that the compound of formula (**VI**) is the compound of formula (**VI^C**) - (**VI^K**).

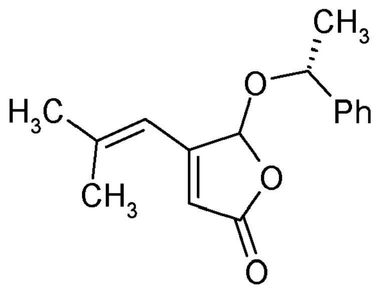

(**VI^C**)

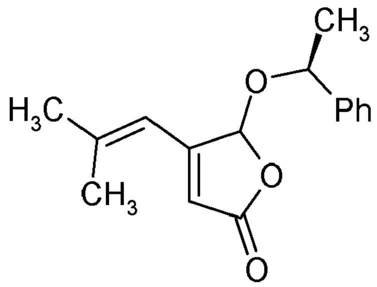

(**VI^D**)

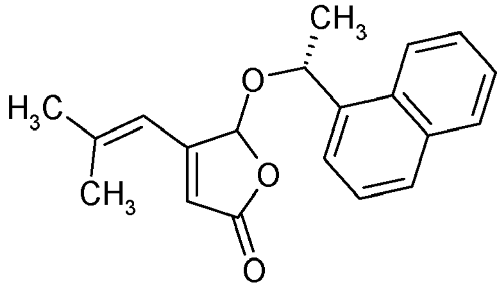

(**VI^E**)

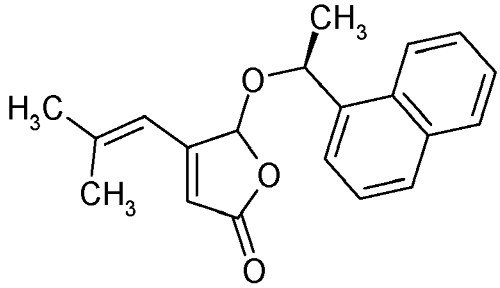

(**VI^F**)

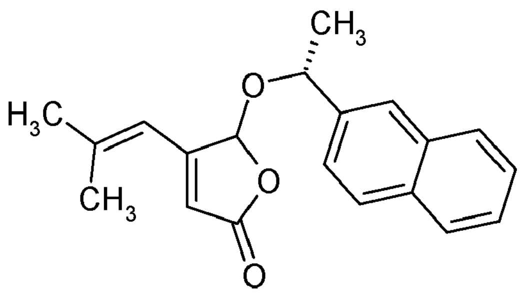

(**VI^G**)

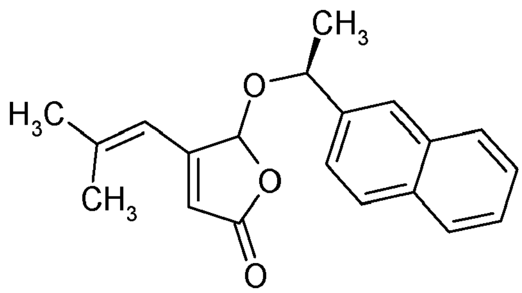

(**VI^H**)

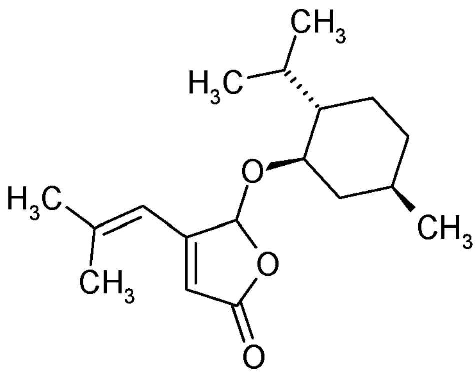

(**VI^J**)

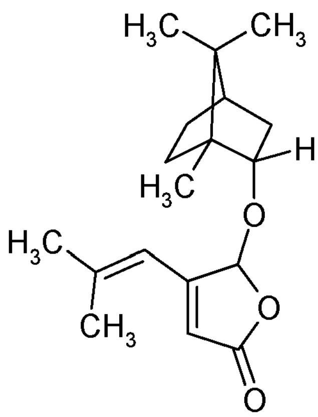

(**VI^K**)

In a further embodiment **A1E5,** the invention provides a compound according to embodiment **A1E1** wherein R is $R^1$-C(O)- or $R^2$-$SO_2$- and $R^1$ and $R^2$ are chiral ($C_5$-$C_{15}$) hydrocarbon groups.

In another aspect **A2,** the invention provides a compound of formula (**IX**)

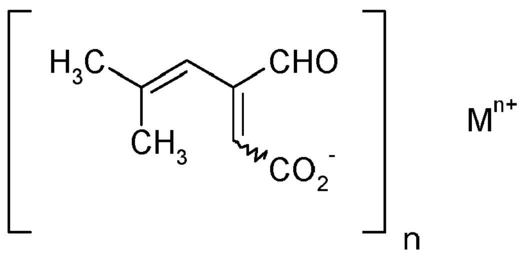

(**IX**)

The compound of formula (**IX**) may exist as either the (*E*)- or (*Z*)-geometric isomer, or as a mixture of the two geometric isomers.

In a first embodiment **A2E1,** the invention provides a compound of formula **(IX)** wherein: n is 1 and $M^+$ is selected from $Li^+$, $Na^+$, $K^+$, $Rb^+$, $NH_4^+$, (($C_1$-$C_3$)alkyl)$NH_3^+$, (($C_1$-$C_3$)alkyl)$_2NH_2^+$, (($C_1$-$C_3$)alkyl)$_3NH^+$ and (($C_1$-$C_3$)alkyl)$_4N^+$; or n is 2 and $M^{2+}$ is selected from $Mg^{2+}$, $Ca^{2+}$ and $Zn^{2+}$.

In a further embodiment **A2E2,** the invention provides a compound according to embodiment **A2E1** wherein n is 1 and $M^+$ is selected from $NH_4^+$ and (($C_1$-$C_3$)alkyl)$NH_3^+$.

In a further embodiment **A2E3,** the invention provides a compound according to embodiment **A2E1** wherein n is 1 and $M^+$ is selected from $Li^+$, $Na^+$ and $K^+$.

In another aspect **A3,** the invention provides a compound of formula (**VII**).

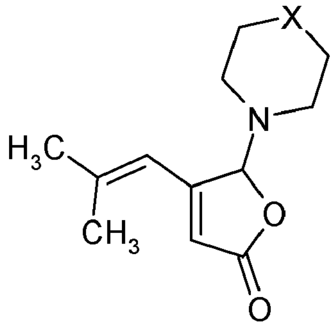

(**VII**)

In a first embodiment **A3E1,** the invention provides a compound of formula (**VII**) wherein -X- represents a single bond, $-CH_2-$, -O-; -NH-,-N(($C_1$-$C_3$)alkyl)-, -N(benzyl)-, or

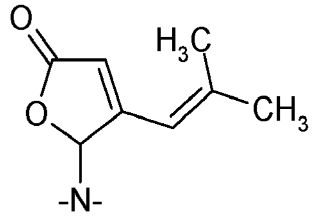

In a further embodiment **A3E2,** the invention provides a compound according to embodiment **A3E1** selected from:

4-(2-methylpropenyl)-5-pyrrolidin-1-yl-5*H*-furan-2-one;
4-(2-methylpropenyl)-5-piperidin-1-yl-5*H*-furan-2-one;
4-(2-methylpropenyl)-5-morpholin-4-yl-5*H*-furan-2-one; and
1,4-bis-(4-(2-methylpropenyl)-5*H*-furan-2-on-5-yl)piperazine.

In another aspect **A4,** the invention provides a compound of formula (**VIII**).

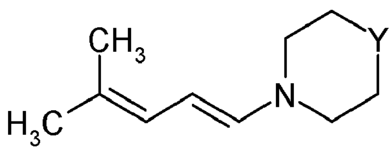

(**VIII**)

In a first embodiment **A4E1,** the invention provides a compound of formula (**VIII**) wherein -Y- represents a single bond, $-CH_2-$, -O-; -NH-, -N(($C_1$-$C_3$)alkyl)-, -N(benzyl)-, or

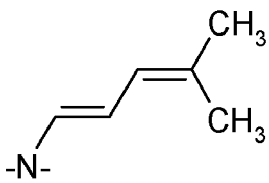

In a further embodiment **A4E2,** the invention provides a compound according to embodiment **A4E1** selected from:

4-(4-methyl-1,3-pentadien-1-yl)morpholine;

1-(4-methyl-1,3-pentadien-1-yl)-piperazine,
1-(4-methyl-1,3-pentadien-1-yl)-4-methylpiperazine,
4-ethyl-1-(4-methyl-1,3-pentadien-1-yl)-piperazine,
4-benzyl-1-(4-methyl-1,3-pentadien-1-yl)-piperazine, and
1,4-*bis*-(4-methyl-1,3-pentadien-1-yl)piperazine.

In another aspect **A5,** the invention provides a process for the manufacture of the compound of formula (**VI^A^**) comprising the step of treating a compound of formula (**VII**) with water in the presence of an acid catalyst.

In a first embodiment **A5E1,** the invention provides a process for the manufacture of the compound of formula (**VI^A^**), comprising the steps of:

(a) preparing a compound of formula (**VII**)

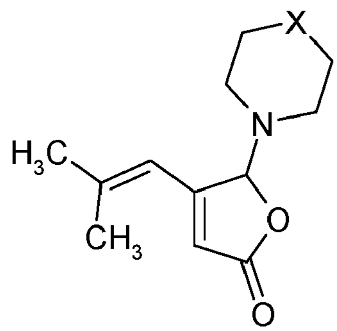

(**VII**)

wherein -X- represents a single bond, $-CH_2-$, -O-, -NH-, $-N((C_1-C_3)alkyl)-$, -N(benzyl)-, or

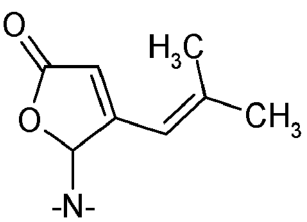

and

(b) treating the compound of formula (**VII**) with water in the presence of an acid catalyst.

In a further embodiment **A5E2,** the invention provides a process according to embodiment **A5E1** wherein the compound of formula (**VII**) from step (a) is isolated prior to hydrolysis step (b).

In a further embodiment **A5E3,** the invention provides a process according to embodiment **A5E1** wherein hydrolysis step (b) is carried out directly following step (a) such that the compound of formula (**VII**) or (**VII^B^**) is not isolated prior to hydrolysis step (b).

In a further embodiment **A5E4,** the invention provides a process according to embodiments **A5E1, A5E2** or **A5E3** wherein the compound of formula (**VII**) is prepared by treating a compound of formula (**VIII**)

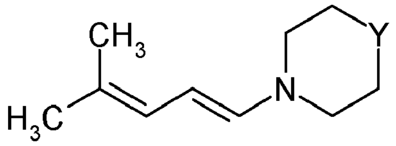

(**VIII**)

wherein -Y- represents a single bond, $-CH_2-$, -O-; -NH-, $-N((C_1-C_3)alkyl)-$, -N(benzyl)-, or

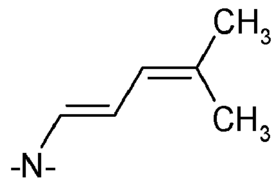

with glyoxylic acid or its hydrate.

In another aspect **A6,** the invention provides a process for the manufacture of a compound of formula (**VI**) wherein R is other than hydrogen, $R^1$-C(O)-, and $R^2-SO_2-$, comprising the step of treating a compound of formula (**VI^A^**) with an alcohol R-OH in the presence of an acid catalyst.

In an embodiment **A6E1,** the invention provides a process for the manufacture of a compound of formula (**VI**) wherein R is other than hydrogen, $R^1$-C(O)-, and $R^2-SO_2-$, comprising the steps of:

(a) preparing a compound of formula (**$VI^A$**) using a process according to any of embodiments **A5E1, A5E2, A5E3** and **A5E4** as defined above; and
(b) treating the compound of formula (**$VI^A$**) with an alcohol R-OH in the presence of an acid catalyst.

In another aspect **A7,** the invention provides a process for the manufacture of a compound of formula (**VI**) wherein R is other than hydrogen, $R^1$-C(O)-, and $R^2-SO_2-$, comprising the step of treating acompound of formula (**VII**) with an alcohol R-OH in the presence of stoichiometric acid.

In an embodiment **A7E1,** the invention provides a process for the manufacture of a compound of formula (**VI**) wherein R is other than hydrogen, $R^1$-C(O)-, and $R^2-SO_2-$, comprising the steps of:

(a) preparing a compound of formula (**VII**); and
(b) treating the compound of formula (**VII**) with an alcohol R-OH in the presence of stoichiometric acid.

In another aspect **A8,** the invention provides a process for the manufacture of a compound of formula (**VI**) wherein R is $R^1$-C(O)-, comprising the step of treating a compound of formula (**$VI^A$**) with an acid chloride $R^1$-C(O)-Cl or acid anhydride $(R^1-C(O))_2O$.

In an embodiment **A8E1,** the invention provides a process for the manufacture of a compound of formula (**VI**) wherein R is $R^1$-C(O)-, comprising the steps of:

(a) preparing a compound of formula (**VI**$^A$) using a process according to any of embodiments **A5E1, A5E2, A5E3** and **A5E4** as defined above; and
(b) treating the compound of formula (**$VI^A$**) with an acid chloride $R^1$-C(O)-Cl or acid anhydride $(R^1-C(O))_2O$.

In another aspect **A9,** the invention provides a process for the manufacture of a compound of formula (**VI**) wherein R is $R^2-SO_2-$, comprising the step of treating a compound of formula (**$VI^A$**) with a sulfonyl chloride $R^2-SO_2-Cl$.

In an embodiment **A9E1,** the invention provides a process for the manufacture of a compound of formula (**VI**) wherein R is $R^2-SO_2-$, comprising the steps of:

(a) preparing a compound of formula (VIA) using a process according to any of embodiments **A5E1, A5E2, A5E3** and **A5E4** as defined above; and
(b) treating the compound of formula (**$VI^A$**) with a sulfonyl chloride $R^2-SO_2-Cl$.

In another aspect **A10,** the invention provides a process for the manufacture of an enamine derivative of 4-methyl-2-pentenal.

In a first embodiment **A10E1,** the invention provides a process for the manufacture of an enamine derivative of 4-methyl-2-pentenal comprising reacting acetaldehyde with isobutyraldehyde in the presence of a suitable amine.

In a further embodiment **A10E2,** the invention provides a process according to embodiment **A10E1** wherein the suitable amine is a secondary amine.

In a further embodiment **A10E3,** the invention provides a process according to embodiment **A10E2** wherein the secondary amine is selected from: $((C_1-C_4)alkyl)_2NH$, pyrrolidine, piperidine, morpholine, piperazine, N-methylpiperazine, N-ethylpiperazine and N-benzylpiperazine.

In a further embodiment **A10E4,** the invention provides a process according to embodiment **A10E3** wherein

the secondary amine is selected from pyrrolidine, piperidine, morpholine, and piperazine.

In a further embodiment **A10E5,** the invention provides a process according to any of embodiments **A10E1, A10E2, A10E3** and **A10E4** wherein the reaction is performed in the presence of an acid catalyst.

In a further embodiment **A10E6,** the invention provides a process according to any of embodiments **A10E1, A10E2, A10E3, A10E4** and **A10E5** wherein the isobutyraldehyde is combined with the suitable amine before addition of the acetaldehyde.

In a further embodiment **A10E6,** the invention provides a process according to any of embodiments **A10E1, A10E2, A10E3, A10E4** and **A10E5** wherein the isobutyraldehyde and acetaldehyde are added to the reaction vessel simultaneously.

In another aspect **A11,** the invention provides a process for the manufacture of 3-aminomethyl-5-methylhexanoic acid (**II**).

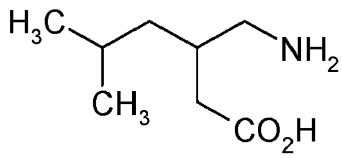

(**II**)

In a first embodiment **A11E1,** the invention provides a process for the manufacture of 3-aminomethyl-5-methylhexanoic acid (**II**) or a pharmaceutically acceptable salt thereof, comprising the steps:

(a) preparing 5-hydroxy-4-(2-methyl-1-propenyl)-5*H*-2-furanone (**VI$^{A}$**)

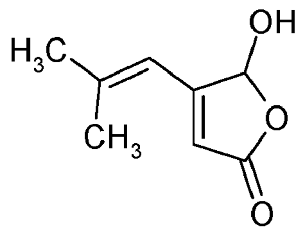

(**VI$^{A}$**)

(b) converting said 5-hydroxy-4-(2-methyl-1-propenyl)-5*H*-2-furanone (VIA) into 5-hydroxy-4-(2-methylpropyl)-3,4-dihydro-5*H*-2-furanone (**I$^{A}$**)

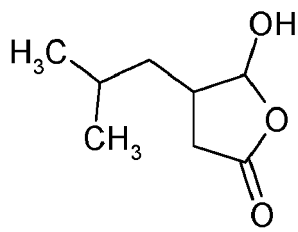

(**I$^{A}$**)

and

(c) converting said 5-hydroxy-4-(2-methylpropyl)-3,4-dihydro-5*H*-2-furanone (**I$^{A}$**) into 3-aminomethyl-5-methylhexanoic acid (**II**).

In a further embodiment **A11E2,** the invention provides a process according to embodiment **A11E1** wherein the 3-aminomethyl-5-methylhexanoic acid (**II**) is (S)-3-aminomethyl-5-methylhexanoic acid ((S)-**II**)

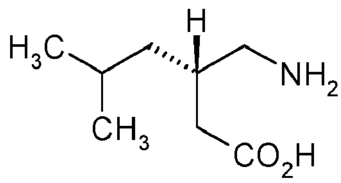

((*S*)-**II**)

wherein said (S)-3-aminomethyl-5-methylhexanoic acid has an enantiomeric excess of at least 80%.

In a further embodiment **A11E3,** the invention provides a process according to embodiment **A11E1** or **A11E2** wherein step (a) comprises a process according to embodiments **A5E1, A5E2, A5E3** or **A5E4** as described above.

In a further embodiment **A11E4,** the invention provides a process according to embodiment **A11E1, A11E2** or **A11E3** wherein step (b) comprises the steps:

(b1) treating the 5-hydroxy-4-(2-methyl-1-propenyl)-5*H*-2-furanone (**VI$^A$**) with a metal oxide, hydroxide, carbonate or bicarbonate, ammonia, a mono- di- or tri-($C_1$-$C_3$)alkylamine, or a tetra-($C_1$-$C_3$)alkylammonium hydroxide to form a salt of formula (**IX**)

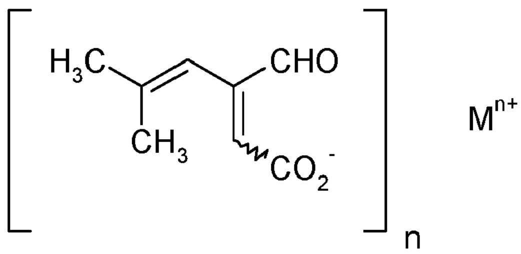

(**IX**)

wherein:

n is 1 and $M^+$ is selected from $Li^+$, $Na^+$, $K^+$, $Rb^+$, $NH_4^+$, (($C_1$-$C_3$)alkyl)$NH_3^+$, (($C_1$-$C_3$)alkyl)$_2NH_2^+$, (($C_1$-$C_3$)alkyl)$_3NH^+$ and (($C_1$-$C_3$)alkyl)$_4N^+$; or
n is 2 and $M^{2+}$ is selected from $Mg^{2+}$, $Ca^{2+}$ and $Z_n^{2+}$;

(b2) hydrogenating the salt of formula **(IX)** to obtain a salt of formula (**X**)

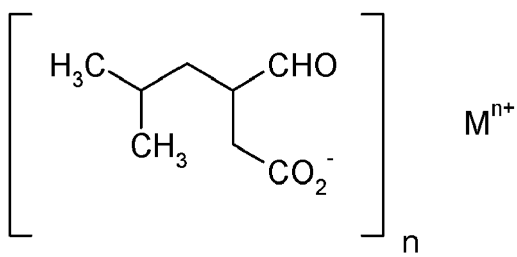

(**X**) ;

and
(b3) treating the salt of formula (**X**) with an acid.

In a further embodiment **A11E5,** the invention provides a process according to embodiment **A11E1, A11E2** or

**A11E3** wherein step (b) comprises the steps:

(b1) converting the 5-hydroxy-4-(2-methyl-1-propenyl)-5*H*-2-furanone (**VI$^A$**) to a compound of formula (**VI**) as defined in embodiment **A1E3,** wherein R is a chiral ($C_5$-$C_{15}$)hydrocarbon group;
(b2) hydrogenating the compound of formula (VI) to obtain a compound of formula (**XI**)

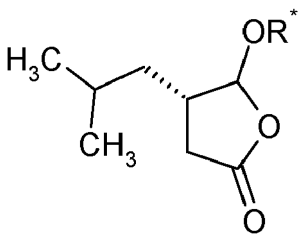

(**XI**)

wherein R* is a chiral ($C_5$-$C_{15}$)hydrocarbon group; and
(b3) treating the compound of formula (XI) with an acid to give ((S)-**I$^A$**).

In another aspect A12, the invention provides a further process for the manufacture of 3-aminomethyl-5-methylhexanoic acid (**II**).
In a first embodiment A12E1, the invention provides a process for the manufacture of 3-aminomethyl-5-methylhexanoic acid (**II**) or a pharmaceutically acceptable salt thereof, comprising the steps:

(a) preparing 5-hydroxy-4-(2-methyl-1-propenyl)-5*H*-2-furanone (**VI$^A$**)

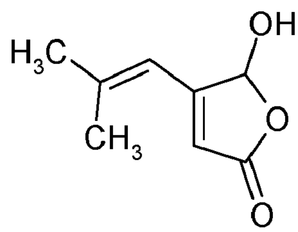

(**VI$^A$**)

(b) treating the 5-hydroxy-4-(2-methyl-1-propenyl)-5*H*-2-furanone (**VI$^A$**) with ammonia or a mono-($C_1$-$C_3$)alkylamine to form a salt of formula **(IX$^A$)**

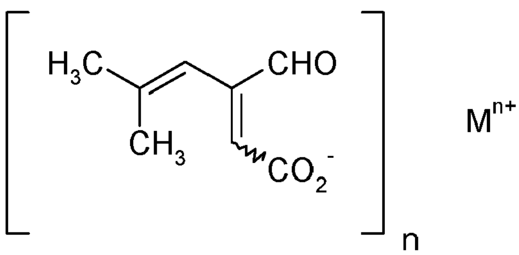

(**IX$^A$**)

wherein n is 1 and $M^+$ is selected from $NH_4^+$ and (($C_1$-$C_3$)alkyl)$NH_3^+$

(c) hydrogenating the salt of formula **(IX$^A$)** to obtain a salt of formula (**X$^A$**)

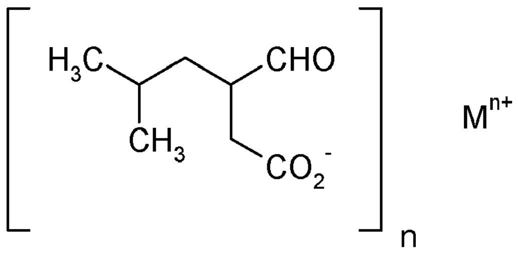

($X^A$) ;

and

(d) treating the salt of formula **($X^A$)** with a transaminase or an amine oxidase/imine reductase enzyme to provide 3-aminomethyl-5-methylhexanoic acid (**II**).

In a further embodiment **A12E2,** the invention provides a process according to embodiment **A12E1** wherein the 3-aminomethyl-5-methylhexanoic acid (**II**) is (S)-3-aminomethyl-5-methylhexanoic acid ((*S*)-**II**)

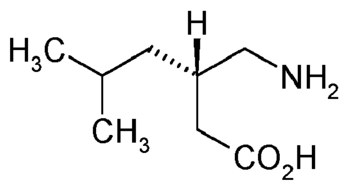

((*S*)-**II**)

wherein said (S)-3-aminomethyl-5-methylhexanoic acid has an enantiomeric excess of at least 80%.

In a further embodiment **A12E3,** the invention provides a process according to embodiment **A12E1** or **A12E2** wherein step (a) comprises a process according to embodiments **A5E1, A5E2, A5E3** or **A5E4** as described above.

In another aspect **A13,** the invention provides a further process for the manufacture of 5-hydroxy-4-(2-methylpropyl)-3,4-dihydro-5*H*-2-furanone ($I^A$).

In a first embodiment **A13E1,** the invention provides a further process for the manufacture of 5-hydroxy-4-(2-methylpropyl)-3,4-dihydro-5*H*-2-furanone ($I^A$) which comprises the steps of:

(a) obtaining 3-isobutylidene-2-oxopentanedioic acid ($XII^A$) or its cyclised isomer ($XII^B$)

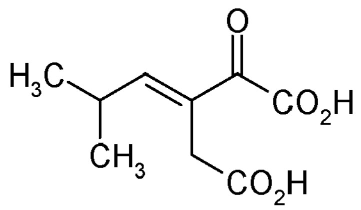

($XII^A$)

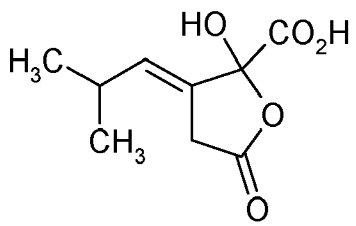

($XII^B$) ,

and

(b) sequentially or simultaneously reducing the carbon-carbon double bond and decarboxylating the $\alpha$-keto acid functional group.

In a further embodiment **A13E2,** the invention provides a process according to embodiment **A13E1** wherein the carbon-carbon double bond is reduced to provide 3-isobutyl-2-oxopentanedioic acid (**XV**) or its cyclised isomer (**XV^A^**)

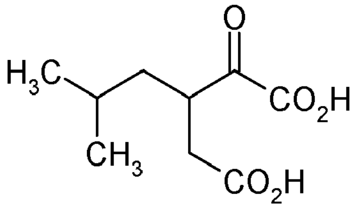

(**XV**)

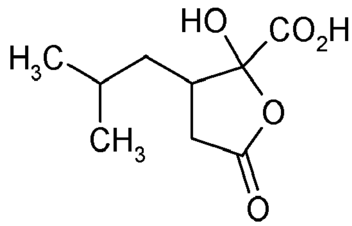

(**XV^A^**)

before the decarboxylation of the α-keto acid functional group.

In a further embodiment **A13E3,** the invention provides a process according to embodiment **A13E1** wherein the α-keto acid functional group is decarboxylated to provide 3-formyl-5-methyl-3-pentenoic acid (**XVI** or its cyclised isomer (**XVI^A^**)

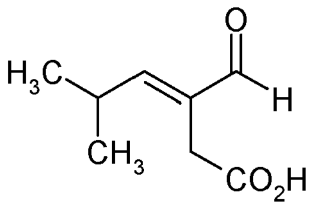

(**XVI**)

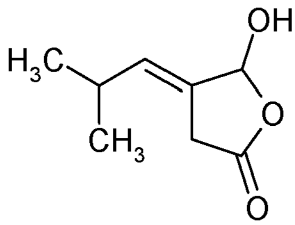

(**XVI^A^**)

before the reduction of the carbon-carbon double bond.

In a further embodiment **A13E4,** the invention provides a process according to embodiment **A13E1** wherein the α-keto acid functional group is decarboxylated and the carbon-carbon double bond is reduced simultaneously.

In a further embodiment **A13E5,** the invention provides a process according to embodiments **A13E1, A13E2, A13E3** or **A13E4** wherein the decarboxylation is carried out in the presence of a decarboxylase enzyme.

In a further embodiment **A13E6,** the invention provides a process according to embodiments **A13E1, A13E2, A13E3, A13E4** or **A13E5** wherein the reduction of the carbon-carbon double bond is carried out in the presence of an enoate reductase enzyme.

In another aspect **A14,** the invention provides a compound selected from:

3-isobutylidene-2-oxopentanedioic acid;
3-isobutyl-2-oxopentanedioic acid; and
3-formyl-5-methyl-3-pentenoic acid,

or a salt, ($C_1$-$C_6$)alkyl ester or cyclised isomer thereof.

In another aspect **A15,** the invention provides a process for the manufacture of (*S*)-3-aminomethyl-5-methylhexanoic acid ((S)-**II**)

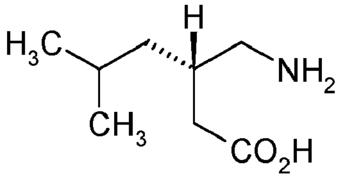

((*S*)-**II**)

or a pharmaceutically acceptable salt thereof, comprising the steps:

(a) manufacturing 5-hydroxy-4-(2-methylpropyl)-3,4-dihydro-5*H*-2-furanone (**I^A^**) using a process according to any one of embodiments **A13E1, A13E2, A13E3, A13E4, A13E5** or **A13E6** as defined above, and
(b) converting said 5-hydroxy-4-(2-methylpropyl)-3,4-dihydro-5*H*-2-furanone into (*S*)-3-aminomethyl-5-methylhexanoic acid.

In another aspect **A16,** the invention provides a process for converting (*R*)-3-aminomethyl-5-methylhexanoic acid into (*S*)-3-aminomethyl-5-methylhexanoic acid comprising treating the (*R*)-3-aminomethyl-5-methylhexanoic acid with a transaminase enzyme or an amine oxidase/imine reductase enzyme.
In another aspect **A17,** the invention provides a process for increasing the proportion of (*S*)-3-aminomethyl-5-methylhexanoic acid in a mixture of (*R*)- and (*S*)-3-aminomethyl-5-methylhexanoic acid comprising treating the mixture with a transaminase enzyme or an amine oxidase/imine reductase enzyme.
In another aspect **A18,** the invention provides a transaminase enzyme that is useful for the conversion of 5-hydroxy-4-(2-methylpropyl)-3,4-dihydro-5*H*-2-furanone ($\mathbf{I^A}$) into Pregabalin.
In a first embodiment **A18E1,** the invention provides a transaminase enzyme having an amino acid sequence that has at least 95% homology to the amino acid sequence

MNKPQSWEARAETYSLYGFTDMPSLH$\mathbf{X^{27}}$RGTVVVTHGEGPY$\mathbf{X^{41}}$VDV$\mathbf{X^{45}}$GRRYLDAN
SGLYNMVAGFDHKGLIDAAKAQYERFPGYHSFFGRMSDQTVMLSEKLVEVSPFDSGR
VFYTNSGSEANDTMVKMLWFLHAAEGKPQKRKILTR$\mathbf{X^{147}}$NAYHGVTAVSASMTG$\mathbf{X^{163}}$P
$\mathbf{X^{165}}$NSVFGLPLPGFVHL$\mathbf{X^{180}}$CPHYWRYGEEGETEEQFVARLARELEETIQREGADTIAG
FFAEPVMGAGGVIPPAKGYFQAILPILRKYDIPVISDEVICGFGRTGNTWGCVTYDFTPD
AIISSKNLTAGFFPVGAVILGPEL$\mathbf{X^{304}}$KRLETAIEAIEEFPHGFTA$\mathbf{X^{324}}$GHPVGCAIALKAID
VVMNEGLAENVRRLAPRFEERLKHIAERPNIGEYRGIGFMWALEAVKDKASKTPFDGN
LSVS$\mathbf{X^{401}}$RIANTC$\mathbf{X^{408}}$DLGLIC$\mathbf{X^{415}}\mathbf{X^{416}}\mathbf{X^{417}}$GQSVIL$\mathbf{X^{424}}$PPFILTEAQMDEMFDKLEKALD
KVFAEVA (SEQ ID NO. 1)

wherein

$\mathbf{X^{27}}$ is selected from glutamine (Q) and glutamic acid (E);
$\mathbf{X^{41}}$ is selected from isoleucine (I) and valine (V);
$\mathbf{X^{45}}$ is selected from asparigine (N) and histidine (H);
$\mathbf{X^{147}}$ is selected from asparigine (N) and glutamine (Q);
$\mathbf{X^{163}}$ is selected from leucine (L) and methionine (M);
$\mathbf{X^{165}}$ is selected from tyrosine (Y) and histidine (H);
$\mathbf{X^{180}}$ is selected from threonine (T); glycine (G) and serine (S);
$\mathbf{X^{304}}$ is selected from alanine (A) and serine (S);
$\mathbf{X^{324}}$ is selected from glycine (G) and serine (S);
$\mathbf{X^{401}}$ is selected from lysine (K) and glutamic acid (E);
$\mathbf{X^{408}}$ is selected from threonine (T) and glutamine (Q);
$\mathbf{X^{415}}$ is selected from serine (S) and alanine (A);
$\mathbf{X^{416}}$ is selected from proline (P) and alanine (A);
$\mathbf{X^{417}}$ is selected from leucine (L) and methionine (M); and
$\mathbf{X^{424}}$ is selected from cysteine (C) and serine (S).

In a further embodiment **A18E2,** the invention provides a transaminase enzyme according to embodiment **A18E1** having the amino acid sequence of SEQ ID NO. 1.
In a further embodiment **A18E3,** the invention provides a transaminase enzyme according to embodiment **A18E1** or **A18E2** wherein:

$X^{27}$ is glutamic acid (E);
$X^{147}$ is glutamine (Q);
$X^{165}$ is histidine (H);
$X^{304}$ is serine (S);
$X^{324}$ is glycine (G);
$X^{401}$ is lysine (K);
$X^{408}$ is glutamine (Q);
$X^{416}$ is alanine (A);
$X^{417}$ is methionine (M); and
$X^{424}$ is serine (S).

In a further embodiment **A18E4,** the invention provides a transaminase enzyme according to embodiment **A18E2** having an amino acid sequence selected from:

MNKPQSWEARAETYSLYGFTDMPSLHQRGTVVVTHGEGPYIVDVNGRRYLDANSGLYNMVAGFDHKGLIDAAKAQYERFPGYHSFFGRMSDQTVMLSEKLVEVSPFDSGRVFYTNSGSEANDTMVKMLWFLHAAEGKPQKRKILTRNNAYHGVTAVSASMTGLPYNSVFGLPLPGFVHLTCPHYWRYGEEGETEEQFVARLARELEETIQREGADTIAGFFAEPVMGAGGVIPPAKGYFQAILPILRKYDIPVISDEVICGFGRTGNTWGCVTYDFTPDAIISSKNLTAGFFPVGAVILGPELAKRLETAIEAIEEFPHGFTASGHPVGCAIALKAIDVVMNEGLAENVRRLAPRFEERLKHIAERPNIGEYRGIGFMWALEAVKDKASKTPFDGNLSVSERIANTCTDLGLICSPMGQSVILCPPFILTEAQMDEMFDKLEKALDKVFAEVA (SEQ ID NO. 2);

MNKPQSWEARAETYSLYGFTDMPSLHERGTVVVTHGEGPYIVDVNGRRYLDANSGLYNMVAGFDHKGLIDAAKAQYERFPGYHSFFGRMSDQTVMLSEKLVEVSPFDSGRVFYTNSGSEANDTMVKMLWFLHAAEGKPQKRKILTRNNAYHGVTAVSASMTGLPYNSVFGLPLPGFVHLTCPHYWRYGEEGETEEQFVARLARELEETIQREGADTIAGFFAEPVMGAGGVIPPAKGYFQAILPILRKYDIPVISDEVICGFGRTGNTWGCVTYDFTPDAIISSKNLTAGFFPVGAVILGPELSKRLETAIEAIEEFPHGFTAGGHPVGCAIALKAIDVVMNEGLAENVRRLAPRFEERLKHIAERPNIGEYRGIGFMWALEAVKDKASKTPFDGNLSVSKRIANTCQDLGLICSALGQSVILCPPFILTEAQMDEMFDKLEKALDKVFAEVA (SEQ ID NO. 3);

MNKPQSWEARAETYSLYGFTDMPSLHERGTVVVTHGEGPYVVDVNGRRYLDANSGLYNMVAGFDHKGLIDAAKAQYERFPGYHSFFGRMSDQTVMLSEKLVEVSPFDSGRVFYTNSGSEANDTMVKMLWFLHAAEGKPQKRKILTRQNAYHGVTAVSASMTGLPHNSVFGLPLPGFVHLTCPHYWRYGEEGETEEQFVARLARELEETIQREGADTIAGFFAEPVMGAGGVIPPAKGYFQAILPILRKYDIPVISDEVICGFGRTGNTWGCVTYDFTPDAIISSKNLTAGFFPVGAVILGPELSKRLETAIEAIEEFPHGFTAGGHPVGCAIALKAIDVVMNEGLAENV

RRLAPRFEERLKHIAERPNIGEYRGIGFMWALEAVKDKASKTPFDGNLSVSKRIANTCQ DLGLICSALGQSVILSPPFILTEAQMDEMFDKLEKALDKVFAEVA (SEQ ID NO. 4);

MNKPQSWEARAETYSLYGFTDMPSLHERGTVVVTHGEGPYIVDVNGRRYLDANSGLY NMVAGFDHKGLIDAAKAQYERFPGYHSFFGRMSDQTVMLSEKLVEVSPFDSGRVFYT NSGSEANDTMVKMLWFLHAAEGKPQKRKILTRQNAYHGVTAVSASMTGMPHNSVFG LPLPGFVHLTCPHYWRYGEEGETEEQFVARLARELEETIQREGADTIAGFFAEPVMGA GGVIPPAKGYFQAILPILRKYDIPVISDEVICGFGRTGNTWGCVTYDFTPDAIISSKNLTA GFFPVGAVILGPALSKRLETAIEAIEEFPHGFTAGGHPVGCAIALKAIDVVMNEGLAENV RRLAPRFEERLKHIAERPNIGEYRGIGFMWALEAVKDKASKTPFDGNLSVSKRIANTCQ DLGLICSAMGQSVILSPPFILTEAQMDEMFDKLEKALDKVFAEVA (SEQ ID NO. 5);

MNKPQSWEARAETYSLYGFTDMPSLHERGTVVVTHGEGPYVVDVNGRRYLDANSGL YNMVAGFDHKGLIDAAKAQYERFPGYHSFFGRMSDQTVMLSEKLVEVSPFDSGRVFY TNSGSEANDTMVKMLWFLHAAEGKPQKRKILTRQNAYHGVTAVSASMTGLPHNSVFG LPLPGFVHLGCPHYWRYGEEGETEEQFVARLARELEETIQREGADTIAGFFAEPVMGA GGVIPPAKGYFQAILPILRKYDIPVISDEVICGFGRTGNTWGCVTYDFTPDAIISSKNLTA GFFPVGAVILGPELSKRLETAIEAIEEFPHGFTAGGHPVGCAIALKAIDVVMNEGLAENV RRLAPRFEERLKHIAERPNIGEYRGIGFMWALEAVKDKASKTPFDGNLSVSKRIANTCQ DLGLICAAMGQSVILSPPFILTEAQMDEMFDKLEKALDKVFAEVA (SEQ ID NO. 6); and

MNKPQSWEARAETYSLYGFTDMPSLHERGTVVVTHGEGPYIVDVHGRRYLDANSGLY NMVAGFDHKGLIDAAKAQYERFPGYHSFFGRMSDQTVMLSEKLVEVSPFDSGRVFYT NSGSEANDTMVKMLWFLHAAEGKPQKRKILTRQNAYHGVTAVSASMTGLPHNSVFGL PLPGFVHLSCPHYWRYGEEGETEEQFVARLARELEETIQREGADTIAGFFAEPVMGAG GVIPPAKGYFQAILPILRKYDIPVISDEVICGFGRTGNTWGCVTYDFTPDAIISSKNLTAG FFPVGAVILGPELSKRLETAIEAIEEFPHGFTAGGHPVGCAIALKAIDVVMNEGLAENVR RLAPRFEERLKHIAERPNIGEYRGIGFMWALEAVKDKASKTPFDGNLSVSKRIANTCQD LGLICSAMGQSVILSPPFILTEAQMDEMFDKLEKALDKVFAEVA (SEQ ID NO. 7).

In another aspect **A19,** the invention provides a process for the manufacture of (S)-3-aminomethyl-5-methylhexanoic acid ((S)-**II**)

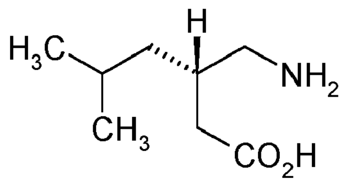

((*S*)-**II**)

or a pharmaceutically acceptable salt thereof, comprising the step of treating 5-hydroxy-4-(2-methylpropyl)-3,4-dihydro-5*H*-2-furanone (**I**[A]) and an amine with a transaminase enzyme according to any one of embodiments **A18E1, A18E2, A18E3** and **A18E4.**

Detailed Description of Preferred Embodiments

The term "alkyl" means a straight-chain or branched-chain saturated aliphatic hydrocarbon radical containing the specified number of carbon atoms. Examples of alkyl radicals include methyl, ethyl, *n*-propyl, isopropyl, *n*-butyl, isobutyl, *sec*-butyl, *tert*-butyl, neopentyl and *n*-hexyl.

The term "alkoxy" means a group made up of an alkyl group as defined above connected to an oxygen atom. Examples of alkoxy groups include methoxy, ethoxy and isopropoxy.

The term "alkoxy-alkyl" means a straight-chain or branched-chain saturated aliphatic hydrocarbon radical in which an alkoxy group is substituted for an alkyl hydrogen atom. An example of an alkoxy-alkyl group is 2-methoxyethyl.

The term "haloalkyl" means an alkyl group as defined above wherein one or more hydrogen atoms are replaced by fluorine, chlorine, bromine or iodine. When more than one hydrogen atom is replaced, the replacing halogen atoms may be the same or different. Examples of haloalkyl groups include fluoromethyl, difluoromethyl, trifluoromethyl, chlorodifluoromethyl, 2,2,2-trifluoroethyl and 3-bromopropyl.

The term "aryl" means a phenyl or naphthyl group.

The term "aryl-alkyl" means a straight-chain or branched-chain saturated aliphatic hydrocarbon radical in which an aryl group is substituted for an alkyl hydrogen atom. An example of an aryl-alkyl group is benzyl.

The term "cycloalkyl" means a saturated monocyclic or polycyclic carbocyclic ring containing the specified number of carbon atoms. Examples of monocyclic cycloalkyl groups include cyclopropyl, cyclobutyl, cyclopentyl and cyclohexyl. Examples of polycyclic cycloalkyl groups include bicyclo[2,2,1]heptyl and bicyclo[3,2,1]octyl.

The term "optionally substituted" with reference to an alkyl or aryl group means that a hydrogen atom of the alkyl or aryl group may be replaced by one of the groups listed. The substitution may be made at any position within the alkyl or aryl group. When the optional substitution is with "one or more groups" then any number of hydrogen atoms of the alkyl or aryl group, up to a maximum equal to the number of hydrogens present in the alkyl or aryl group, may be replaced, and each replacement is independent of the others.

The term "enantiomeric excess", sometimes abbreviated as "e.e.", is a measure, for a given sample, of the excess of one enantiomer in excess of its antipode and is expressed as a percentage. Enantiomeric excess is defined as:

$$100 \times (er-1) / (er+1)$$

where "er" is the ratio of the more abundant enantiomer to the less abundant enantiomer.

5-Hydroxy-4-(2-methylpropyl)-3,4-dihydro-5*H*-2-furanone (**I**[A]) is a convenient intermediate in the manufacture of 3-aminomethyl-5-methylhexanoic acid (**II**). Treatment of racemic (**I**[A]) with ammonia in the presence of a chemical reducing agent provides racemic 3-aminomethyl-5-methylhexanoic acid ((*R*/*S*)-**II**) (see WO2008/127646A2). The reaction is presumed to involve the ring-opened isomer (**I**[B]).

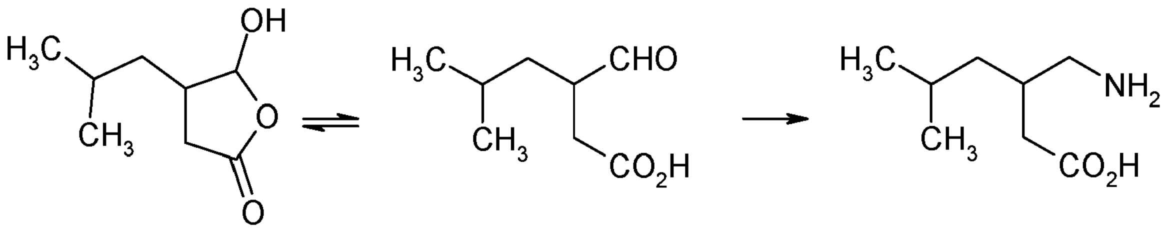

($I^A$) ($I^B$) (II)

Reductive amination with an amine donor in the presence of a transaminase enzyme can provide enantiomerically enriched 3-aminomethyl-5-methylhexanoic acid. Suitable amine donors are primary amines such as mono-alkylamines, particularly isopropylamine, and α-amino acids.

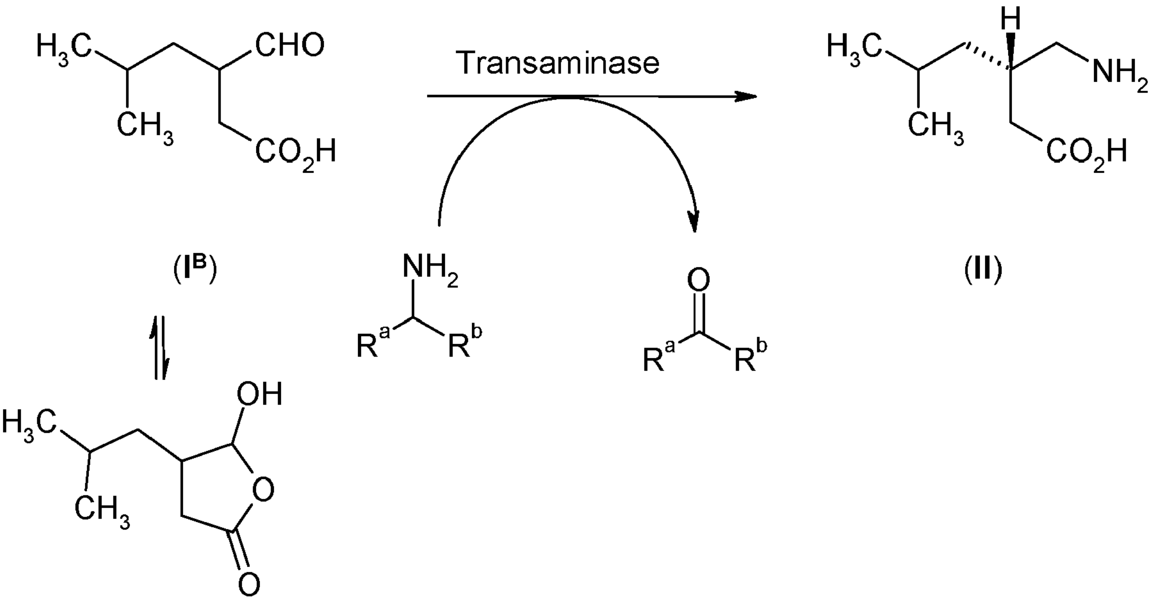


($I^B$) (II)

($I^A$)

The reaction of ($I^A$) with a suitable amine dehydrogenase/ imine reductase in the presence of ammonia can also be a suitable route to pregabalin. A co-factor such as NADH or NADPH may be needed in a stoichiometric amount, or a second oxidoreductase, such as formate dehydrogenase, may be included to recycle the co-factor.

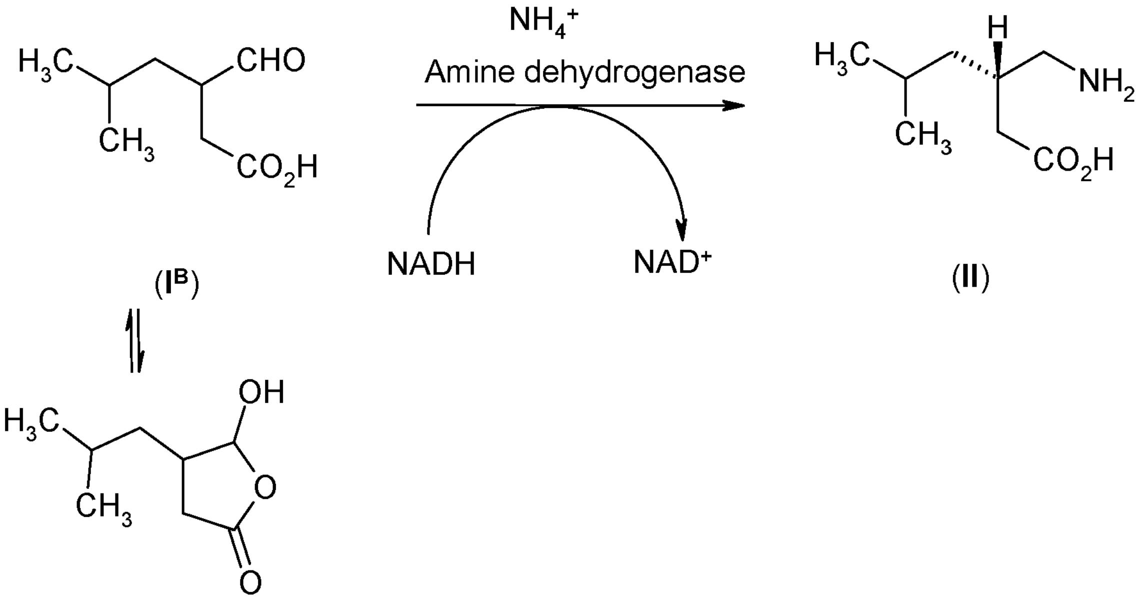


($I^B$) (II)

($I^A$)

Where the dihydrofuranone (**I**$^A$) has a defined stereochemistry at the C-4 position, this stereochemistry may be preserved during the reductive amination reaction. Since Pregabalin has the (S)-stereochemistry it may be preferred to have this stereochemistry already present in the dihydrofuranone.

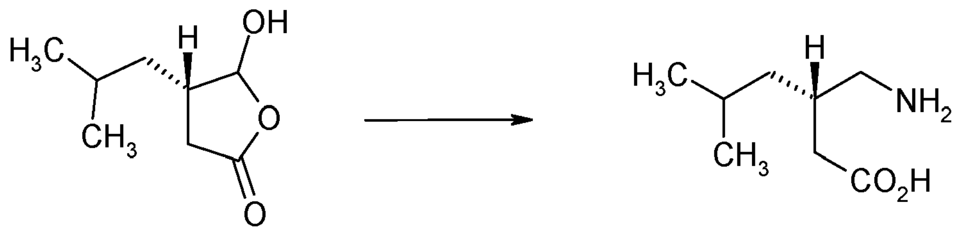

((*S*)-**I**$^A$) ((*S*)-**II**)

Alternatively, the dihydrofuranone (**I**$^A$) may be used as in racemic form. The desired stereoisomer of the product may then be obtained either by carrying out the reductive amination reaction under conditions that allow for the stereoselective formation of a single enantiomer, such as by carrying out the transformation in the presence of a transaminase enzyme, or by subjecting the product to a separate resolution step, such as by crystallization with a chiral acid or base.

The dihydrofuranone (**I**$^A$) may be conveniently prepared in racemic or enantiomerically enriched form using the methods set out below.

In a first method, the dihydrofuranone (**I**$^A$) is prepared by reduction of 5-hydroxy-4-(2-methyl-1-propenyl)-5H-2-furanone (**VIA**).

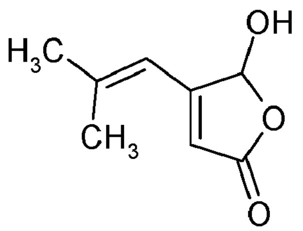

(**VI**$^A$)

The reduction may conveniently be accomplished by hydrogenation in the presence of a suitable catalyst. Suitable catalysts include homogeneous and heterogeneous catalysts. The catalyst typically comprises a transition metal such as palladium, platinum, rhodium, ruthenium or nickel, or a salt or oxide thereof. Heterogeneous catalysts include finely divided metals and substrate-supported metals and metal oxides, where the substrate may be carbon, silica, alumina or any other suitable inert material. Homogeneous catalysts include phosphine ligand complexes of transition metals. When the phosphine ligand is chiral then the catalyst is chiral. When an achiral catalyst is used, then the product is the racemic dihydrofuranone (**I$^A$**). The use of a chiral catalyst may provide the dihydrofuranone (**I$^A$**) in an enantioselective manner.

The selectivity of the hydrogenation reaction, and the overall yield, maybe improved when the reaction is carried out in an alkaline medium. Without being bound by theory, it is thought that in the presence of a base the furanone exists predominantly as the ring-opened salt (**IX**).

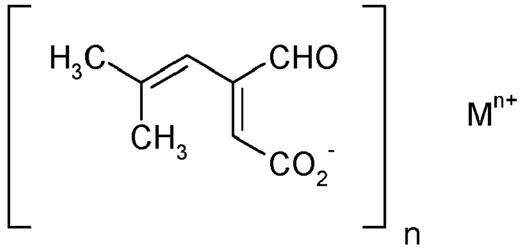

(**IX**)

Any suitable base may be used provided that it does not interfere with the hydrogenation process, such as by poisoning the catalyst. Examples of suitable bases include alkali (such as Li, Na, K and Rb) and alkaline earth metal (such as Ca and Mg) oxides, hydroxides, carbonates and bicarbonates. Other metal salts, such as zinc salts, may also be used. Alkali metal salts may be preferred due to their good solubility and/or low toxicity. Amine bases such as ammonia, and primary, secondary and tertiary amines may be used to prepare ammonium salts. Tetra-alkylammonium hydroxide may also be used, leading to the formation of tetra-alkylammonium salts.

Hydrogenation of the salt of formula **(IX)** provides a salt of formula (**X**).

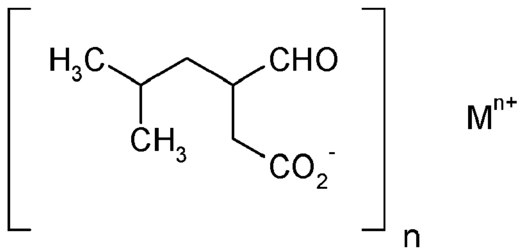

(**X**)

The dihydrofuranone (**I$^A$**) is recovered, after the hydrogenation reaction, by treatment of this salt with a suitable acid. Alternatively, the salt may be converted directly to 3-aminomethyl-5-methylhexanoic acid (**II**) by treatment with a transaminase or amine oxidase/imine reductase enzyme. In this case it may be preferable to use the ammonium salt ($M^+ = NH_4^+$) or a primary alkylammonium salt ($M^+$ = alkyl-$NH_3^+$) since the ammonium or alkylammonium ion provides the co-substrate for the enzyme. The use of the isopropylammonium salt in combination with a transaminase enzyme is a preferred example.

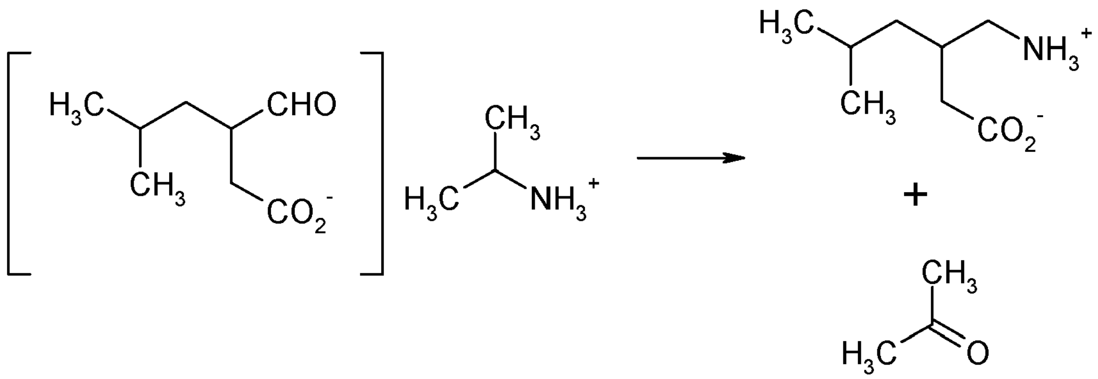

Furanones of formula (**VI**) wherein R is other than hydrogen may also be reduced by hydrogenation. Where R is an alkyl, haloalkyl, alkoxyalkyl alkenyl, cycloalkyl, cycloalkyl-alkyl, aryl or aryl-alkyl group, these furanones may be prepared from the compound of formula (**VI$^{A}$**) by reaction with an alcohol R-OH in the presence of an acid catalyst. Where R is $R^1$-C(O)- the furanones may be prepared from the compound of formula (**VI$^{A}$**) by reaction with an acid anhydride $(R^1\text{-C(O)})_2O$ or acid chloride $R^1$-C(O)-Cl, optionally in the presence of a base, for example a tertiary amine. Where R is $R^2$-$SO_2$- the furanones may be prepared from the compound of formula (**VI$^{A}$**) by reaction with a sulfonyl chloride $R^2$-$SO_2$-Cl, optionally in the presence of a base, for example a tertiary amine.

Following the hydrogenation step the dihydrofuranone of formula (**I$^{A}$**) can be generated from the reduction product by treatment with acid (where R is an alkyl, haloalkyl, alkoxyalkyl alkenyl, cycloalkyl, cycloalkyl-alkyl, aryl or aryl-alkyl group) or base (where R is $R^1$-C(O)- or $R^2$-$SO_2$-).

The use of a chiral R group may provide a chiral hydrogenation product without the need for a chiral catalyst.

For example, the furanone (**VI$^{A}$**) is reacted with a chiral alcohol R*-OH to afford a chiral ether derivative (**VI$^{B}$**).

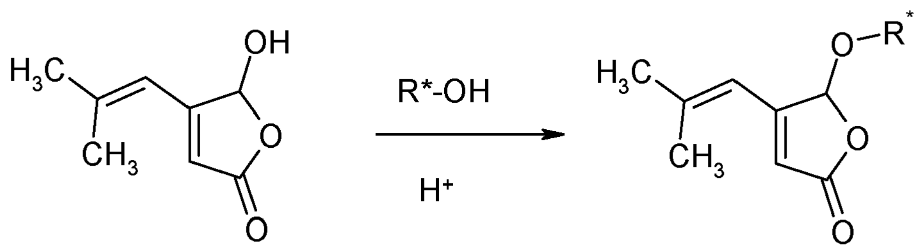


(**VI$^{A}$**) (**VI$^{B}$**)

Suitable chiral alcohols may include α-aryl alcohols such as 1-phenylethanol and 1-naphthylethanol, as well as terpene alcohols such as menthol and borneol. Hydrogenation of derivative (**VI$^{B}$**) may proceed in an enantioselective manner, and treatment of the resulting product with a suitable acid and in the presence of water then provides the dihydrofuranone (**I$^{A}$**) in chiral form.

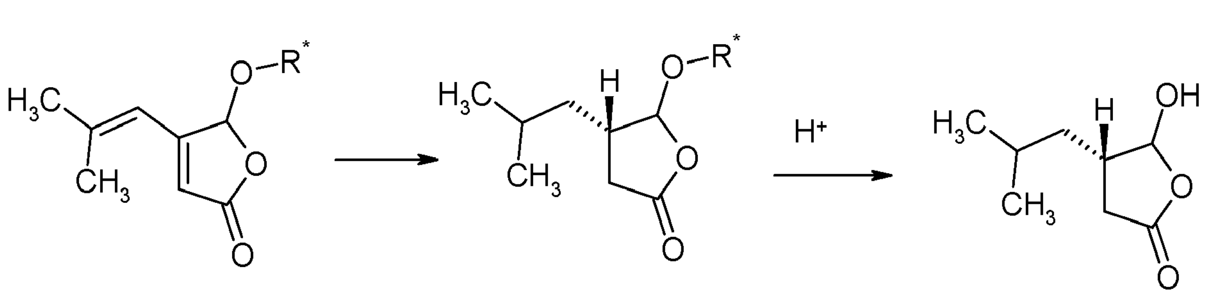


(**VI$^{B}$**) ((S)-**I$^{A}$**)

The furanone (**VI$^{A}$**) may be prepared from a compound of formula (**VII**)

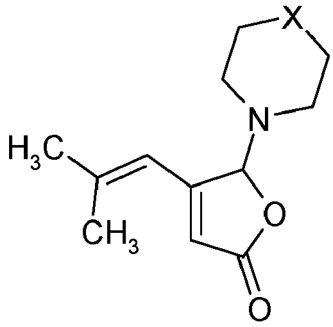

(**VII**)

wherein -X- represents a single bond, $-CH_2-$, -O-, -NH-, $-N((C_1-C_3)alkyl)-$, -N(benzyl)-, or

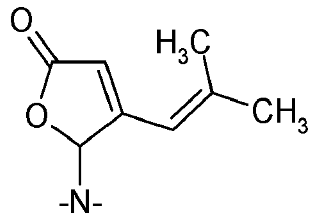

by treating the compound of formula (**VII**) with water in the presence of an acid catalyst. Suitable acids include mineral acids such as sulphuric acid. Alternatively, the compound of formula (**VII**) can be treated with an alcohol R-OH to provide directly a compound of formula (**VI**) wherein R is an alkyl, haloalkyl, alkoxyalkyl alkenyl, cycloalkyl, cycloalkyl-alkyl, aryl or aryl-alkyl group.

The compounds of formula (**VII**) can be prepared by the reaction of a dienamine of formula (**VIII**).

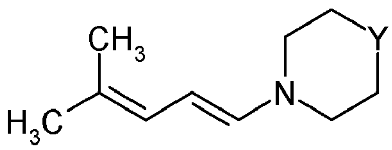

(**VIII**)

wherein -Y- represents a single bond, $-CH_2-$, -O-; -NH-, $-N((C_1-C_3)alkyl)-$, -N(benzyl)-, or

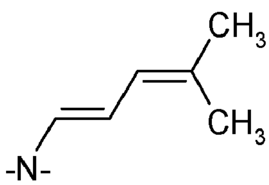

with glyoxylic acid or its hydrate.

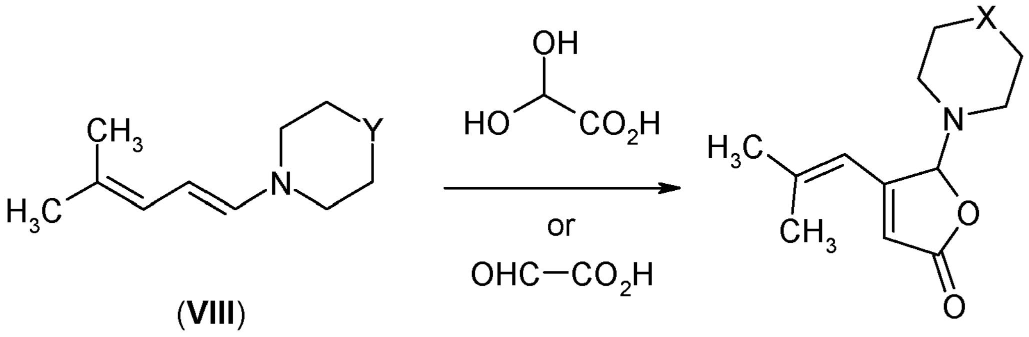


(VIII)

(VII)

It will be understood that -X- in formula (**VII**) corresponds to -Y- in the starting material of formula (**VIII**), except that when -Y- is

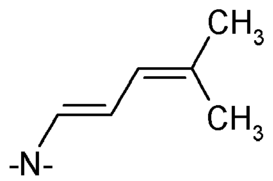

then –X- is

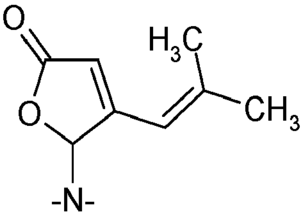

The compounds of formula (**VIII**) wherein -Y- represents a single bond or $-CH_2-$ have been prepared by the reaction of 4-methyl-2-pentenal with pyrrolidine or piperidine (Kienzle, F. et al., Helv. Chim. Acta 1985, 68(5), 1133-39). Other compounds of formula (**VIII**) may be prepared analogously.

Alternatively, condensation of isobutyraldehyde and acetaldehyde in the presence of a suitable amine such as pyrrolidine, piperidine or morpholine with catalytic acid in a solvent such as acetonitrile provides the dienamine derivatives (**VIII**).

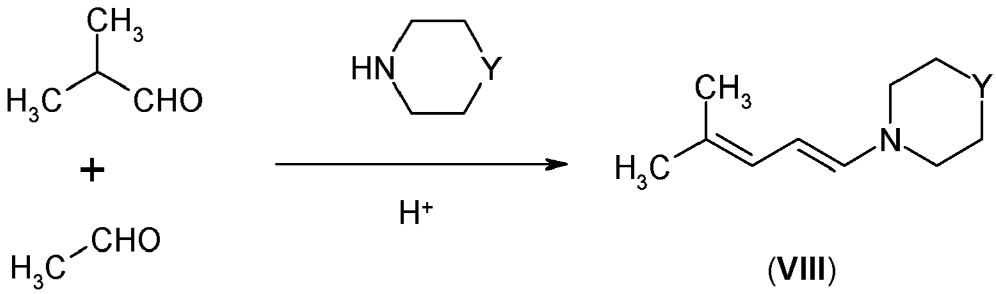


(VIII)

Y = single bond, $CH_2$, O, N(alkyl), N(benzyl)

If piperazine is used as the amine then a *bis*-dienamine is obtained.

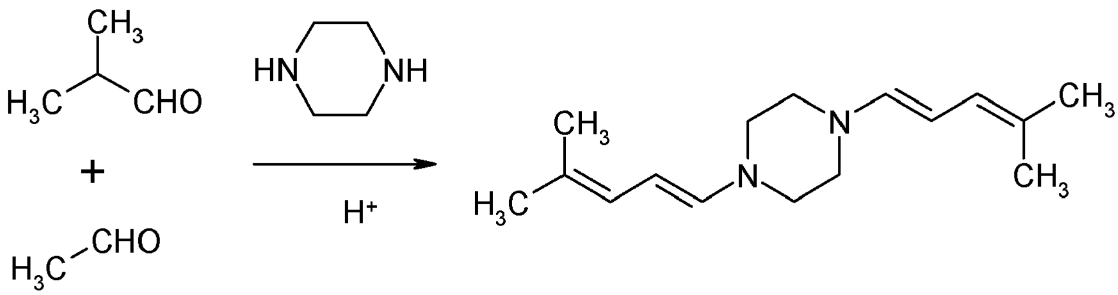


The compound of formula (**VIII**) wherein Y is NH may be obtained by using mono-protected piperazine as the

amine, followed by a deprotection step.

Acyclic secondary amines such as diethylamine and di-isopropylamine may also be used, but cyclic secondary amines are preferred.

This mode of reaction of isobutyraldehyde and acetaldehyde differs from the direct base catalysed reaction (eg using potassium carbonate as base: UK patent GB834100) which only gives the adduct 2,2-dimethyl-3-hydroxybutanal where isobutyraldehyde acts as a nucleophile.

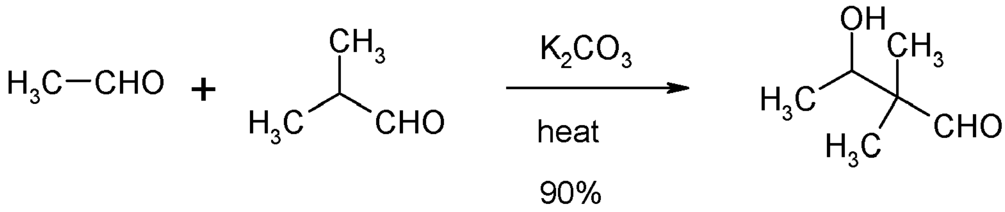


Without wishing to be bound by any particular theory, it is postulated that both the acetaldehyde and the isobutyraldehyde are initially converted to their enamine derivatives. In the presence of the acid catalyst, the more basic isobutyraldehyde enamine is converted to its iminium ion. This electrophilic species reacts preferentially with the less sterically hindered nucleophile, which is the acetaldehyde enamine - this ensures reaction the desired way around.

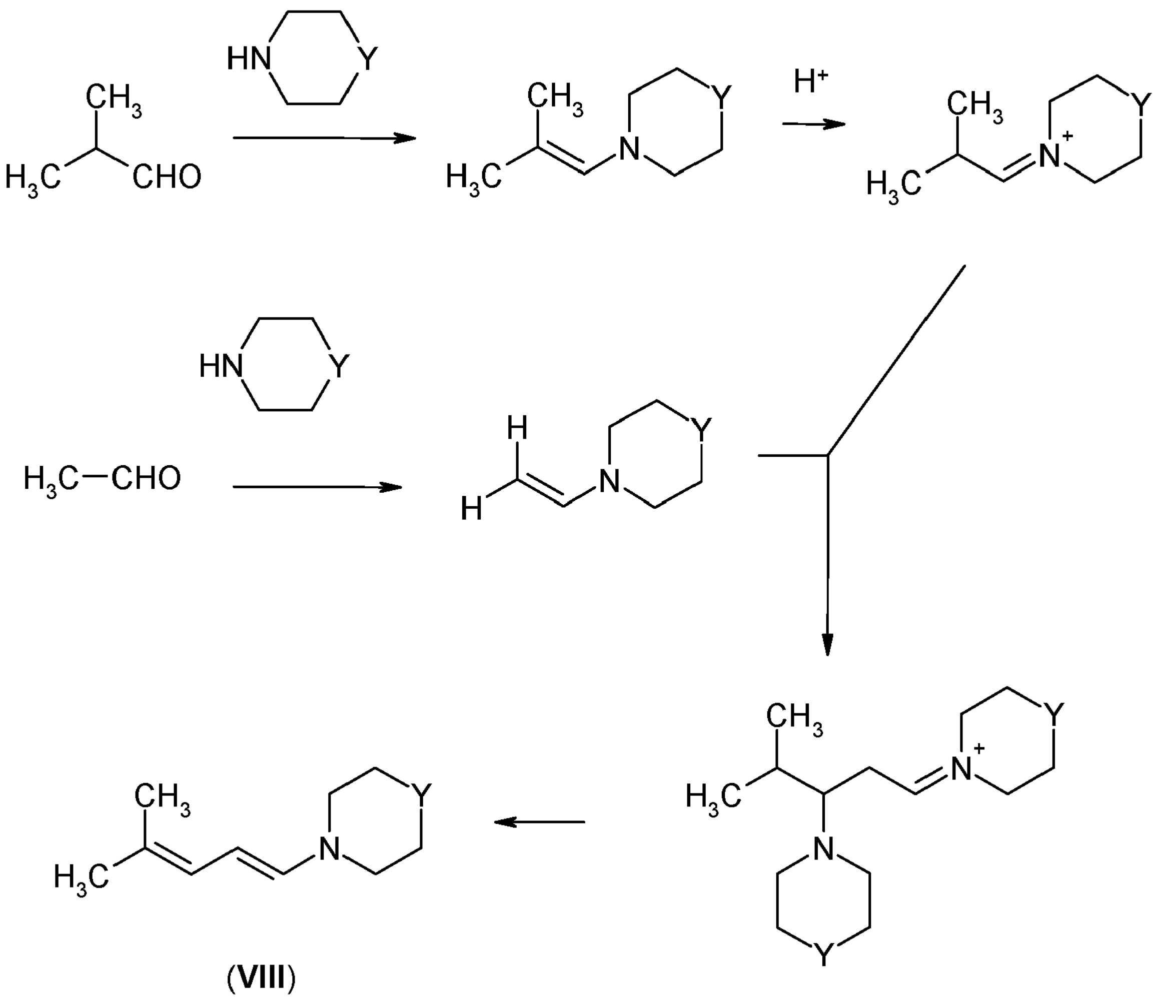


(VIII)

The method of the present invention is more economical and more amenable to scale-up than the literature methods of effecting this 'umpolung' of normal acetaldehyde reactivity, in which acetaldehyde is converted to an O-silylated enol derivative and coupled with the isobutyraldehyde under Mukiyama aldol conditions, In the present invention, both coupling partners are activated simultaneously - electronic and steric effects directing the observed reactivity pattern. The other advantage is that the product dienamine is the desired 'activated' form of 4-methyl-2-pentenal for reaction

with glyoxylic acid to form the desired 5-aminofuranones (**VII**).

These dienamine derivatives of formula (**VIII**) may be isolated and purified, or alternatively they can be treated directly with glyoxylic acid (or its hydrate), which provides furanone derivative (**VII**) directly.

The furanone derivatives (**VII**) may be isolated and purified. Treatment with aqueous acid then provides furanone (**VIA**).

Overall, the transformations set out above provide a short route for the manufacture of pregabalin using inexpensive and safe starting materials.

In an alternative embodiment, the dihydrofuranone (**I**) is prepared from 3-isobutylidene-2-oxopentanedioic acid (**XII**), which is readily obtained by the condensation of isobutyraldehyde with 2-oxopentanedioic acid (α-ketoglutaric acid) (**XIV**).

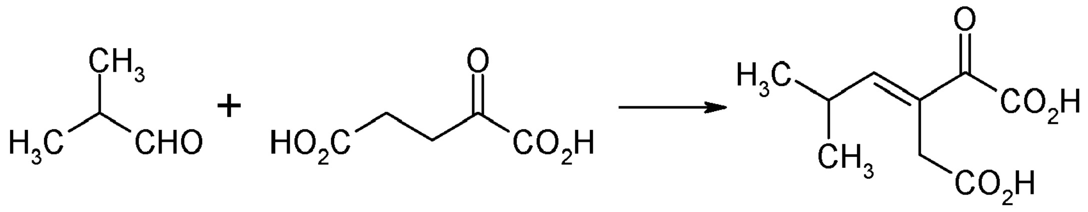

(**XIV**) (**XII**)

Conversion of diacid (**XII**) to dihydrofuranone (**I**) requires a decarboxylation step and a reduction step. These two process steps may be carried out separately, in which case either the decarboxylation step or the reduction step may be the first step, or the two processes may be carried out simultaneously. When the reduction step is carried out first, the intermediate (**XV**) will be produced. When the decarboxylation step is carried out first the intermediate (**XVI**) will be produced.

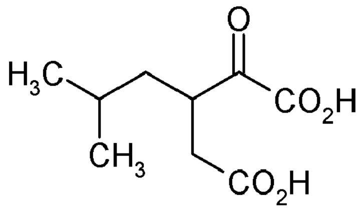

(**XV**)

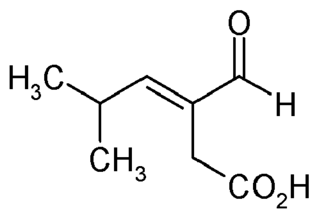

(**XVI**)

The reduction step may be carried out chemically, such as by hydrogenation, but is preferably achieved using an enzyme-mediated reduction, such as by treating with an enoate reductase enzyme. The decarboxylation step is preferably performed by treating the compound with a decarboxylase enzyme.

Where enzyme-mediated transformations are contemplated, the enzyme may be an isolated enzyme, including an enzyme immobilized on a carrier, it may be a partially isolated enzyme preparation such as a cell homogenate, or it may be a non-isolated enzyme, in which case a whole-cell preparation is used. Cells may include those which express the desired enzyme naturally, and cells that have been manipulated so as to express the desired enzyme.

The enzyme-mediated reductive amination of the compound of formula ($I^A$) is reversible, and so treatment of 3-aminomethyl-5-methylhexanoic acid (**II**) with a transaminase enzyme or an amine oxidase/imine reductase enzyme can lead to the formation of the dihydrofuranone ($I^A$). The ring-opened isomer of this is compound ($I^B$), which is epimerizable. In view of this, it is possible to convert ((*R*)-**II**) into ((*S*)-**II**), or to increase the optical purity of a mixture of ((*R*)-**II**) and ((*S*)-**II**) using such an enzyme.

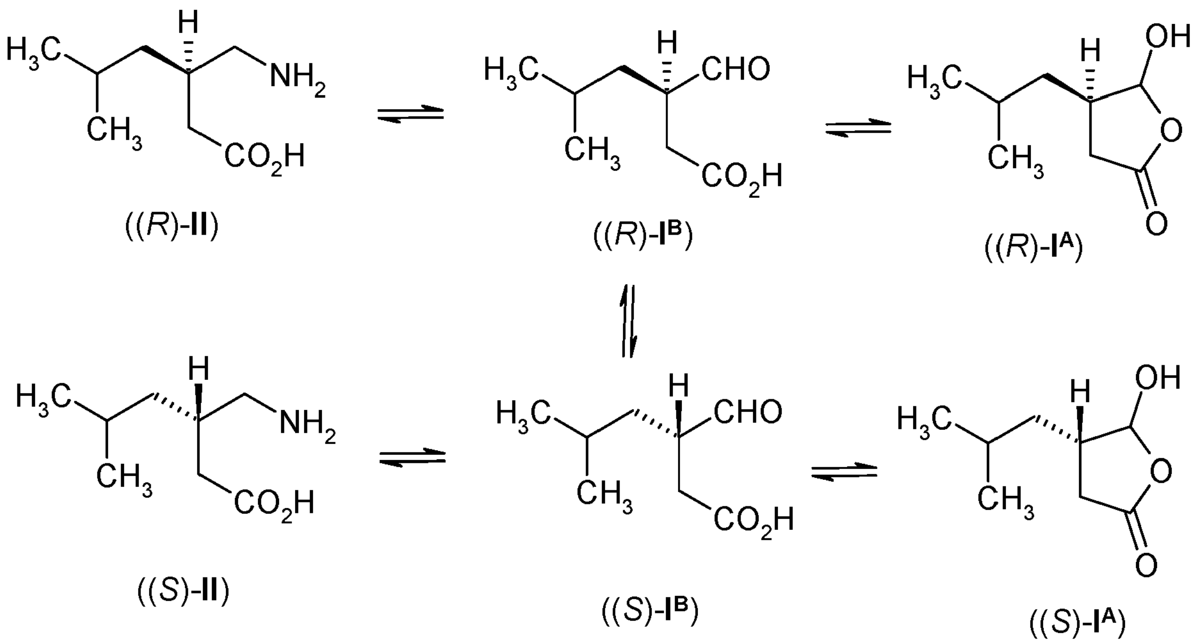

((*R*)-**II**) ((*R*)-**I**[B]) ((*R*)-**I**[A])

((*S*)-**II**) ((*S*)-**I**[B]) ((*S*)-**I**[A])

Examples

The invention is illustrated by the following non-limiting examples in which the following abbreviations and definitions are used:

| | |
|---|---|
| bp | Boiling point |
| CPME | Cyclopentyl methyl ether |
| d | Doublet |
| DIW | De-ionised water |
| dd | Doublet of doublets |
| eq or eq. | Equivalent |
| e.e. or ee | Enantiomeric excess |
| $ES^+$ | Positive mode electrospray ionization |
| EtOAc | Ethyl Acetate |
| EtOH | Ethanol |
| GC | Gas chromatography |
| GC/MS | Gas chromatography / Mass spectroscopy |
| HOAc | Acetic acid |
| HPLC | High Performance Liquid Chromatography |
| Hr or h | Hour |
| $^1H$ NMR | Proton Nuclear Magnetic Resonance Spectroscopy |
| L | Litre |
| LCMS | Liquid chromatography / Mass spectroscopy |
| m | Multiplet |
| mbar | Millibar |
| MeOH | Methanol |
| min | Minute |
| mL | Millilitre |
| mmol | Millimole |
| mol | Mole |
| Mp | Melting point |
| MTBE | Methyl tert-butyl Ether |
| $NAD^+$ | Nicotinamide adenine dinucleotide (oxidised) |
| $NADP^+$ | Nicotinamide adenine dinucleotide phosphate (oxidised) |
| NADH | Nicotinamide adenine dinucleotide (reduced) |
| NADPH | Nicotinamide adenine dinucleotide phosphate (reduced) |

| | |
|---|---|
| PLP | Pyridoxal phosphate |
| ppm | Parts per million |
| pTsOH | Para-toluenesulfonic acid |
| q | Quartet |
| qNMR | Quantitative nuclear magnetic resonance spectroscopy |
| $R_f$ | Retention factor |
| RT | Room temperature |
| s | Singlet |
| t | Triplet |
| ThDP | Thiamine diphosphate |
| TLC | Thin layer chromatography |
| TsOH | Toluenesulfonic acid (= pTsOH) |
| UPLC | Ultra Performance Liquid Chromatography |
| XRD | X-ray diffraction crystallography |
| $\delta$ | Chemical shift |

Commercial chemicals were used as received unless stated otherwise. Thin layer chromatography was performed on pre-coated plastic plates (Merck silica 60F254), and visualised using UV light and $KMnO_4$ dip. Proton ($^1H$) and carbon ($^{13}C$) NMR spectra were recorded on a Varian INOVA 300 MHz spectrometer. Chemical shifts are quoted relative to tetramethylsilane and referenced to residual solvent peaks as appropriate. Unless otherwise indicated, chiral HPLC analysis was performed using an Agilent 1200 HPLC system and data was processed using the Chemstation software or with a Varian semiprep/analytical HPLC using Galaxie software.

**Example 1**

**Preparation of 4-(2-methyl-1-propenyl)-5-morpholino-5*H*-2-furanone from 4-methyl-2-pentenal**

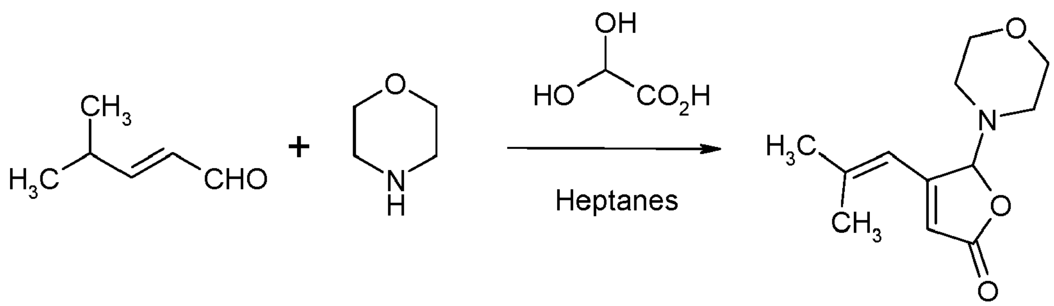


A 50% solution of glyoxylic acid in water (29.6g, 0.2mol) was added to a biphasic stirred mixture of morpholine (17.8g, 0.2mol) and heptanes (75mL), which had been pre-cooled to 0-10°C. The temperature was kept less than 10°C. The mixture was warmed to 20°C and 4-methyl-2-pentenal (19.6g, 0.2mol) was added. The mixture was stirred at 45°C for 20h. A large quantity of solid was formed. Water (100mL) was added at ambient temperature and the mixture stirred for 2h. The mixture was extracted with cyclopentyl methyl ether (100mL) and the organic solution washed twice with water (100mL) and concentrated to leave 30.4g of crude product. This solid was purified by recrystallisation from methanol (100mL) to afford 17.7g (40%) of pure 4-(2-methyl-1-propenyl)-5-morpholino-5*H*-2-furanone.
GC/MS: m/z = 223
$^1H$ NMR: $\delta$ 6.0 (s, 1H); 5.9 (s, 1H); 5.5 (s, 1H), 3.7 (d, 4H), 2.7 (d, 4H), 2.00 (s, 3H), 1.95 (s, 3H).

**Example 2**

**Preparation of 4-(4-methyl-1,3-pentadien-1-yl)morpholine**

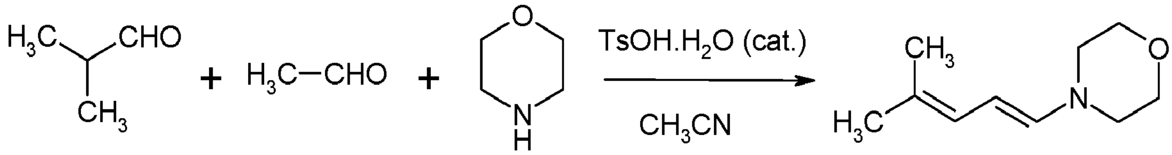


Isobutyraldehyde (46.90g, 0.65mol, 1.43eq.) was stirred in acetonitrile (300mL). Morpholine (56.63g, 0.65mol, 1.43eq.) followed by pTsOH (8.63g, 0.1 equiv) were slowly added at room temperature to the isobutyraldehyde solution. A solution of acetaldehyde (20g, 0.454mol) in acetonitrile (100mL) was added drop-wise over 1 hr with internal temperature monitoring at 50°C. After complete addition the mixture was stirred for 30min at 50°C and then cooled to room temperature before evaporation of the solvent *in vacuo* to give an orange oil (123.3g). Assay of the crude by quantitative NMR using benzyl benzoate as the internal standard was 40% which give 65% yield of the desired dienamine.
GC/MS: m/z = 167
$^1$H NMR: δ 6.01 (d, 1H); 5.69 (d, 1H); 5.31 (dd, 1H), 3.70 (m, 4H), 2.89 (m, 4H), 1.71 (s, 3H), 1. 63 (s, 3H).

**Example 3**

**Preparation of 4-(2-methyl-1-propenyl)-5-morpholino-5*H*-2-furanone from crude 4-(4-methyl-1,3-pentadien-1-yl)morpholine**

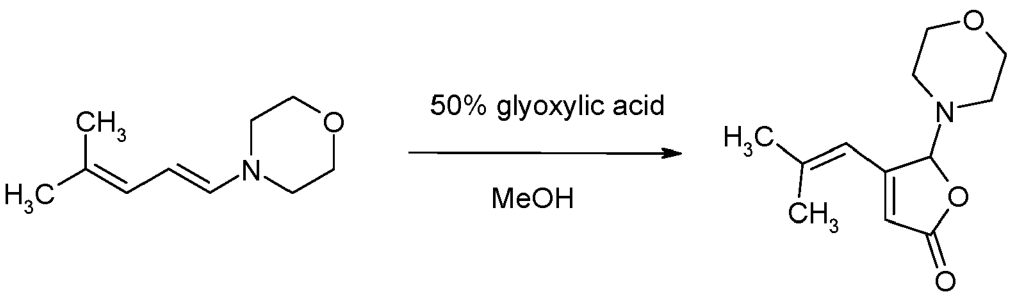


The crude 4-(4-methyl-1,3-pentadien-1-yl)morpholine dienamine of example 2 (123.3g, 40% assay) was dissolved in methanol (300mL) at room temperature. On complete dissolution, glyoxylic acid (50wt%, 60g, 1.1eq) was added and the resultant biphasic mixture stirred at 50°C for 18h. The reaction mixture was cooled to room temperature and the solvent removed by rotary evaporation. The residue was partitioned between ethyl acetate (200mL) and saturated sodium carbonate solution (200mL). The aqueous phase was extracted with ethyl acetate (100mL) and combined organics washed with brine and evaporated to a thick oil which solidified on standing (89.0g, 67% assay by qNMR, 60% yield).
GC/MS: m/z = 223
NMR: as example 1.

**Example 4**

**Preparation of 4-(2-methyl-1-propenyl)-5-morpholino-5*H*-2-furanone in one pot from isobutyraldehyde and acetaldehyde.**

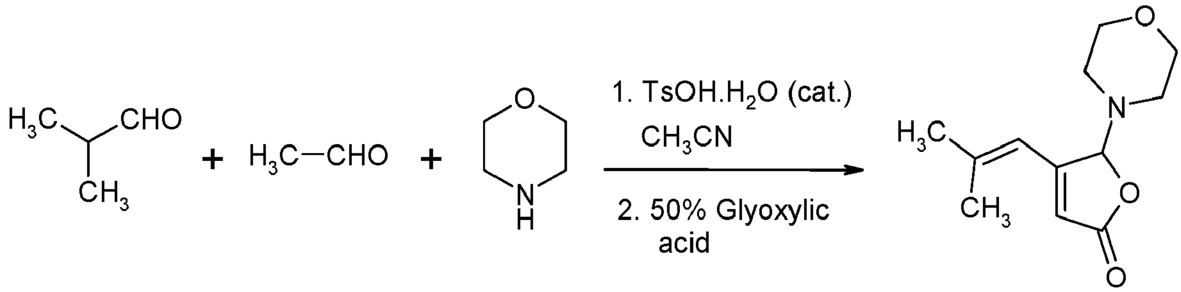


Isobutyraldehyde (102.90g, 1.43mol) was stirred in acetonitrile (600mL). Morpholine (124.3g, 1.43mol) followed by pTsOH (19.0g, 0.1 equiv) were slowly added at room temperature to the isobutyraldehyde solution. A solution of acetaldehyde (44.05g, 1.0mol) in acetonitrile (150mL) was added drop-wise over 1hr with internal temperature monitoring at 50°C. After complete addition the mixture was stirred for 30min at 50°C and then cooled to <10°C. Glyoxylic acid (50wt%, 211.3g, 1.43mol) was added and the resultant biphasic mixture stirred at 50°C for 18h. The reaction mixture was cooled to room temperature and the solvent removed by rotary evaporation. The residue was partitioned between ethyl acetate (1L) and saturated sodium carbonate solution (1L). The aqueous was extracted with ethyl acetate and combined organics washed with brine and evaporated to a thick oil which solidified on standing (166g). The crude product was triturated with MTBE at room temperature. The product is collected by filtration and washed with MTBE to give pure 4-(2-methyl-1-propenyl)-5-morpholino-5*H*-2-furanone (84g, 37% yield) identical to the material prepared in Example 1. Analysis of the crude solid (166g) indicated ca 50% yield.

**Example 5**

**Preparation of 1,4-bis-(4-methyl-1,3-pentadien-1-yl)piperazine**

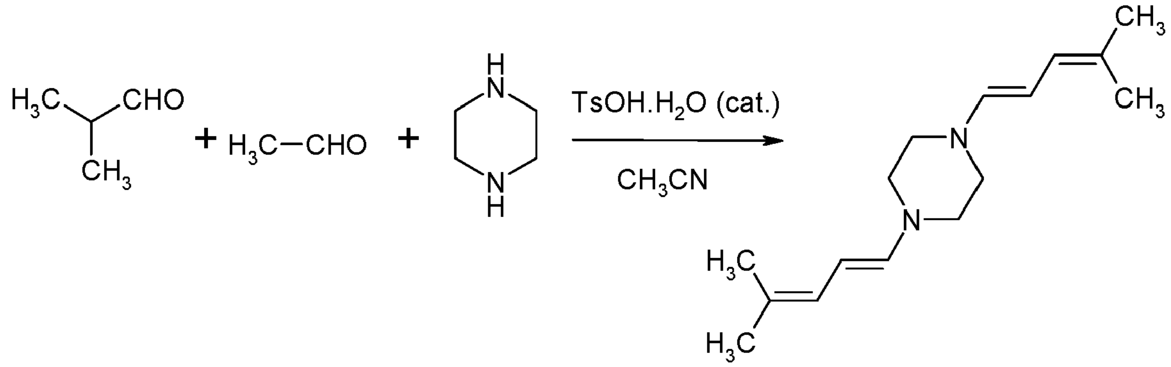


A solution of piperazine (43.0g, 0.5mol) and 4-toluenesulfonic acid monohydrate (4.4g, 0.023mol) in acetonitrile (600mL) was prepared. This solution was heated to 50°C and isobutyraldehyde (100mL, 1.10mol) added over 10 minutes. The solution went red-orange in colour and a transient white precipitate occurred. A solution of acetaldehyde (30.8g, 0.70mol) in acetonitrile (30 mL) was then added via syringe pump over 3h at 50°C. A suspension was formed which was stirred at 50°C for 0.5h. The solvent was removed and the residual solid isolated from methanol (500mL) at -5°C. It was filtered and washed with chilled methanol and dried to afford 52.9g (60%) of 1,4-bis-(4-methyl-1,3-pentadien-1-yl)piperazine.
GC/MS: m/z = 246.

$^1$H NMR: $\delta$ 6.00 (d, 2H); 5.70 (d, 2H); 5.28 (dd, 2H); 2.92 (s, 8H); 1.73 (s,6H); 1.68 (s, 6H). $^{13}$C NMR ($CDCl_3$): 140.6 (C), 126.3 (CH), 123.4 (CH), 100.2 (CH), 48.2 ($CH_2$), 25.8 ($CH_3$), 18.1 ($CH_3$).

**Example 6**

**Preparation of 5,5'-(piperazine-1,4-diyl)bis(4-(2-methylprop-1-en-1-yl)furan-2(5*H*)-one) from 1,4-bis-(4-methyl-1,3-pentadien-1-yl)piperazine.**

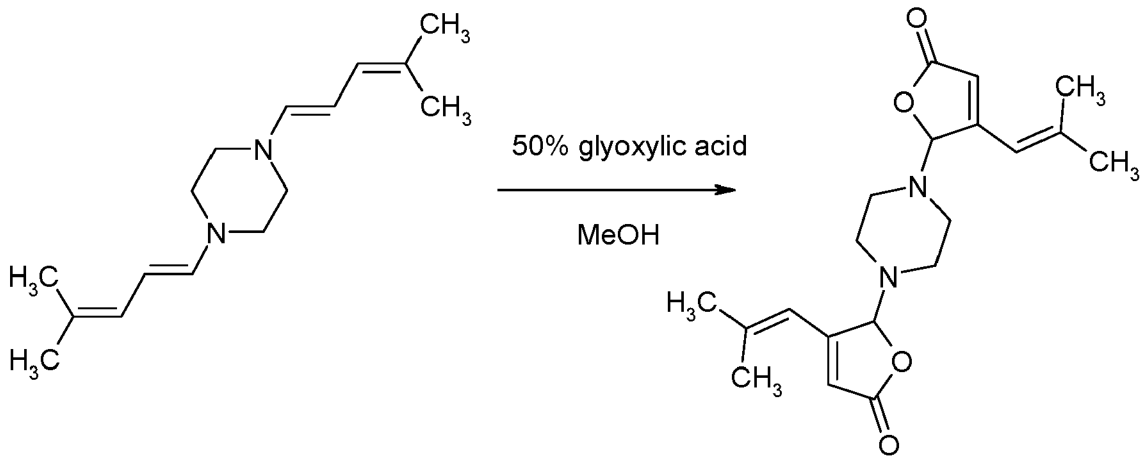


1,4-bis-(4-Methyl-1,3-pentadien-1-yl)piperazine (37.3g, 0.151mol; see Example 5) was charged to methanol (300mL). The temperature was adjusted to 34°C and a 50% solution of glyoxylic acid in water (44.9g, 0.302 mol) was added rapidly (over 5 minutes). The resulting suspension was stirred at 45°C for 15h; cooled to 0-10°C for 2h and filtered. The solid was washed with methanol (100mL) and dried to afford pure 5,5'-(piperazine-1,4-diyl)bis(4-(2-methylprop-1-en-1-yl)furan-2(5H)-one) (32.3g, 60%). An extra 5% could be obtained from the mother-liquor by concentration.

$^{1}H$ NMR: $\delta$ 5.96 (d, 2H), 5.87 (d, 2H), 5.56 (m, 2H), 2.71 (s, 8H), 2.07-1.90 (m,12H). $^{13}C$ NMR ($CDCl_3$): 172.3 (C), 159.2 (C), 150.9 (C), 116.6 (CH), 115.4 (CH), 99.2 (CH), 46.7 ($CH_2$), 28.2 ($CH_3$), 21.4 ($CH_3$).

This reaction can also be conducted in isopropanol, acetonitrile-water, toluene-water or in heptane water with similar yields.

**Example 7**

**Preparation of 5,5'-(piperazine-1,4-diyl)bis(4-(2-methylprop-1-en-1-yl)furan-2(5H)-one) in one pot from isobutyraldehyde and acetaldehyde.**

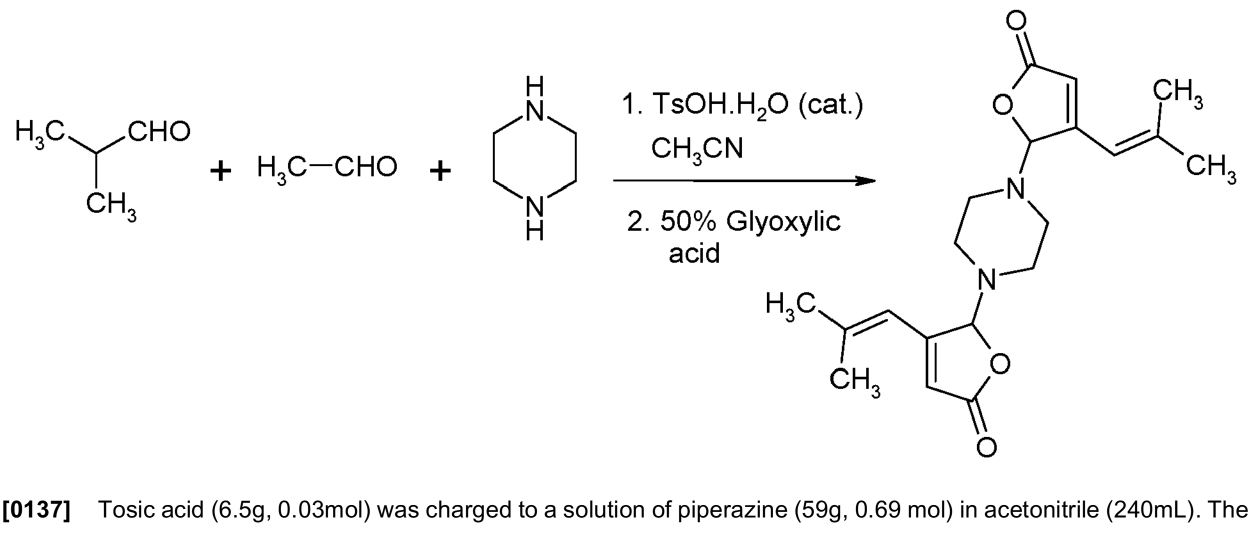


Tosic acid (6.5g, 0.03mol) was charged to a solution of piperazine (59g, 0.69 mol) in acetonitrile (240mL). The

reaction was heated to 50°C and agitated until dissolution of solids was observed, before addition of isobutyraldehyde (138mL, 1.51mol). The reaction was held at 50°C before a solution of acetaldehyde (60mL; 1.07mol) in acetonitrile (30mL) was charged to the vessel over 3hrs via syringe pump. On complete addition, the reaction was stirred for a further 30 minutes before a 50% w/w solution of glyoxylic acid in water (148g, 1.0 mol) was added over 5min to the reaction mixture followed by water (50ml). The reaction was then heated to 70°C for 2h, then to 50°C overnight. The reaction was then cooled to 5°C and held for 30 minutes. 5,5'-(Piperazine-1,4-diyl)bis(4-(2-methylprop-1-en-1-yl)furan-2(5H)-one) precipitated and was isolated by filtration and washed with acetonitrile (2 x 100mL) to give the desired product (126 g, 93.5% assay, 66% yield from acetaldehyde).

## Example 8

### Solvent free preparation of 5,5'-(piperazine-1,4-diyl)bis(4-(2-methylprop-1-en-1-yl)furan-2(5*H*)-one)

Isobutyraldehyde (79g, 100mL, 1.1mol) was charged to a 3-necked round bottom flask and argon was flushed through the system. Piperazine (43g, 0.5 mol) and TsOH-$H_2O$ (4.4g; 0.023 mol) were divided into 4 equal portions containing piperazine (10.75 g) and TsOH-$H_2O$ (1.1g). Addition of the first portion of piperazine (10.75g) resulted in exotherm from 24°C to 35°C. This was followed by the first portion of TsOH (1.1g). The reaction was agitated until all the piperazine had dissolved (t=35°C), after which the second portion of piperazine (10.75g) was added, followed by TsOH (1.1g) (t=41°C). Stirring was continued until all of piperazine had dissolved (t=41°C), then the third portion of piperazine (10.75g) was added, followed by TsOH (1.1g). After the 3rd charge a clear solution was generated and then the fourth portion of piperazine (10.75g) & TsOH-$H_2O$ were added, followed by TsOH (1.1g) (t=52°C). On complete addition the reaction was stirred at 50°C for 30 min.

Another flask (50mL) was charged with acetaldehyde (30.8g, 39mL, 0.7mol) and placed in an ice-water bath (0-2°C). The acetaldehyde flask was connected to the 3-necked flask by cannula via septa. A stream of argon was then passed through the acetaldehyde flask at the rate which ensures that the addition of the acetaldehyde was complete within 3 h. After all acetaldehyde was added, the reaction was stirred for 30 min at 50°C, then cooled to 40°C and 50% glyoxylic acid (104g, 78mL) was added dropwise over 45 min at a rate to keep temperature below 50°C. Water (100mL) was then added and the reaction was heated at 70°C for 5 h. The reaction was cooled to 4°C in an ice-water bath. The precipitated product was filtered and filter cake was washed with cold water (100 mL). After drying in vacuo at 50°C 88.9g (71%) of desired product was obtained. Precipitate contains 78.5% of the title compound (HPLC assay).

## Example 9

### Alternative preparation of 5,5'-(piperazine-1,4-diyl)bis(4-(2-methylprop-1-en-1-yl)furan-2(5*H*)-one) with simultaneous addition of both aldehydes

Piperazine (236.6g) was charged to a clean 3L dry vessel fitted with thermometer, addition funnel (100mL) and reflux condenser. pTsOH (25.3g) was charged to vessel followed by acetonitrile (550mL). The agitator was started and the vessel was inerted with nitrogen. Isobutyraldehyde (150mL, 27% of the total charge) was charged to the agitated piperazine / pTsOH slurry and a temperature rise to ca 43°C was observed. The white suspension was then heated to 50°C (+/- 5°C). A pre-mixed chilled solution of isobutyraldehyde (400mL, 73% of total charge) and chilled acetaldehyde (260mL) were charged to a clean, dry 1L vessel and this mixture was maintained in an ice bath. The isobutyraldehyde / acetaldehyde mixture was charged to the contents of the 3L vessel in 100mL aliquots over 5-6h at 50°C. The contents of the reaction flask changed from a white suspension to a wine red solution and finally an orange suspension during the addition of the acetaldehyde. After complete addition the suspension was agitated at 50°C for ca. 0.5 to 1.0h.

Aqueous glyoxylic acid (50% wt/wt) solution was charged to the suspension over 10-15 min and the temperature rose to ca 75°C. 5,5'-(Piperazine-1,4-diyl)bis(4-(2-methylprop-1-en-1-yl)furan-2(5H)-one) was observed to crystallise from solution. The suspension was stirred at 70°C (+/ 5°C) for 6h, then cooled with stirring to ambient. The batch was then cooled to -5 to 0°C for and held for 3-4h before the suspension was filtered and the cake washed with chilled methanol (1 x 500mL) then 2 x 500mL of methanol at ambient temperature. The washed product was dried in a vacuum oven at 40-50°C to constant weight to afford 474g of 98% pure 5,5'-(piperazine-1,4-diyl)bis(4-(2-methylprop-1-en-1-yl)furan-2(5H)-one) (70%).

**Example 10**

**Preparation of 5-hydroxy-4-(2-methylprop-1-en-1-yl)furan-2(5*H*)-one from 4-(2-methyl-1-propenyl)-5-morpholino-2(5*H*)-furanone or 5,5'-(piperazine-1,4-diyl)bis(4-(2-methylprop-1-en-1-yl)furan-2(5*H*)-one).**

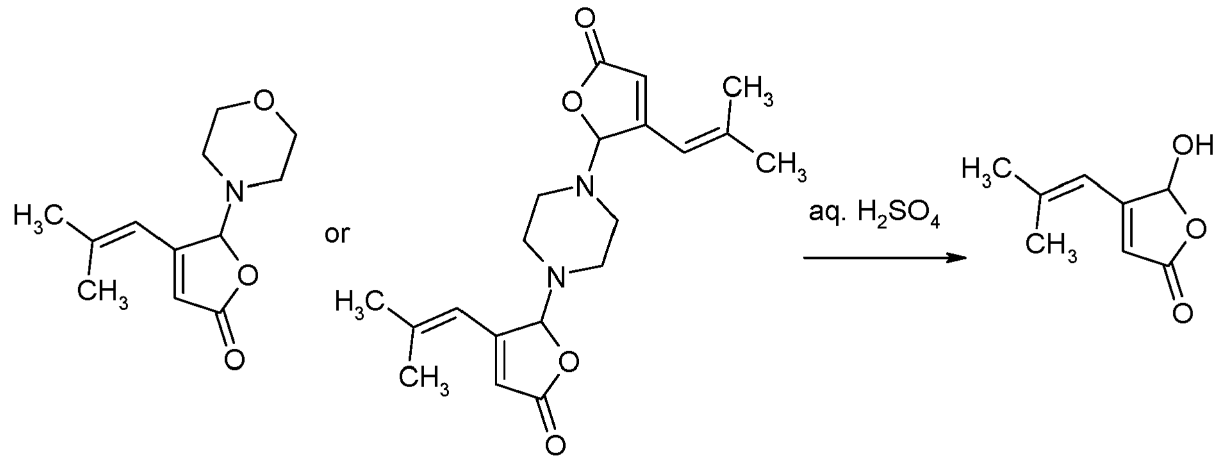


A 10% w/w aqueous sulphuric acid solution (110g) was charged to 4-(2-methyl-1-propenyl)-5-morpholino-2(5*H*)-furanone (20.0g, 0.896mol) and the mixture was stirred at reflux for 4h or until TLC (100:3 CPME: HOAc) indicated reaction completion. The mixture remained a suspension at all times. It was then cooled to 5°C, held for 2h, and filtered. The white solid was washed with water and dried to afford 5-hydroxy-4-(2-methylprop-1-en-1-yl)furan-2(5*H*)-one (12.9g, 93%).

Alternatively, a 10% w/w aqueous solution of sulphuric acid (412g) was charged to a vessel containing 5,5'-(piperazine-1,4-diyl)bis(4-(2-methylprop-1-en-1-yl)furan-2(5*H*)-one) (60g, 0.167mol). The contents were agitated and then heated to reflux. The reaction was held at reflux until the starting material was consumed, which was determined by dissolution. On completion of reaction the batch was cooled to 35°C at which point the target compound began to crystallise. The slurry was further cooled to 0-5°C and then transferred to a filter. The product was filtered, washed with water (2 x 100mL) and then dried under vacuo at <50°C, to give 5-hydroxy-4-(2-methylprop-1-en-1-yl)furan-2(5*H*)-one (42.0g, 81%).
GC/MS: m/z = 154

$^1$H NMR ($CDCl_3$): $\delta$ 6.10 (s, 1H); 5.92 (s, 1H); 5.88 (s, 1H); 5.35 (bs, 1H, O-H); 1.98 (s,3H); 1.93 (s, 3H). $^{13}$C NMR ($CDCl_3$): 172.8 (C), 161.4 (C), 152.3 (C), 115.3 (CH), 114.9 (CH), 99.5 (CH), 28.2 ($CH_3$), 21.4 ($CH_3$).

**Example 11**

**Preparation of 5-hydroxy-4-(2-methylprop-1-en-1-yl)furan-2(5H)-one in one pot from isobutyraldehyde and acetaldehyde.**

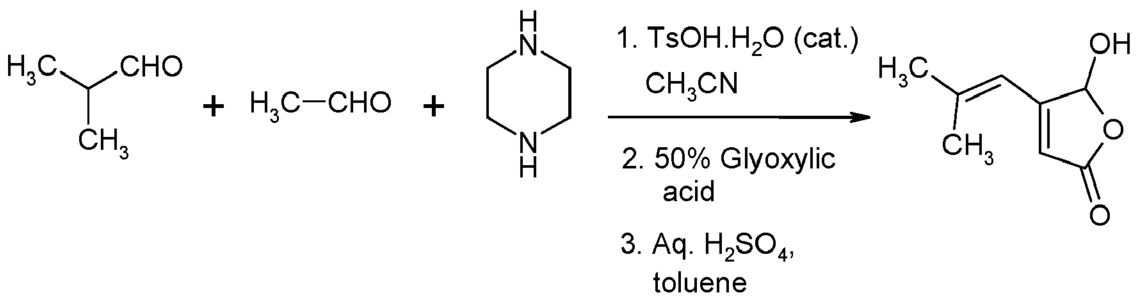

A mixture of piperazine (43.0g, 0.5 mol) and isobutyraldehyde (200mL) were refluxed under Dean-Stark until the theoretical amount of water (18mL) collected. The excess isobutyraldehyde was distilled off to leave a crystalline mass of 1,4 bis(2-methylpropen-1-yl)piperazine. This was dissolved in acetonitrile (1.2L) and extra isobutyraldehyde (100 mL, 1.0mol) added. Extra piperazine (4.4g) was added followed by p-toluenesulfonic acid (4.4g, 23.2mmol). The temperature was adjusted to 40°C and a solution of acetaldehyde (44g, 1.0mol) in acetonitrile (40mL) was added over 4h. The reaction mixture was stirred at 40°C for 1h and at ambient temperature for 4h. The acetonitrile was distilled off and the residue suspended in toluene (400mL). A mixture of 50% glyoxylic acid (148g) and water (150mL) was added over 0.5h. The mixture was then stirred at 45°C for 15h. There was a solid precipitate. Dilute sulphuric acid (10%, 1L) was added and the mixture refluxed for 3h. A biphasic mixture resulted. The toluene phase was separated. It contained ca 50% yield (based on acetaldehyde) of 5-hydroxy-4-(2-methylpropen-1-yl)-2-furanone by analysis

**<u>Example 12</u>**

**<u>Preparation of 5-methoxy-4-(2-methylprop-1-en-1-yl)furan-2(5H)-one</u>**

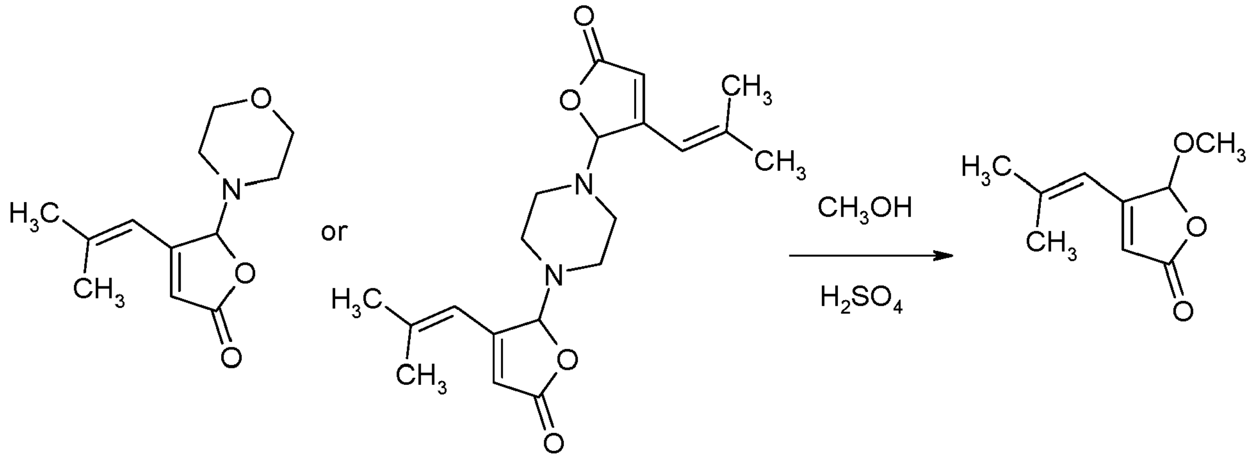


4-(2-Methyl-1-propenyl)-5-morpholino-2(5*H*)-furanone (7.70g, 34.5mmol) was stirred in MeOH (50mL) with $H_2SO_4$ (4mL, 2.2eq.) at reflux for 3h until disappearance of the starting material by GC. The reaction mixture was cooled to room temperature. The solvent was then evaporated *in vacuo* and the residue was diluted with EtOAc (50mL). The organic phase was washed with $H_2O$ (3x 50mL). Solvent was then evaporated *in vacuo* to give 5-methoxy-4-(2-methylprop-1-en-1-yl)furan-2(5H)-one as an orange oil (5.50g, 95%) which can be used without purification.

Alternatively, 5,5'-(piperazine-1,4-diyl)bis(4-(2-methylprop-1-en-1-yl)furan-2(5*H*)-one) (130g, 363mmol) and MeOH (690mL) were charged in 2L round bottom flask with mechanical agitation to form a suspension. Concentrated $H_2SO_4$ (43mL, 762mmol) was added dropwise with stirring at 20-25°C - a slight exotherm was observed, and nature of the solids present changed from dispersed and easily mixed to a thicker slurry. The reaction mixture was refluxed for 5h. After 0.5h complete dissolution (orange liquid) was observed. After 5h LCMS showed ~99% of the desired product. The mixture was cooled to ambient and left for crystallization of piperazine sulfate. The sediments were filtered, and filter cake was washed with ice cold MeOH (400mL). The filtrate was concentrated *in vacuo* (40°C bath temperature), and the residue was partitioned between water (50mL) and MTBE (300mL). Water layer was separated and additionally extracted (2x300mL of MTBE). Combined organic extracts were washed with saturated aq. $NaHCO_3$ (200mL). MTBE layer was dried over $Na_2SO_4$ with stirring for 1.5h, filtered and evaporated to give 5-methoxy-4-(2-methylprop-1-en-1-yl)furan-2(5H)-one as an orange oil (120g, 98%).

Distilled at 0.2-0.3 mbar vacuum with bp 100-105°C. GC/MS: m/z = 168; $^1$H NMR (400 MHz, $CDCl_3$) $\delta$ 5.59 (1 H, s) 5.88-5.86 (1 H, m) 5.75 (1H, d, J=0.6) 3.53 (3H, s) 2.01-2.00 (3H, m) 1.97-1.96 (3H, m). $^{13}$C NMR ($CDCl_3$): 171.4 (C), 159.2 (C), 151.9 (C), 115.8 (CH), 115.2 (CH), 104.4 (CH), 56.1 ($CH_3$), 28.2 ($CH_3$), 21.4 ($CH_3$).

The following compounds were prepared from 4-(2-methyl-1-propenyl)-5-morpholino-2(5*H*)-furanone according to the first of the above methods by replacing methanol with 3-methylbutanol (isoamyl alcohol), *n*-pentanol (amyl alcohol) or *n*-butanol:

**Example 12A**

**5-(3-Methylbutoxy)-4-(2-methylprop-1-en-1-yl)furan-2(5H)-one**

Distilled at 0.4 mbar vacuum with bp 139-140°C; GC/MS: m/z = 224; $^1$H-NMR (400 MHz, $CDCl_3$) δ 5.94 (1H, s) 5.87-5.84 (1H, m) 5.80 (1H, s) 3.86-3.80 (1H, m) 3.70-3.64 (1H, m) 2.00 (3H, s) 1.96 (3H, s) 1.77-1.66 (1H, m) 1.57-1.50 (2H, m) 0.93-0.91 (3H, m) 0.91-0.89 (3H, m)

**Example 12B**

**4-(2-Methylprop-1-en-1-yl)-5-pentyloxyfuran-2(5H)-one**

Distilled at 0.08 mbar vacuum with bp 123-134°C; GC/MS: m/z = 224; $^1$H-NMR (400 MHz, $CDCl_3$) δ 5.94 (s, 1H); 5.86 (s, 1H); 5.80 (s, 1H); 3.84-3.75 (m, 1H); 3.68-3.59 (m, 1H); 2.01 (s, 3H); 1.95 (s, 3H); 1.69-1.59 (m, 2H); 1.38-1.29 (m, 4H); 0.95-0.85 (m, 3H)

**Example 12C**

**5-Butoxy-4-(2-methylprop-1-en-1-yl)furan-2(5H)-one**

Distilled at 0.05 mbar vacuum with bp 136-146°C; GC/MS: m/z = 210; $^1$H-NMR (400 MHz, $CDCl_3$) δ 5.94 (s, 1H); 5.86 (s, 1H); 5.80 (s, 1H); 3.85-3.76 (m, 1H); 3.69-3.60 (m, 1H); 2.00 (s, 3H); 1.95 (s, 3H); 1.68-1.58 (m, 2H); 1.45-1.32 (m, 2H); 0.93 (t, 3H, J=7.4 Hz)

**Example 13**

**Preparation of 5-methoxy-4-(2-methylpropyl)-dihydrofuran-2(3H)-one**

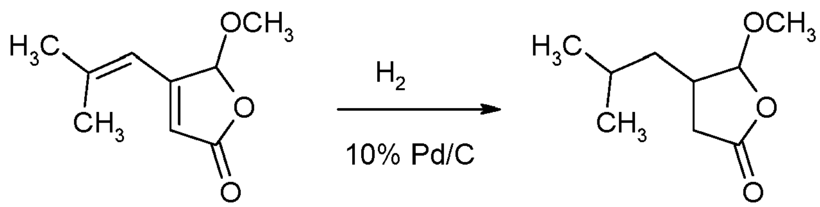


A solution of 5-methoxy-4-(2-methylprop-1-en-1-yl)furan-2(5H)-one (100 g, see example 12) in MTBE (700mL), 5g (5 wt%) of 10% Pd/C was charged to a hydrogenation vessel. 1 atm of hydrogen gas was introduced and pressure was kept constant during the reaction. After 7h at 20°C, the reaction was filtered through a celite pad (ø60mm, H=30mm) and rinsed with MTBE (3x50mL). The filtrate was washed with 1M $NaHCO_3$ solution (200mL), water, brine and dried on $Na_2SO_4$. After solvent removal 5-methoxy-4-(2-methylpropyl)-dihydrofuran-2(3H)-one was obtained as colourless liquid (98g, 96%).
GC/MS: m/z = 172
$^1$H NMR (400 MHz, $CDCl_3$) δ 5.25 (d, *J* = 5.0 Hz, 0.53 H, major isomer), 5.04 (d, *J* = 2.4 Hz, 0.26H, minor isomer), 3.48 (s, 0.92H, minor isomer), 3.46 (s, 1.66H, major isomer), 2.78 (dd, J = 17.7, 8.6 Hz, 0.33H), 2.59 - 2.42 (m, 1.27H, major isomer), 2.42-2.34 (m, 0.33H, minor isomer), 2.29 (dd, *J* = 16.7, 11.8 Hz, 0.63H, major isomer), 2.15 (dd, *J* = 17.7, 4.6 Hz, 0.33H, minor isomer), 1.67 - 1.31 (m, 2.63H both isomers), 1.28 - 1.19 (m, 0.33H, minor isomer), 0.95 - 0.86 (m, 6H both isomers). $^{13}$C NMR ($CDCl_3$): 177.7 (C, major isomer), 176.04 (C, minor isomer), 110.1 (CH, minor isomer), 106.1 (CH, major isomer), 57.0 ($CH_3$, minor isomer), 56.6 ($CH_3$, major isomer), 41.2 ($CH_2$, minor isomer), 39.1 ($CH_2$, minor isomer), 38.3 ($CH_2$, major isomer), 37.1 ($CH_2$, major isomer), 33.9 (CH, minor isomer), 32.8 (CH, major isomer), 26.0 (CH, major isomer), 25.8 (CH, minor isomer), 23.0 ($CH_3$, major isomer), 22.9 (minor isomer), 22.7 (major isomer), 22.6 (minor isomer).

Using the same general method and starting from the compounds of examples 12A, 12B and 12C respectively, the following compounds were also obtained:

**Example 13A**

**5-(3-Methylbutoxy)-4-(2-methylpropyl)-dihydrofuran-2(3H)-one**

GC/MS: m/z = 228; $^1$H-NMR (400 MHz, $CDCl_3$) δ 5.35 (0.86H, d, J=5.0 Hz) 5.13 (0.14H, d, J=2.7 Hz) 3.87-3.79 (m, 1H) 3.57-3.45 (m, 1H) 2.58-2.37 (2H, m) 2.35-2.27 (0.79H, m) 2.20-2.11 (0.21H, m) 1.74-1.62 (1H, m) 1.61-1.44 (4H, m) 1.42-1.31 (1H, m) 0.95-0.86 (12H, m)

**Example 13B**

**4-(2-Methylpropyl)-5-pentyloxydihydrofuran-2(3H)-one**

GC/MS: m/z = 228; $^1$H-NMR (400 MHz, $CDCl_3$) δ 5.35 (0.77H, d, J=5.0 Hz) 5.13 (0.23H, d, J=2.6 Hz,) 3.83-3.75 (1 H, m) 3.55-3.40 (1 H, m) 2.59-2.37 (2H, m) 2.32 (0.73H, dd, J=16.6, 11.8 Hz) 2.15 (0.27H, dd, J=17.7, 5.0 Hz) 1.67-1.45 (4H, m) 1.41-1.25 (5H, m) 0.96-0.86 (9H, m)

**Example 13C**

**5-Butoxy-4-(2-methylpropyl)-dihydrofuran-2(3H)-one**

GC/MS: m/z = 214; $^1$H-NMR (400 MHz, $CDCl_3$) δ 5.35 (0.8H, d, J=5.0 Hz) 5.13 (0.2H, d, J=2.6 Hz,) 3.85-3.75 (1H, m) 3.56-3.41 (1H, m) 2.59-2.38 (2H, m) 2.31 (0.76H, dd, J=16.5, 11.7 Hz) 2.15 (0.24H, dd, J=17.6, 5.0 Hz) 1.66-1.45 (4H, m) 1.43-1.30 (3H, m) 0.95-0.87 (9H, m)

**Example 14**

**Preparation and hydrogenation of 5-(L-Menthyloxy)-4-(2-methyl-1-propenyl)-2(5H)-furanone**

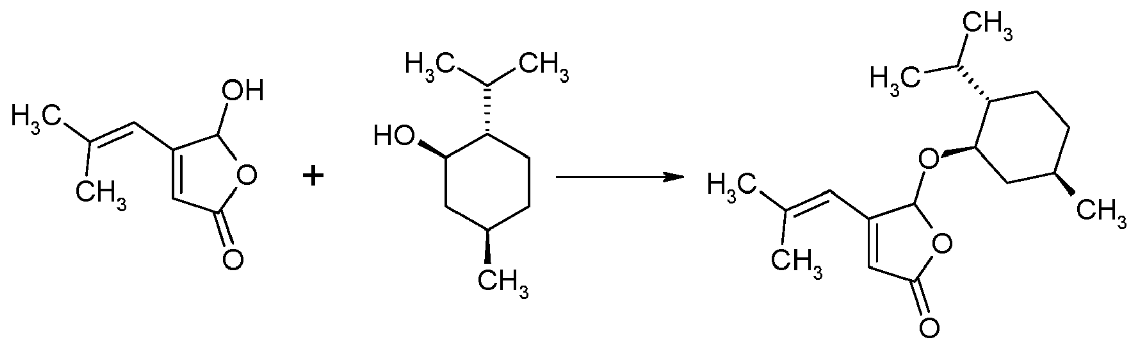

A mixture of 5-hydroxy-4-(2-methyl-1-propenyl)-2-furanone (59.5g, 0.386 mol), L-menthol (89.5g, 1.5 equivalents) and methanesulfonic acid (1.5g) was stirred at 70-80°C under vacuum (to remove water) for 100h. The liquid mass was poured (hot) into acetonitrile (450mL) and the title compound isolated by cooling, filtration and washing. The yield was 76.2g (67%). Suitable crystals for XRD were grown by slow evaporation of an acetone solution. The configuration at the acetal carbon was (R).

Hydrogenation of 5-(L-menthyloxy)-4-(2-methyl-1-propenyl)-2-furanone in ethyl acetate using the conditions of Example 13, gave the saturated derivative in quantitative yield. By $^1$H NMR, the compound was a 1:1 mixture of diastereomers.

**Example 15**

**Preparation of 5-acetoxy-4-(2-methylprop-1-en-1-yl)furan-2(5*H*)-one**

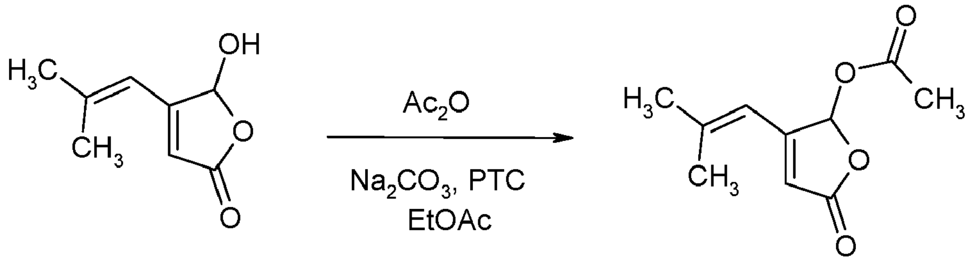


A suspension of 5-hydroxy-4-(2-methylprop-1-en-1-yl)furan-2(5*H*)-one (19g, 0.123mol) in ethyl acetate (100mL) was treated with solid sodium carbonate (13.1g, 0.123mol) and tetrabutylammonium hydrogensulfate (0.5g). Acetic anhydride (18.8g, 1.5eq.) was added in one portion. A mildly exothermic reaction ensued. The mixture was stirred overnight, water (100mL) added and the organic phase separated (pH of aqueous phase was 6.0). The ethyl acetate phase was water washed and concentrated to leave a solid (24.6g, 100%). This was pure by TLC (100:3 CPME: acetic acid). If this reaction is carried out in isopropyl acetate, the pure compound can be isolated by cooling the isopropyl acetate solution after the water washing in about 70% yield.
m/z: 196

## Example 16

### Hydrogenation of 5-acetoxy-4-(2-methylprop-1-en-1-yl)furan-2(5*H*)-one

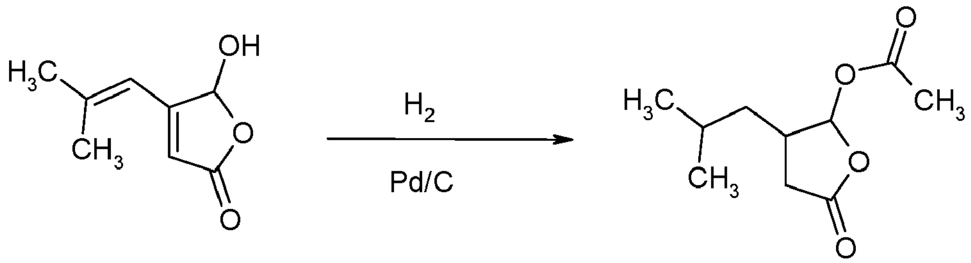


The material of example 34 was hydrogenated under similar conditions to those outlined in Example 13 to give a >90% yield of 5-acetoxy-4-(2-methylpropyl)-3,4-dihydro-2(5*H*)-furanone as a 1:1 mixture of diastereomers. The product is accompanied by ca. 5% of 4-(2-methylpropyl)-3,4-dihydrofuran-2(5*H*)-one. The product can be hydrolysed as in Example 30 to give yields of 5-hydroxy-4-(2-methylpropyl)-3,4-dihydro-5*H*-2-furanone/ 3-formyl-5-methylhexanoic acid.

## Example 17

### Preparation of 5-hydroxy-4-(2-methylpropyl)dihydrofuran-2(3H)-one ($I^A$) by hydrogenation of 5-hydroxy-4-(2-methylprop-1-en-1-yl)furan-2(5H)-one in alkaline solution

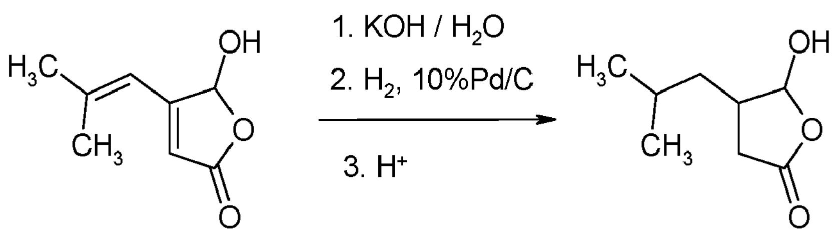


5-Hydroxy-4-(2-methylpropen-1-yl)-2-furanone (3.35g, 21.7mmol) and water (20mL) were charged to a hydrogenator. Potassium hydroxide (1.21g, 1 eq) was then charged to the vessel and the contents were heated to 40°C until

dissolution had occurred. 10% Pd/carbon catalyst (0.67g) was charged to the vessel and the reactor contents were then hydrogenated at 40°C and 5barg hydrogen pressure. On completion of reaction the reaction was cooled and the catalyst removed by filtration. The pH was adjusted to pH 2 by addition of 36% hydrochloric acid and the aqueous layer was washed with toluene to extract the desired product. The combined toluene extracts were concentrated to give the title compound (3.17g, 92%).

**Example 18**

**Hydrogenation of 5-hydroxy-4-(2-methylprop-1-en-1-yl)furan-2(5H)-one in neutral solution,**

5-Hydroxy-4-(2-methylpropen-1-yl)-2-furanone was hydrogenated in 6mL of water per gram starting material with 10% w/w of Pd/C catalyst. (22h, 40°C, 10 bar). On filtration, an oily phase separated from the water. This was a 1:1 mixture of 5-hydroxy-4-(2-methylpropyl)dihydrofuran-2(3H)-one (**I**$^{A}$) and 4-(2-methylpropyl)-dihydrofuran-2-one. Compound (**I**$^{A}$) can easily be separated pure by an acid base extraction. This reaction was repeated in organic solvents with 2-propanol giving relatively pure (**I**$^{A}$).

**Example 19**

**Preparation of 5-hydroxy-4-(2-methylpropyl)dihydrofuran-2(3H)-one (I$^{A}$) from 5,5'-(piperazine-1,4-diyl)bis(4-(2-methylprop-1-en-1-yl)furan-2(5*H*)-one) in one pot**

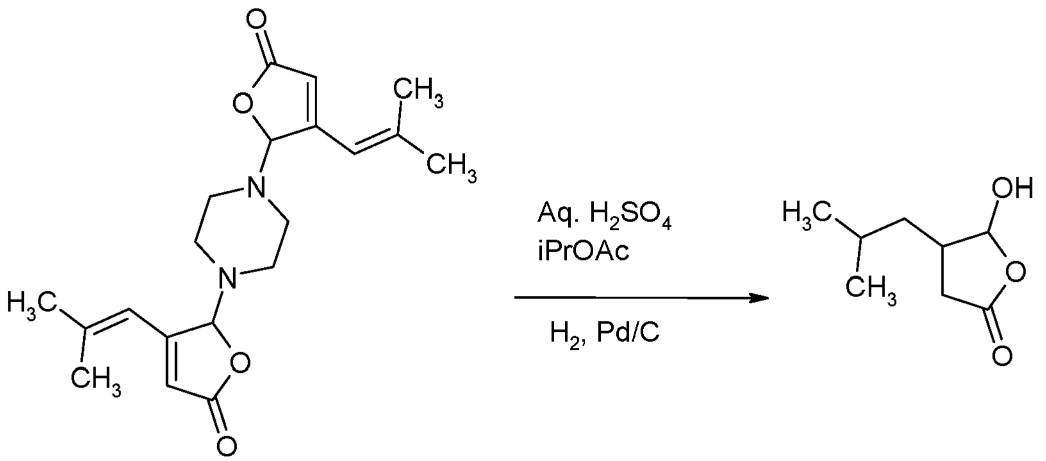


5,5'-(Piperazine-1,4-diyl)bis(4-(2-methylprop-1-en-1-yl)furan-2(5*H*)-one) (50.0g, 0.14mol) was charged to water (300mL) containing sulphuric acid (16.0g, 0.163mol). Isopropyl acetate (200mL) was added. 50% water wet 5% palladium on carbon (3.0g) was added and the mixture was hydrogenated (5bar hydrogen) at ambient temperature for 15h. The reaction mixture was filtered from catalyst, washed with isopropyl acetate (300mL) - this was also used to wash out the vessel. The organic phase was separated, washed with water (100mL) and concentrated on a rotary evaporator to afford 38.8g of clear oil. This was a mixture of desired compound (**I**$^{A}$) and the over-reduced lactone (4-(2-methylpropyl)-dihydrofuran-2-one). It was readily purified by extraction with aqueous potassium carbonate solution and wash with toluene. Acidification of the potassium carbonate extract with formic acid gave the desired product (**I**$^{A}$) (25.6 g, 58%). $^{1}H$ NMR ($D_2O$ with added $K_2CO_3$): δ 9.5 (1H), 2.8 (m, 2H), 2.40 (m,2H), 1.55 (m, 2H), 1.30 (m, 2H), 0.95 (m, 6H).

**Example 20**

**Preparation of 3-isobutylidene-2-oxopentanedioic acid mono-potassium salt.**

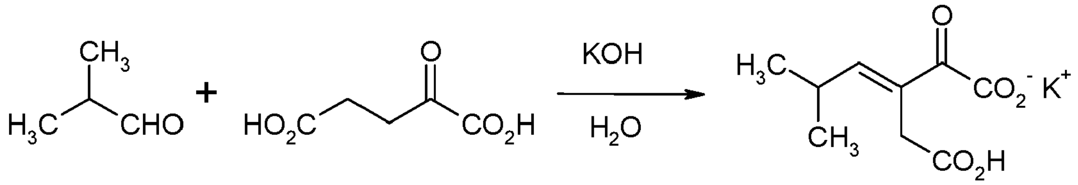


A 2L round bottom was charged 300g α-ketoglutaric acid and 450mL of ice water. With stirring and cooling 314g of potassium hydroxide in 450mL water was charged keeping the pot temperature less than 25°C. Reactor was placed under nitrogen and isobutyraldehyde was added at 0.2mL/min over 50hrs. The resulting two phase yellow solution was separated and washed with 100mL MTBE. The lower aqueous phase pH was adjusted to 3.2-3.4 and was concentrated to 1/3 volume at 65°C under vacuum. After cooling to <5°C the resulting solids were collected and washed with several small volumes of water to give after drying 230g of a white solid containing 60% 3-isobutylidene-2-oxopentanedioic acid mono-potassium salt and remainder KCl.
$^{1}H$ NMR ($D_2O$ at pH 8-10, 400MHz) δ 6.4 (d, 1H), 3.3 (s, 2H), 2.7 (m, 1H), 0.96 (d, 6H). $^{13}C$ NMR ($D_2O$ at pH 8-10, 400MHz) δ 23 ($2CH_3$), 31 (CH), 35 ($CH_2$), 133 (C), 164 (CH), 170 (C), 176 (C), 182 (C).

## Example 21

### Preparation of 3-(2-methylpropyl)-2-oxopentanedioic acid mono-potassium salt

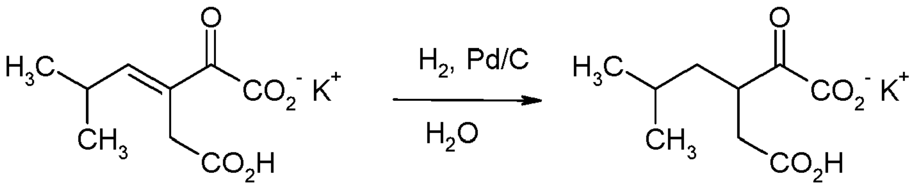


An aqueous solution of 3-isobutylidene-2-oxopentanedioic acid mono-potassium salt as generated in example 20 was adjusted to pH 6-8 and 5% Pd/C was added and the solution was hydrogenated at 10bar $H_2$ 25°C for 10hours. The catalyst was removed by filtration and the pH of the solution adjusted to pH 3.8. The mixture was cooled to <5°C and filtered and washed with a small amount of cold water. The resulting white crystals were dried at 40°C under vacuum to give the title compound, 286g, 58% yield over 2 steps.
$^{1}H$ NMR ($D_2O$ at pH 8-10, 400MHz) δ 3.5 (m, 1H), 2.5 (dd, 1H), 2.3 (dd, 1H), 1.6-1.5 (m, 2H) 1.3-1.2 (m, 1H) 0.9 (d, 6H). $^{13}C$ NMR ($D_2O$ at pH 8-10, 400MHz) δ 21.7 ($CH_3$), 22.2 ($CH_3$), 25.5 (CH), 38.4 ($CH_2$), 39.3 ($CH_2$), 43.4 (CH), 167.8 (C), 170.6 (C), 180.7 (C).

## Example 22

### Expression of Decarboxylase Enzymes in *E. coli*

PubMed was used to search the literature for decarboxylase enzymes with a broad spectrum of activity. The KEGG (Kyoto Encyclopedia of Genes and Genomes) program was used to search for microbial decarboxylases with activity on compounds somewhat similar in structure to 3-(2-methylpropyl)-2-oxopentanedioic acid or 3-isobutylidene-2-oxopentanedioic acid. Seven classes of decarboxylases were chosen for investigation based on reports of activity on compounds with some structural similarity. The decarboxylase classes were glutamate decarboxylase, diaminopimelate decarboxylase, indolepyruvate decarboxylase, branched-chain α-keto acid decarboxylase, aromatic-L-amino-acid decarboxylase, lysine decarboxylases and benzoylformate decarboxylase. Forty one sequences of genes for proven, or putative decarboxylase enzymes were selected. The genes were codon optimized for expression in E. *coli*, synthesized by GeneArt (Germany), DNA2.0 (Menlo Park, CA USA), or Blue Heron Biotechnology (Bothell, WA USA), cloned into expression vector pSTRC52 (Pfizer Inc., USA), and put into the expression strain *E. coli* BDG62 (Pfizer Inc., USA) (Table 1 below). Two genes were amplified from genomic DNA from the appropriate organisms by PCR and cloned into the same expression vector and *E. coli* strain. Four decarboxylase enzymes were purchased from Sigma and tested for activity on 3-(2-methylpropyl)-2-oxopentanedioic acid.
Each *E. coli* strain containing the cloned decarboxylase gene was grown overnight in LB broth with the appropriate antibiotic. A small amount (50μL - 100μL) of the overnight seed culture was used to inoculate 4.0mL of Terrific

Broth medium with appropriate antibiotics in 20mm diameter culture tubes. The cultures were grown in a shaking incubator at 32°C and 300rpm. After 5h of growth IPTG was added to 0.4mM final concentration to induce the enzyme expression. The cultures were returned to the incubator and grown for an additional 19h.

## Example 23

**Decarboxylation of 3-(2-methylpropyl)-2-oxopentanedioic acid potassium salt and 3-isobutylidene-2-oxopentanedioic acid potassium salt with Decarboxylase Enzymes**

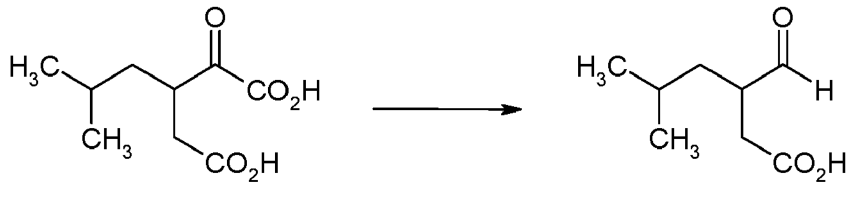

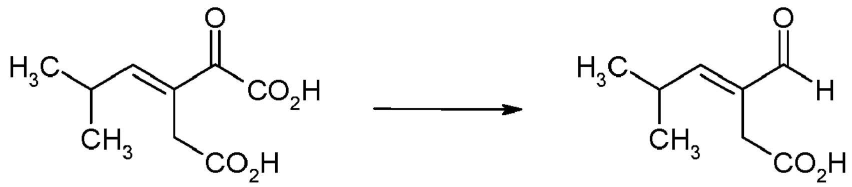

Recombinant decarboxylase enzymes were tested for decarboxylation of 3-(2-methylpropyl)-2-oxopentanedioic acid (**XV**) and 3-isobutylidene-2-oxopentanedioic acid (**XII**) using *E. coli* cells prepared as described in Example 22. Reactions (1mL) were carried out at 37°C in potassium phosphate buffer (100mM, pH 6.4) with decarboxylase (60mg wet cells), 50mM 3-(2-methylpropyl)-2-oxopentanedioic acid or 3-isobutylidene-2-oxopentanedioic acid, ThDP (0.1mM), and $MgSO_4$ (2.5mM). Decarboxylation of 3-(2-methylpropyl)-2-oxopentanedioic acid and 3-isobutylidene-2-oxopentanedioic acid was determined by a UPLC assay. An aliquot (0.1mL) of the reaction was treated with 0.5mL of potassium phosphate buffer (100mM, adjusted to pH 2.2 with phosphoric acid) and 0.3mL of a solution of 2,4-dinitrophenylhydrazine (20mM in 1M HCl:acetonitrile, 3:1, v/v) for 30min at 50°C. The derivatized samples were diluted with 0.5mL of acetonitrile, filtered and analyzed by UPLC on a Agilent Eclipse Plus C18 column (100mm x 3.0mm, 1.8μm) eluted with 0.1% trifluoroacetic acid in water:acetonitrile (55:45, v/v) at 1.1mL/min. The column was maintained at 40°C and the effluent was monitored at 360nm and $ES^+$ mass spectroscopy. Positive results were indicated by the presence of a peak for 3-formyl-5-methylhexanoic acid from 3-(2-methylpropyl)-2-oxopentanedioic acid or 3-formyl-5-methylhex-3-enoic acid from 3-isobutylidene-2-oxopentanedioic acid. Results of the analyses are shown in Table 1.

**Table 1**

| Enzyme Name | Source Organism | Reaction on (XV) | Reaction on (XII) |
|---|---|---|---|
| **Glutamate decarboxylases** | | | |
| Aa gad | *Arthrobacter aurescens* | -[a] | NT[b] |
| Rs gad | *Rhodococcus* sp. RHA1 | - | NT |
| Sc gad | *Streptomyces coelicolor* | - | NT |
| Dz gad | *Dickeya zeae* | - | NT |
| Pa gad | *Pyrobaculum arsenaticum* | - | NT |
| Mm gad | *Mycobacterium marinum* | - | NT |
| UT gad | UTI89 metagenomic DNA | - | NT |
| Rm gad | *Ralstonia metallidurans* | - | NT |
| Re gad | *Ralstonia eutropha* | - | NT |
| Xn gad | *Xenorhabdus nematophila* | - | NT |

(continued)

| Enzyme Name | Source Organism | Reaction on (XV) | Reaction on (XII) |
|---|---|---|---|
| **Glutamate decarboxylases** | | | |
| Pd gad | *Photobacterium damselae* | - | NT |
| **Branched-chain $\alpha$-Keto Acid decarboxylase** | | | |
| Pa bkd | *Photorhabdus asymbiotica* | - | - |
| Sv bkdA | *Streptomyces virginiae* | - | - |
| Ll kdcA | *Lactococcus lactis* | + | + |
| **Indolepyruvate decarboxylases** | | | |
| Ss ipd | *Staphylococcus saprophyticus* | - | + |
| Dd ipd | *Desulfovibrio desulfuricans* | + | + |
| Bm ipd | *Bacillus megaterium* | - | - |
| Dv ipd | *Desulfovibrio vulgaris* | - | - |
| Aa ipd | *Aromatoleum aromaticum* | - | - |
| Ab ipd | *Azospirillum brasilense* | - | - |
| Bs ipd | *Bradyrhizobium* sp. BTAi1 | - | - |
| NC ipd | NC10 bacterium | - | - |
| Rr ipd | *Rhodospirillum rubrum* | - | - |
| **Aromatic-L-amino-acid decarboxylases** | | | |
| Ab dcd | *Acinetobacter baumannii* | - | - |
| Bp dcd | *Bacillus pumilus* | - | - |
| Cg dcd | *Chryseobacterium gleum* | - | - |
| As dcd | *Anaeromyxobacter* sp. Fw109-5 | - | - |
| Bs dcd | *Burkholderia* sp. 383 | + | - |
| Cs dcd | *Cyanothece* sp. PCC 8801 | - | - |
| **Lysine decarboxylases** | | | |
| Cr lysA | *Candidatus Carsonella ruddii* | - | - |
| Dt lysA | *Dictyoglomus thermophilum* | - | - |
| Sa lysa | *Sphingopyxis alaskensis* | - | - |
| **Benzoylformate decarboxylases** | | | |
| Rp bfd | *Rhodopseudomonas palustris* | + | + |
| Sc bfd | *Streptomyces coelicolor* | + | + |
| Rc bfd | *Ricinus communis* | - | - |
| Pp bfd | *Pseudomonas putida* | + | + |
| Ms bfd | *Mycobacterium smegmatis* | - | - |
| As bfd | *Arthrobacter* sp. FB24 | + | + |
| Pb bfd | *Pseudomonas brassicacearum* | + | + |
| Ct bfd | *Comamonas testosterone* | + | + |
| Dd bfd | *Desulfovibrio desulfuricans* | + | + |
| Rj bfd | *Rhodococcus jostii* | + | + |

(continued)

| | | | |
|---|---|---|---|
| **Benzoylformate decarboxylases** | | | |
| Pf bfd | *Pseudomonas fluorescens* | + | + |
| **Purified Enzymes (purchased from Sigma-Aldrich)** | | | |
| L-Histidine Decarboxylase | *Lactobacillus* 30a | - | NT |
| L-Lysine Decarboxylase | *Bacterium cadaveris* | - | NT |
| L-Tyrosine Decarboxylase | *Streptococcus faecalis* | - | NT |
| Pyruvate Decarboxylase | *Saccharomyces cerevisiae* | - | NT |
| [a] -: No detection of appropriate product<br>[b] NT: Not tested<br>[c] +: Detection of appropriate product | | | |

**Example 24**

**Preparation of 3-Formyl-5-methylhex-3-enoic acid**

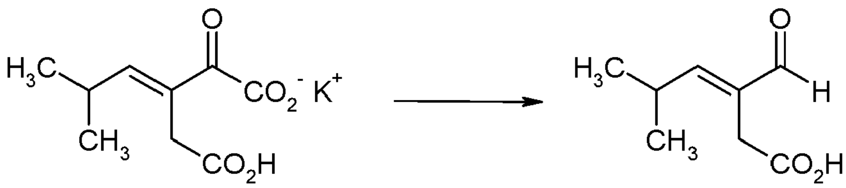

In a 250mL round bottom flask 80g of cell concentrate comprising E. *Coli* expressing *Pseudomonas putida* benzoylformate decarboxylase (see example 23) was charged. To this was added a pH 6.2 adjusted solution of 3-isobutylidene-2-oxopentanedioic acid mono-potassium salt (10g, see example 20) in 50mL water, with 0.5g of magnesium sulfate and 0.5g of thiamine pyrophosphate. The resulting pH 6.2 slurry was heated to 50°C and stirred for 145hrs keeping the pH adjusted between 6.2 and 7.2 with concentrated HCl. The reaction mixture was cooled to RT and centrifuged. The aqueous decants were washed with 50mL MTBE with minimum agitation. The pH of the aqueous phase was adjusted to 4 and filtered through celite. The filtrate was extracted with minimum agitation three times with 50mL MTBE. The MTBE was dried over anhydrous sodium sulfate and concentrated to a thick red oil. This oil was extracted with several portions of hot hexanes; the combined hexanes were cooled to <0° and the resulting crystals were isolated and dried in air to give 3-formyl-5-methylhex-3-enoic acid as a white solid (0.6g).
$^1$H NMR ($D_2O$ at pH 8-10, 400MHz) $\delta$ 9.2 (s, 1H), 6.6 (d, 1H), 3.1 (s, 2H), 2.7 (m, 1H), 1 (d, 6H)

**Example 25**

**Expression of Enoate Reductase Homologues in *E. coli***

The DNA sequence corresponding to the gene for *Lycopersicon esculentum* (tomato) 12-oxophytodienoate reductase 1 (OPR1) was retrieved from the Genbank database (accession number AC Q9XG54) and was synthesized by GeneArt (Germany). The sequence was codon optimized for expression in *E. coli,* and was subcloned into an *E. coli* expression plasmid pSTRC18 (Pfizer Inc., USA). The protein sequence is shown below. The OPR1 expression construct was transformed into BL21(DE3) *E. coli* (Stratagene, Agilent Technologies, Santa Clara, CA, USA) as directed and overnight cultures were incubated in LB + streptomycin media. The LB culture was used to inoculate expression cultures (LB, M9Y, or TB), which were incubated at 37°C (210rpm) After the culture reached a suitable biomass concentration (OD 1 at A600), IPTG was added (1 mM) and cultures were incubated for another 20 h (30°C, 210 rpm). The cells were harvested by centrifugation (4000 $\times$ *g*, 30 min, 4°C) and stored at -20 °C.

The BLASTP program was used to search the NCBI non-redundant protein sequences database for gene sequences related to 12-Oxophytodienoate reductase (OPR1) from *Lycopersicon esculentum.* Thirty eight sequences for related genes were selected, codon optimized for expression in *E.coli*, and subcloned into the pET28b(+) *E. coli*

expression plasmid (Novagen, EMD Chemicals, Gibbstown, NJ, USA). The OPR1 related expression constructs were transformed into BL21(DE3) *E. coli* (Stratagene, Agilent Technologies, Santa Clara, CA, USA) as directed and overnight cultures were grown in LB + kanamycin media. The LB cultures were used to inoculate expression cultures grown in Overnight Express Instant TB Medium (Novagen, EMD Chemicals, Gibbstown, NJ, USA). Cultures were incubated for 20h at 30°C, and the cells were harvested by centrifugation (4000 × *g*, 30min, 4°C) and stored at -20 °C.

*Lycopersicon esculentum* (tomato) 12-Oxophytodienoate Reductase 1 protein sequence:

MENKVVEEKQ VDKIPLMSPC KMGKFELCHR VVLAPLTRQR SYGYIPQPHA ILHYSQRSTN GGLLIGEATV ISETGIGYKD VPGIWTKEQV EAWKPIVDAV HAKGGIFFCQ IWHVGRVSNK DFQPNGEDPI SCTDRGLTPQ IRSNGIDIAH FTRPRRLTTD EIPQIVNEFR VAARNAIEAG FDGVEIHGAH GYLIDQFMKD QVNDRSDKYG GSLENRCRFA LEIVEAVANE IGSDRVGIRI SPFAHYNEAG DTNPTALGLY MVESLNKYDL AYCHVVEPRM KTAWEKIECT ESLVPMRKAY KGTFIVAGGY DREDGNRALI EDRADLVAYG RLFISNPDLP KRFELNAPLN KYNRDTFYTS DPIVGYTDYP FLETMT (SEQ ID NO. 8)

*Lycopersicon esculentum* (tomato) 12-Oxophytodienoate Reductase 1 codon optimized sequence:

ATGGAAAACAAAGTTGTGGAAGAAAAACAGGTTGATAAAATCCCGCTGATGAGCCC
GTGTAAAATGGGTAAATTCGAGCTGTGTCATCGCGTTGTACTGGCACCGCTGACTC
GTCAGCGTTCTTATGGTTACATTCCGCAGCCGCACGCAATCCTGCATTACTCTCAG
CGCAGCACCAACGGTGGCCTGCTGATCGGTGAAGCAACCGTGATCAGCGAAACT
GGCATCGGTTACAAAGATGTGCCGGGTATCTGGACGAAAGAGCAGGTTGAGGCCT
GGAAACCGATCGTCGACGCGGTGCATGCCAAAGGTGGTATTTTCTTTTGTCAGATC
TGGCACGTTGGTCGTGTATCCAACAAAGATTTTCAGCCGAACGGCGAAGATCCGA
TTTCCTGTACTGACCGCGGCCTGACCCCGCAGATCCGTTCCAACGGCATTGACAT
TGCCCACTTCACCCGTCCACGTCGCCTGACTACTGACGAGATTCCGCAGATCGTG
AACGAGTTCCGCGTTGCAGCGCGTAATGCTATTGAAGCGGGTTTCGATGGCGTCG
AGATTCATGGTGCCCACGGTTACCTGATCGACCAATTCATGAAAGACCAAGTTAAC
GACCGCAGCGATAAGTATGGCGGTTCTCTGGAGAACCGTTGTCGCTTCGCGCTGG
AAATCGTTGAAGCAGTAGCCAACGAGATTGGCTCCGACCGTGTTGGTATCCGTATC
TCTCCATTCGCACACTACAACGAAGCGGGCGACACTAACCCGACCGCACTGGGCC
TGTATATGGTGGAGAGCCTGAATAAATACGACCTGGCGTATTGTCACGTGGTCGA
GCCGCGCATGAAAACCGCCTGGGAAAAGATTGAGTGCACCGAAAGCCTGGTGCC
GATGCGTAAAGCCTACAAAGGCACCTTCATCGTAGCTGGTGGCTACGACCGTGAA
GACGGTAACCGCGCTCTGATCGAAGACCGTGCCGACCTGGTTGCGTACGGTCGT
CTGTTCATCAGCAACCCAGACCTGCCGAAGCGTTTTGAACTGAACGCTCCGCTGA
ACAAATACAACCGTGACACTTTCTACACTTCCGACCCGATCGTTGGTTACACCGAT
TACCCGTTTCTGGAAACTATGACTTAATAA (SEQ ID NO. 9)

**Example 26**

**Reduction of (*E*)-3-formyl-5-methylhex-3-enoic acid with Recombinant Reductases**

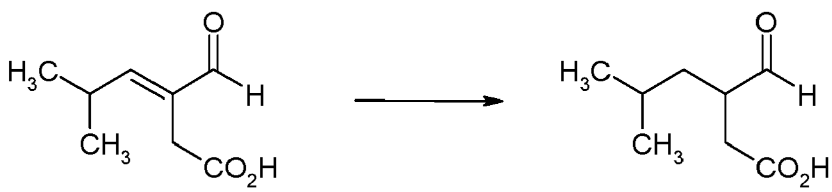

Recombinant enoate reductases were tested for reduction of (*E*)-3-formyl-5-methylhex-3-enoic acid using E. *coli* cells prepared as described in Example 25. Reactions (0.5mL) were carried out at 30°C in potassium phosphate buffer (100mM, pH 7.0) with *E. coli* cells (100mg wet cells/mL), NADPH (10mM), NADH (10mM) and (*E*)-3-formyl-5-methylhex-2-enoic acid (10mM). After 16 h, acetonitrile (0.5ml) was added to each reaction and the resulting mixtures were centrifuged (2000rpm x 5min). Aliquots (0.1mL) of the resulting supernatants were treated with 0.1mL of potassium phosphate buffer (100mM, adjusted to pH 2.2 with phosphoric acid) and 0.225mL of a solution of 2,4-dinitrophenylhydrazine (20mM in 1M HCl:acetonitrile, 3:1, v/v) for 30min at 50°C. The derivatized samples were diluted with 0.225mL

of acetonitrile and analyzed by HPLC on a Phenomenex Lux 5μ Amylose-2 column (250mm x 4.6mm id) eluted with 0.1% trifluoroacetic acid in water:acetonitrile (65:35, v/v) at 2mL/min. The column was maintained at 50°C and the effluent was monitored at 360nm. Results of HPLC analyses are shown in Table 2.

**Table 2**

| Entry | Enzyme Name /Source | Accession Number | % Conversion[1] |
|---|---|---|---|
| 1 | Old yellow enzyme 1 / *Saccharomyces carlsbergensis* | Q02899 | 6.5 |
| 2 | Old yellow enzyme 2 / *Saccharomyces cerevisiae* | Q03558 | 9.2 |
| 3 | Old yellow enzyme 3 / *Saccharomyces cerevisiae* | P41816 | 8.6 |
| 4 | NADH:flavin oxidoreductase / *Zymomonas mobilis* | Q5NLA1 | 85.0 |
| 5 | fumarate reductase / *Shewanella frigidimarina* | Q07WU7 | 3.4 |
| 6 | Pentaerythritol tetranitrate reductase / *Enterobacter cloacae* | Q6JL81 | 85.6 |
| 7 | Unnamed enzyme / *Arabidopsis thaliana* | BAH57049 | 3.7 |
| 8 | Allyl alcohol dehydrogenase / *Nicotiana tabacum* | BAA89423 | 5.0 |
| 9 | Artemisinic aldehyde delta-11 (13) reductase / *Artemisia annua* | 1WLY_A | 37.1 |
| 10 | 2-haloacrylate reductase / *Burkholderia sp. WS* | NP_390263 | 4.2 |
| 11 | NADPH dehydrogenase / *Bacillus subtilis* subsp. subtilis str. 168 | YP_390263 | 82.8 |
| 12 | NADH:flavin oxidoreductase/NADH oxidase / *Thermoanaerobacter pseudoethanolicus* | YP_001664021 | 54.9 |
| 13 | Unnamed enzyme / *Clostridium tyrobutyricum* | CAA71086 | 6.0 |
| 14 | Unnamed enzyme / *Moorella thermoautotrophica* | Q2RGT7 | 4.8 |
| 15 | oxophytodienoate reductase (OPR1) / *Lycopersicon esculentum* | Q9XG54 | 31.4 |
| 16 | Oxophytodienoate reductase (OPR3) / *Lycopersicon esculentum* | Q9FEW9 | 15.7 |
| 17 | N-ethylmaleimide reductase / *Shigella sonnei* | Q3Z206 | 100.0 |
| 18 | 12-oxo-phytodienoic acid reductase / *Zea mays* | Q49HE0 | 100.0 |
| 19 | 12-oxo-phytodienoic acid reductase / *Zea mays* | Q49HE4 | 100.0 |
| 20 | Unnamed enzyme / *Vitis vinifera* | A5BF80 | 7.5 |
| 21 | Unnamed enzyme / *Populus trichocarpa* | B9MWG6 | 100.0 |
| 22 | 12-oxophytodienoate reductase / *Arabidopsis thaliana* | Q8GYB8 | 100.0 |
| 23 | 12-oxophytodienoate reductase / Castor bean | B9SK95 | 5.4 |
| 24 | NADH:flavin oxidoreductase/NADH oxidase / *Klebsiella variicola* | D0YIM0 | 100.0 |
| 25 | Unnamed enzyme / *Citrobacter koseri* | A8AH31 | 100.0 |
| 26 | N-ethylmaleimide reductase / *Citrobacter sp.* | C1M473 | 100.0 |
| 27 | N-ethylmaleimide reductase / *Citrobacter rodentium* | D2THI8 | 100.0 |
| 28 | N-ethylmaleimide reductase / *Salmonella paratyphi* | Q5PH09 | 100.0 |
| 29 | N-ethylmaleimide reductase / *Cronobacter turicensis* | C9Y3L1 | 100.0 |
| 30 | Unnamed enzyme / *Providencia stuartii* | B2Q290 | 100.0 |
| 31 | NADPH dehydrogenase / *Yarrowia lipolytica* | Q6CI57 | 9.1 |

(continued)

| Entry | Enzyme Name /Source | Accession Number | % Conversion[1] |
|---|---|---|---|
| 32 | N-ethylmaleimide reductase / *Pseudomonas putida* | Q88I29 | 17.4 |
| 33 | 12-oxophytodienoate reductase / *Pichia stipitis* | 150864790 | 7.8 |
| 34 | NAPDH dehydrogenase / *Pichia stipitis* | 126131638 | 12.6 |
| 35 | Unnamed enzyme / *Pichia guilliermondii* | 146393506 | 21.6 |
| 36 | unnamed enzyme / *Candida glabrata* | 50293551 | 36.5 |
| 37 | Unnamed enzyme / *Debaryomyces hansenii* | 50405397 | 9.1 |
| 38 | Unnamed enzyme/ *Geobacillus kaustophilus* HTA426 | Q5KXG9 | 100.0 |
| 39 | Oxophytodienoate reductase (OPR2) / *Lycopersicon esculentium* | Q9XG54 | 14.0 |

[1]%Conversion of (*E*)-3-formyl-5-methylhex-2-enoic acid to 3-formyl-5-methylhexanoic acid

**Example 27**

**Reduction of (*E*)-3-formyl-5-methylhex-3-enoic acid with Recombinant Reductases and Formate Dehydrogenase**

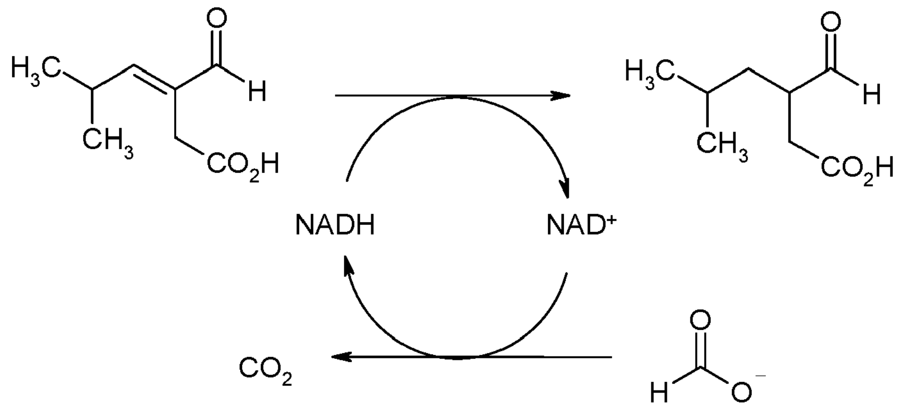


Recombinant enoate reductases were evaluated for reduction of (*E*)-3-formyl-5-methylhex-3-enoic acid with $NAD^+$ and formate dehydrogenase. Enoate reductases were expressed in *E. coli* cells as described in Example 25. Formate dehydrogenase was expressed in *E. coli* cells as follows: The pET26b formate dehydrogenase expression construct was transformed into BL21(DE3) *E. coli* (Stratagene, Agilent Technologies, Santa Clara, CA, USA) as directed and overnight cultures were grown in LB + kanamycin media. The LB cultures were used to inoculate expression cultures grown in Overnight Express Instant TB Medium + kanamycin (Novagen, EMD Chemicals, Gibbstown, NJ, USA). Cultures were incubated for 20 h at 30°C, and the cells were harvested by centrifugation (4000 x *g*, 30 min, 4°C) and stored at -20 °C. A variant (D74M) of oxophytodienoate reductase 3 (Accession number Q9FEW9) was expressed in *E. coli* cells as follows: QuikChange Site-directed Mutagenesis kit from Stratagene (La Jolla, CA,USA) was used to create oxophytodienoate reductase 3 variant D74M as directed. Primers were ordered from Integrated DNA Technologies (Coralville, IA,USA). The pSTRC18 oxophytodienoate reductase 3 (OPR3) expression construct was transformed into BL21(DE3) *E. coli* (Stratagene, Agilent Technologies, Santa Clara, CA, USA) as directed and overnight cultures were grown in expansion broth + streptomycin (Zymo Research, Irvine,CA, USA). The LB cultures were used to inoculate expression cultures grown in Overexpression broth + streptomycin (Zymo Research, Irvine,CA, USA). Cultures were incubated for 20h at 23°C, and the cells were harvested by centrifugation (4000 x *g*, 30min, 4°C) and stored at -20°C.

Reactions (0.5mL) were carried out at 30°C in potassium phosphate buffer (100mM, pH 7.0) with enoate reductases (40mg wet cells/mL), formate dehydrogenase (80mg wet cells/mL), $NAD^+$ (0.02mM), ammonium formate (30mM) and (E)-3-formyl-5-methylhex-3-enoic acid (20mM). After 24 h, reaction mixtures were acidified with 0.025mL of 4N HCl and extracted with 1mL of ethyl acetate. Aliquots (0.5mL) of the ethyl acetate extracts (0.5mL) were dried

over anhydrous sodium sulfate and treated with methanol (0.02mL) and (trimethylsilyl)diazomethane (0.01mL of a 2M solution in diethyl ether) to derivatize carboxylic acid moieties to their corresponding methyl esters. The derivatized samples were analyzed by GC on a Chiraldex™ G-TA column (30M x 0.25mm column, column temperature: 135°C isothermal, injector temperature: 200°C, carrier gas: helium, flow rate approximately 1mL/min) to give the results shown in Table 3.

**Table 3**

| Entry | Enzyme Name /Source | Accession Number | % Conversion[1] |
|---|---|---|---|
| 1 | NADH:flavin oxidoreductase / *Zymomonas mobilis* | Q5NLA1 | 23.8 |
| 2 | Pentaerythritol tetranitrate reductase / *Enterobacter cloacae* | Q6JL81 | 19.6 |
| 3 | NADPH dehydrogenase / *Bacillus subtilis* subsp. subtilis str. 168 | YP_390263 | 17.0 |
| 4 | NADH:flavin oxidoreductase/NADH oxidase / *Thermoanaerobacter pseudoethanolicus* | YP_001664021 | 15.9 |
| 5 | oxophytodienoate reductase (OPR 3) variant D74M / *Lycopersicon esculentum* | | 43.5 |
| 6 | N-ethylmaleimide reductase / *Shigella sonnei* | Q3Z206 | 24.0 |
| 7 | Unnamed enzyme / *Populus trichocarpa* | B9MWG6 | 31.8 |
| 8 | N-ethylmaleimide reductase / *Citrobacter sp.* 30_2 | C1M473 | 22.8 |
| 9 | N-ethylmaleimide reductase / | C9Y3L1 | 26.3 |
| | *Cronobacter turicensis* DSM 18703 | | |
| 10 | Unnamed enzyme / *Geobacillus kaustophilus* HTA426 | Q5KXG9 | 21.7 |
| 11 | 12-oxophytodienoate reductase (OPR1) / *Lycopersicon esculentum* | Q9XG54 | 39.8 |
| [1]%Conversion of (*E*)-3-formyl-5-methylhex-2-enoic acid to 3-formyl-5-methylhexanoic acid (all reactions gave approximately 1:1 mixtures of (*S*)- and (*R*)-enantiomers | | | |

**Example 28**

**Reduction of (*E*)-3-formyl-5-methylhex-3-enoic acid with Recombinant Reductases**

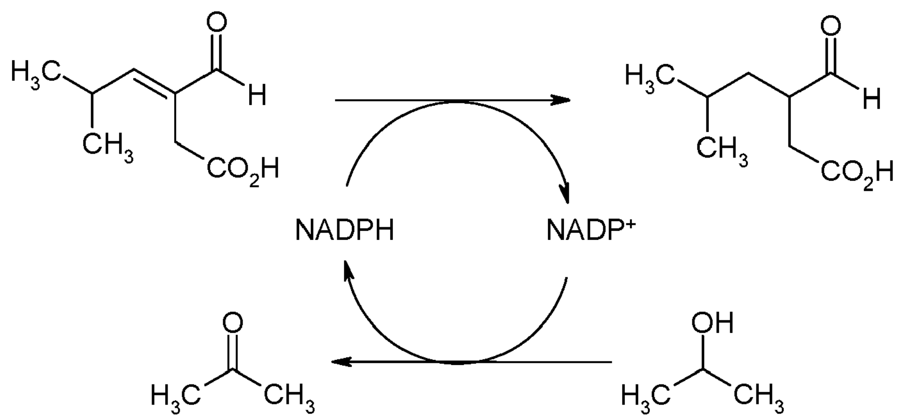

Recombinant enoate reductases were evaluated for reduction of (*E*)-3-formyl-5-methylhex-3-enoic acid with $NADP^+$ and *Lactobacillus brevis* alcohol dehydrogenase (X-zyme). Enoate reductases were expressed in *E. coli* cells as described in Example 25. A variant (D74M) of oxophytodienoate reductase 3 (OPR3) was prepared as described in Example 27. Reactions (0.5mL) were carried out at 30°C in potassium phosphate buffer (100mM, pH 7.0) with enoate reductases (40mg wet cells/mL), *Lactobacillus brevis* alcohol dehydrogenase (32U/mL), $NADP^+$ (0.02mM), 2-propanol (3 vol%) and (*E*)-3-formyl-5-methylhex-2-enoic acid (20mM). After 24 h, reaction mixtures were acidified with 0.025mL of 4N HCl and extracted with 1mL of ethyl acetate. Aliquots (0.5mL) of the ethyl acetate extracts (0.5mL) were dried over anhydrous sodium sulfate and treated with methanol (0.02mL) and (trimethylsilyl)diazomethane (0.01mL of a 2M solution in diethyl ether) to derivatize carboxylic acid moieties to their corresponding methyl esters. The derivatized samples were analyzed by GC on a Chiraldex™ G-TA column (30M x 0.25mm column, column temperature: 135°C isothermal, injector temperature: 200°C, carrier gas: helium, flow rate approximately 1mL/min) to give the results shown in Table 4.

**Table 4**

| Entry | Enzyme Name /Source | Accession Number | % Conversion[1] |
|---|---|---|---|
| 1 | NADH:flavin oxidoreductase / *Zymomonas mobilis* | Q5NLA1 | 73.0 |
| 2 | Pentaerythritol tetranitrate reductase / *Enterobacter cloacae* | Q6JL81 | 100.0 |
| 3 | NADPH dehydrogenase / *Bacillus subtilis* subsp. subtilis str. 168 | YP_390263 | 100.0 |
| 4 | NADH:flavin oxidoreductase/NADH *oxidase* / *Thermoanaerobacter pseudoethanolicus* | YP_001664021 | 100.0 |
| 5 | oxophytodienoate reductase (OPR 3) variant D74M / *Lycopersicon esculentum* | | 96.8 |
| 6 | N-ethylmaleimide reductase / *Shigella sonnei* | Q3Z206 | 100.0 |
| 7 | Unnamed enzyme / *Populus trichocarpa* | B9MWG6 | 100.0 |
| 8 | N-ethylmaleimide reductase / *Citrobacter sp.* 30_2 | C1M473 | 100.0 |
| 9 | N-ethylmaleimide reductase / *Cronobacter turicensis* DSM 18703 | C9Y3L1 | 99.5 |
| 10 | Unnamed enzyme / *Geobacillus kaustophilus* HTA426 | Q5KXG9 | 100.0 |
| 11 | 12-oxophytodienoate reductase (OPR1) / *Lycopersicon esculentum* | Q9XG54 | 34.3 |
| [1]%Conversion of (*E*)-3-formyl-5-methylhex-2-enoic acid to 3-formyl-5-methylhexanoic acid (all reactions gave approximately 1:1 mixtures of (S)- and (R)-enantiomers | | | |

## Example 29

### Preparation of 3-formyl-5-methylhexanoic acid

A reaction vessel was charged with 7.5mL potassium phosphate buffer (0.1M, pH 7.0), 8.4mg $NADP^+$, 0.2mL *Lactobacillus brevis* alcohol dehydrogenase (32U/mL, X-zyme), 0.3mL 2-propanol, pentaerythritol tetranitrate reductase (2mL of a 200mg/mL suspension of *E. coli* cells in potassium phosphate buffer), and 156mg of (*E*)-3-formyl-5-methylhex-3-enoic acid, and agitated at 40°C. After 6.75h, the reaction mixture was centrifuged and the supernatant was adjusted to pH 2 with 4N HCl and extracted with ethyl acetate (2 x 10mL). The ethyl acetate extract was dried over anhydrous magnesium sulfate, filtered and concentrated under reduced pressure to give 141 mg of colorless oil (89% yield). $^1H$ NMR ($D_2O$ at pH 8 - 10) $\delta$ 2.53 - 2.43 (m, 2H), 2.34 - 2.28 (m, 1H), 1.53 - 1.42 (m, 1H), 1.41 - 1.34 (m, 1H), 1.20 - 1.12 (m, 1H), 0.76 (d, 3H), 0.74 (d, 3H).

**Example 30**

**Biotransformation of 3-formyl-5-methylhexanoic acid to pregabalin with recombinant ω-transaminases.**

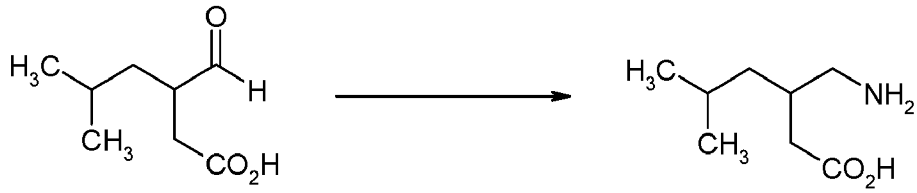

Transamination of 3-formyl-5-methylhexanoic acid to form Pregabalin was evaluated with various recombinant transaminases. Recombinant ω-transaminases from *Vibrio fluvialis, Rhodobacter sphaeroides*, and *Paracoccus denitrificans* were expressed in *E. coli* as follows: The pET28b ω-transaminase expression constructs were transformed into BL21(DE3) *E. coli* (Stratagene, Agilent Technologies, Santa Clara, CA, USA) as directed and overnight cultures were grown in LB + kanamycin media. The LB cultures were used to inoculate expression cultures grown in Overnight Express Instant TB + kanamycin Medium (Novagen, EMD Chemicals, Gibbstown, NJ, USA). Cultures were incubated for 20 h at 30°C, and the cells were harvested by centrifugation (4000 x *g*, 30 min, 4°C) and stored at -20°C.

Reactions (0.5mL) were carried out at 30°C in potassium phosphate buffer (100mM, pH 7.0), pyridoxal phosphate (2mM), isopropylamine (150mM), 3-formyl-5-methylhexanoic acid (50mM) and ω-transaminase (40mg wet cells/mL) from *Vibrio fluvialis, Rhodobacter sphaeroides,* or *Paracoccus denitrificans.* After 24h, reaction samples (0.1 mL) were diluted with 0.4mL acetonitrile:water (1:1, v/v). Aliquots (0.05mL) of the diluted reaction samples were treated with saturated aqueous sodium bicarbonate (0.01mL) and Marfey's reagent (*N*-α-(2,4-dinitro-5-fluorophenyl)alaninamide, 0.2mL of 5g/L solution in acetonitrile) at 40°C. After 1 h, the derivatization reactions were quenched with 0.01 mL 1 N aqueous hydrochloric acid and diluted with 0.23mL of acetonitirle. The derivatized reaction samples were analyzed by UPLC (column: BEH C18, 50mm x 2.1 mm id, gradient elution: 70% A:30% B to 55% A:45% B in 5 min (A = 1% triethylamine (pH 3 with phosphoric acid); B = acetonitrile), flow rate: 0.8mL/min, column temperature: 30°C, detection: 210-400nm) to give the results shown in Table 5.

**Table 5**

| Entry | Enzyme | Accession Number | %Yield Pregabalin | %ee (S)-Pregabalin |
|---|---|---|---|---|
| 1 | *Vibrio fluvialis* ω-transaminase | AEA39183 | 37.5 | 60.6 |
| 2 | *Rhodobacter sphaeroides* ω-transaminase | YP_001043908 | 24 | 84.4 |
| 3 | *Paracoccus denitrificans* ω-transaminase | YP_917746 | 44 | 60.6 |

**Example 31**

**Biotransformation of 3-formyl-5-methylhexanoic acid to pregabalin with recombinant ω-transaminases.**

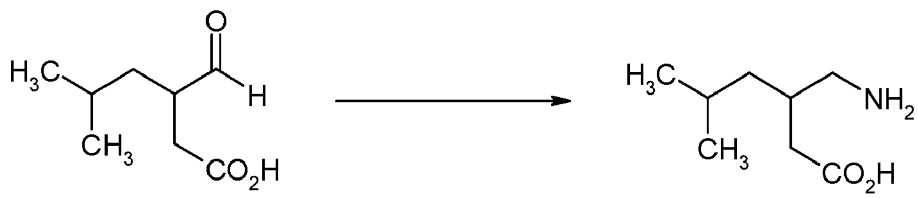

Recombinant variants of *Vibrio fluvialis* were tested for the reductive amination of 3-formyl-5-methylhexanoic acid to form Pregabalin. Variants of *V. fluvialis* ω-transaminase (Accession number AEA39183) were expressed in *E. coli* as follows: QuikChange Site-directed Mutagenesis kits from Stratagene (La Jolla, CA,USA) was used to create *V. fluvialis* aminotransferase variants as directed. Primers were ordered from Integrated DNA Technologies (Coralville, IA,USA). The pET28b ω-transaminase expression constructs were transformed into BL21(DE3) *E. coli* (Stratagene, Agilent Technologies, Santa Clara, CA, USA) as directed and overnight cultures were grown in LB + kanamycin media. The LB cultures were used to inoculate expression cultures grown in Overnight Express Instant TB + kanamycin Medium (Novagen, EMD Chemicals, Gibbstown, NJ, USA). Cultures were incubated for 20h at 30°C, and the cells were harvested by centrifugation (4000 x *g*, 30min, 4°C) and stored at -20°C.

Reactions (0.5mL) were carried out at 30°C in potassium phosphate buffer (100mM, pH 7.0) with pyridoxal phosphate (2mM), isopropylamine (300mM), 3-formyl-5-methylhexanoic acid (100mM) and *V. fluvialis* ω-transaminase wild-type or variants (40mg wet cells/mL). After 28h, reaction samples (0.1mL) were diluted with 0.4mL acetonitrile:water (1:1, v/v). Aliquots (0.05mL) of the diluted reaction samples were treated with saturated aqueous sodium bicarbonate (0.01mL) and Marfey's reagent (*N*-α-(2,4-dinitro-5-fluorophenyl)alaninamide, 0.2mL of 5g/L solution in acetonitrile) at 40°C. After 1 h, the derivatization reactions were quenched with 0.01mL 1N aqueous hydrochloric acid and diluted 0.23mL of acetonitrile. The derivatized reaction samples were analyzed by UPLC (column: BEH C18, 50mm x 2.1 mm id, gradient elution: 70% A:30% B to 55% A:45% B in 5min (A = 1% triethylamine (pH 3 with phosphoric acid); B = acetonitrile), flow rate: 0.8mL/min, column temperature: 30°C, detection: 210-400nm) to give the results shown in Table 6.

**Table 6**

| Entry | *V. fluvialis* ω-transaminase variant | % Conversion | % ee |
|---|---|---|---|
| 1 | W57F/K163L/R415F | 6.1 | 81.1 |
| 2 | Y165F | 9.5 | 75.3 |
| 3 | W147N | 10.0 | 75.0 |
| 4 | A228G | 32.3 | 74.9 |
| 5 | N166V | 16.6 | 74.5 |
| 6 | W57F/A228G | 33.3 | 73.9 |
| 7 | V156M | 13.2 | 70.3 |
| 8 | S159A | 7.9 | 70.2 |
| 9 | W57F/R415F | 10.7 | 68.5 |
| 10 | R415F | 11.8 | 68.1 |
| 11 | M59N | 10.1 | 68.0 |
| 12 | P301K | 19.1 | 67.6 |
| 13 | Y113F | 7.9 | 65.2 |
| 14 | F86G | 32.1 | 64.1 |
| 15 | 1254V | 32.1 | 64.0 |
| 16 | H326N | 15.9 | 63.8 |
| 17 | C414I | 10.1 | 63.0 |
| 18 | W57F/K163L/V153A | 46.1 | 41.2 |
| 19 | W57F/K163L | 43.5 | 36.1 |
| 20 | D21Y | 45.1 | 34.2 |
| 21 | W57F/D21Y | 46.3 | 34.0 |
| 22 | M294V | 42.4 | 33.2 |
| 23 | W57F/M294V | 41.2 | 32.5 |
| 24 | W57F/V177I | 39.9 | 29.0 |
| 25[1] | Vat 565 | 20.0 | 90.5 |

(continued)

| Entry | *V. fluvialis* ω-transaminase variant | % Conversion | % ee |
|---|---|---|---|
| 26[1] | Vfat665 | 15.0 | 93.1 |
| 27[1] | Vfat701 | 9.0 | 94.4 |
| 28[1] | Vfat707 | 7.5 | 95.7 |
| 29[1] | Vfat747 | 50.0 | 97.5 |
| 30[1] | Vfat825 | 75.0 | 95.7 |
| 31[1] | Vfat 850 | 81.3 | 94.6 |
| 32[1] | Vfat 875 | 80.5 | 95.1 |
| 33[1] | Vfat888[2] | 95.0 | 95.5 |
| Note - 1: Entry 25 thru 33 - reactions were performed using 400mM of 3-formyl-5-methylhexanoic acid , 3mM PLP, 800mM IPM at 45°C.<br>2: Vfat 888: | | | |

DNA SEQUENCE

ATGAATAAACCACAAAGCTGGGAAGCGCGTGCTGAAACTTACTCTCTGTAC
GGCTTCACTGATATGCCATCTCTGCACCAGCGTGGTACCGTGGTTGTCACC
CACGGCGAGGGCCCATACATCGTGGACGTCAACGGTCGCCGTTACCTGGA
CGCAAACTCCGGCCTGTACAATATGGTTGCCGGCTTCGACCACAAGGGTCT
GATCGACGCAGCAAAGGCCCAGTACGAACGCTTCCCGGGTTACCATAGCTT
CTTCGGTCGTATGTCTGATCAAACTGTTATGCTGAGCGAGAAACTGGTAGA
GGTGTCTCCATTCGACAGCGGTCGCGTGTTCTATACTAACTCCGGCTCCGA
GGCTAACGATACTATGGTGAAAATGCTGTGGTTTCTGCACGCCGCAGAGGG
CAAGCCGCAAAAACGCAAAATCCTGACTCGTAACAACGCATACCACGGTGT
AACTGCTGTTTCCGCTTCCATGACGGGTCTGCCGTACAACTCTGTATTCGG
CCTGCCGCTGCCGGGTTTCGTTCACCTGACCTGTCCGCACTATTGGCGTTA
CGGCGAAGAAGGTGAAACCGAAGAGCAGTTTGTTGCTCGTCTGGCCCGCG
AGCTGGAGGAAACTATCCAACGTGAAGGCGCGGACACGATTGCGGGCTTC
TTTGCTGAGCCGGTCATGGGCGCGGGCGGCGTAATCCCGCCGGCGAAAG
GTTACTTCCAGGCGATCCTGCCGATTCTGCGTAAGTACGACATCCCGGTTA
TCTCTGATGAAGTTATCTGCGGCTTTGGTCGTACCGGTAATACTTGGGGTTG
CGTTACCTATGACTTCACCCCGGATGCGATCATCTCCAGCAAAAATCTGACC
GCCGGTTTCTTTCCGGTTGGTGCTGTGATTCTGGGTCCGGAACTGGCGAAA
CGCCTGGAAACGGCGATCGAAGCTATCGAAGAGTTCCCGCACGGCTTTAC
GGCCAGCGGTCACCCGGTGGGTTGCGCTATCGCTCTGAAAGCAATCGATG
TTGTGATGAATGAGGGTCTGGCAGAGAACGTGCGCCGCCTGGCACCGCGT
TTTGAGGAGCGTCTGAAACACATTGCCGAACGTCCGAACATCGGTGAATAT
CGTGGCATCGGTTTTATGTGGGCACTGGAGGCTGTGAAAGACAAAGCATCT
AAAACCCCATTCGATGGTAATCTGTCTGTGAGCGAGCGTATCGCTAACACCT
GTACCGACCTGGGCCTGATCTGTAGCCCGATGGGTCAGTCCGTTATCCTGT
GCCCGCCGTTCATCCTGACCGAGGCGCAAATGGATGAGATGTTTGACAAAC

TGGAGAAGGCTCTGGACAAAGTCTTTGCGGAGGTGGCGTAA (SEQ ID NO. 10)

**AMINO ACID SEQUENCE**

MNKPQSWEARAETYSLYGFTDMPSLHQRGTVVVTHGEGPYIVDVNGRRYLDA
NSGLYNMVAGFDHKGLIDAAKAQYERFPGYHSFFGRMSDQTVMLSEKLVEVS
PFDSGRVFYTNSGSEANDTMVKMLWFLHAAEGKPQKRKILTRNNAYHGVTAV
SASMTGLPYNSVFGLPLPGFVHLTCPHYWRYGEEGETEEQFVARLARELEETI
QREGADTIAGFFAEPVMGAGGVIPPAKGYFQAILPILRKYDIPVISDEVICGFGRT
GNTWGCVTYDFTPDAIISSKNLTAGFFPVGAVILGPELAKRLETAIEAIEEFPHGF
TASGHPVGCAIALKAIDVVMNEGLAENVRRLAPRFEERLKHIAERPNIGEYRGIG
FMWALEAVKDKASKTPFDGNLSVSERIANTCTDLGLICSPMGQSVILCPPFILTE
AQMDEMFDKLEKALDKVFAEVA (SEQ ID NO. 2)

**Example 32**

**Enzymatic reduction of (*E*)-3-formyl-5-methylhex-2-enoic acid to 3-formyl-5-methylhexanoic acid and *situ* conversion to pregabalin with ω-transaminase**

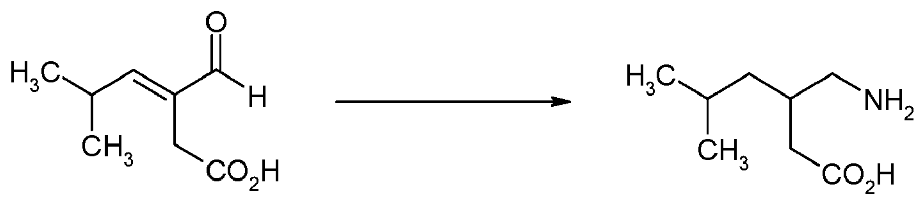

Reduction of (*E*)-3-formyl-5-methylhex-3-enoic acid to 3-formyl-5-methylhexanoic acid with pentaerythritol tetranitrate reductase and *in situ* conversion to pregabalin was evaluated with various ω-transaminases. Recombinant ω-transaminases from *Vibrio fluvialis, Rhodobacter sphaeroides*, and *Paracoccus denitrificans* were expressed in *E. coli* as follows: The pET28b ω-transaminase expression constructs were transformed into BL21(DE3) *E. coli* (Stratagene, Agilent Technologies, Santa Clara, CA, USA) as directed and overnight cultures were grown in LB + kanamycin media. The LB cultures were used to inoculate expression cultures grown in Overnight Express Instant TB + kanamycin Medium (Novagen, EMD Chemicals, Gibbstown, NJ, USA). Cultures were incubated for 20h at 30°C, and the cells were harvested by centrifugation (4000 x *g*, 30min, 4°C) and stored at -20°C.

Reactions (0.5mL) were carried out at 30°C in potassium phosphate buffer (100mM, pH 7.0) with pentaerythritol tetranitrate reductase (40mg wet cells/mL), *Lactobacillus brevis* alcohol dehydrogenase (32U/mL), $NADP^+$ (0.02mM), 2-propanol (3 vol%), pyridoxal phosphate (2 mM), isopropylamine (100mM), (*E*)-3-formyl-5-methylhex-2-enoic acid (20mM) and ω-transaminase (40mg wet cells/mL) from *Vibrio fluvialis, Rhodobacter sphaeroides,* or *Paracoccus denitrificans.* After 43 h, reaction samples (0.1mL) were diluted with 0.1 mL acetonitrile:water (1:1, v/v). Aliquots (0.1mL) of the diluted reaction samples were treated with saturated aqueous sodium bicarbonate (0.01mL) and Marfey's reagent (*N*-α-(2,4-dinitro-5-fluorophenyl)alaninamide, 0.4mL of 5g/L solution in acetonitrile) at 40°C. After 1 h, the derivatization reactions were quenched with 0.1 mL 1N aqueous hydrochloric acid. The derivatized reaction samples were analyzed by UPLC (column: BEH C18, 50mm x 2.1 mm id, gradient elution: 70% A:30% B to 55% A:45% B in 5min (A = 1% triethylamine (pH 3 with phosphoric acid); B = acetonitrile), flow rate: 0.8mL/min, column temperature: 30°C, detection: 210-400nm) to give the results shown in Table 7.

**Table 7**

| Entry | Enzyme | Accession Number | %Yield Pregabalin | %ee (*S*)-Pregabalin |
|---|---|---|---|---|
| 1 | *Vibrio fluvialis* ω-transaminase | AEA39183 | 60.8 | 42.0 |

(continued)

| Entry | Enzyme | Accession Number | %Yield Pregabalin | %ee (*S*)-Pregabalin |
|---|---|---|---|---|
| 2 | *Rhodobacter sphaeroides* ω-transaminase | YP_001043908 | 60.4 | 54.6 |
| 3 | *Paracoccus denitrificans* ω-transaminase | YP_917746 | 33.6 | 61.5 |

**Example 33**

**Enzymatic reduction of (*E*)-3-formyl-5-methylhex-2-enoic acid to 3-formyl-5-methylhexanoic acid and in-situ conversion to pregabalin with *V. fluvialis* ω-transaminase**

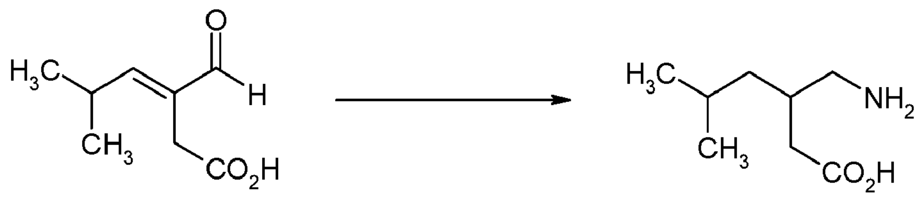

Reduction of (*E*)-3-formyl-5-methylhex-3-enoic acid to 3-formyl-5-methylhexanoic acid with pentaerythritol tetranitrate reductase and in-situ conversion to pregabalin was evaluated with variants of *V. fluvialis* aminotransferase. Variants of *V. fluvialis* ω-transaminase (Accession number AEA39183) were expressed in *E. coli* as follows: QuikChange Site-directed Mutagenesis kits from Stratagene (La Jolla, CA,USA) was used to create *V. fluvialis* aminotransferase variants as directed. Primers were ordered from Integrated DNA Technologies (Coralville, IA,USA). The pET28b ω-transaminase expression constructs were transformed into BL21(DE3) *E. coli* (Stratagene, Agilent Technologies, Santa Clara, CA, USA) as directed and overnight cultures were grown in LB + kanamycin media. The LB cultures were used to inoculate expression cultures grown in Overnight Express Instant TB + kanamycin Medium (Novagen, EMD Chemicals, Gibbstown, NJ, USA). Cultures were incubated for 20h at 30°C, and the cells were harvested by centrifugation (4000 x *g*, 30min, 4°C) and stored at -20°C. Reactions (0.5mL) were carried out at 30°C in potassium phosphate buffer (100mM, pH 7.0) with pentaerythritol tetranitrate reductase (40mg wet cells/mL), *Lactobacillus brevis* alcohol dehydrogenase (32U/mL), $NADP^+$ (0.1mM), 2-propanol (3 vol%), pyridoxal phosphate (2mM), isopropylamine (300mM), (*E*)-3-formyl-5-methylhex-2-enoic acid (100mM) and *V. fluvialis* ω-transaminase wild-type or variants (40mg wet cells/mL). After 48h, reaction samples (0.02mL) were diluted with 0.18mL acetonitrile:water (1:1, v/v). Aliquots (0.1 mL) of the diluted reaction samples were treated with saturated aqueous sodium bicarbonate (0.01mL) and Marfey's reagent (*N*-α-(2,4-dinitro-5-fluorophenyl)alaninamide, 0.4mL of 5 g/L solution in acetonitrile) at 40°C. After 1 h, the derivatization reactions were quenched with 0.1 mL 1 N aqueous hydrochloric acid. The derivatized reaction samples were analyzed by UPLC (column: BEH C18, 50mm x 2.1 mm id, gradient elution: 70% A:30% B to 55% A:45% B in 5 min (A = 1% triethylamine (pH 3 with phosphoric acid); B = acetonitrile), flow rate: 0.8mL/min, column temperature: 30°C, detection: 210-400nm) to give the results shown in Table 8.

**Table 8**

| Entry | *V. fluvialis* transaminase Variant | %Yield Pregabalin | %ee (*S*)-Pregabalin |
|---|---|---|---|
| 1 | M294V | 64.8 | 73.7 |
| 2 | T268M | 47.4 | 77.8 |
| 3 | P233L | 77.3 | 71 |
| 4 | N166V | 38.1 | 73.1 |
| 5 | C424A | 76.5 | 70.6 |
| 6 | L100M | 83.7 | 71.4 |

(continued)

| Entry | *V. fluvialis* transaminase Variant | %Yield Pregabalin | %ee (*S*)-Pregabalin |
|---|---|---|---|
| 7 | S283A | 68.1 | 71 |
| 8 | L417M | 68.7 | 71.8 |
| 9 | Wild-type | 62.7 | 61.5 |

**Example 34**

**Alternative variants of *Vibrio fluvalis* ω-transaminase**

The following further recombinant variants of *Vibrio fluvialis* ω-transaminase were expressed in *E. coli* as follows: The pET28b ω-transaminase expression constructs were transformed into BL21(DE3) E. *coli* (Stratagene, Agilent Technologies, Santa Clara, CA, USA) as directed and overnight cultures were grown in LB + kanamycin media. The LB cultures were used to inoculate expression cultures grown in Overnight Express Instant TB + kanamycin Medium (Novagen, EMD Chemicals, Gibbstown, NJ, USA). Cultures were incubated for 20 h at 30°C, and the cells were harvested by centrifugation (4000 x *g*, 30 min, 4°C) and stored at -20°C.

**Example 34a: Vfat906**

DNA Sequence:

ATGAATAAACCACAAAGCTGGGAAGCGCGTGCTGAAACTTACTCTCTGTACGGCTT
CACTGATATGCCATCTCTGCACgAGCGTGGTACCGTGGTTGTCACCCACGGCGAG
GGCCCATACATCGTGGACGTCAACGGTCGCCGTTACCTGGACGCAAACTCCGGCC
TGTACAATATGGTTGCCGGCTTCGACCACAAGGGTCTGATCGACGCAGCAAAGGC
CCAGTACGAACGCTTCCCGGGTTACCATAGCTTCTTCGGTCGTATGTCTGATCAAA
CTGTTATGCTGAGCGAGAAACTGGTAGAGGTGTCTCCATTCGACAGCGGTCGCGT
GTTCTATACTAACTCCGGCTCCGAGGCTAACGATACTATGGTGAAAATGCTGTGGT
TTCTGCACGCCGCAGAGGGCAAGCCGCAAAAACGCAAAATCCTGACTCGTaacAAC
GCATACCACGGTGTAACTGCTGTTTCCGCTTCCATGACGGGTCTGCCGTACAACTC
TGTATTCGGCCTGCCGCTGCCGGGTTTCGTTCACCTGACCTGTCCGCACTATTGG
CGTTACGGCGAAGAAGGTGAAACCGAAGAGCAGTTTGTTGCTCGTCTGGCCCGCG
AGCTGGAGGAAACTATCCAACGTGAAGGCGCGGACACGATTGCGGGCTTCTTTGC
TGAGCCGGTCATGGGCGCGGGCGGCGTAATCCCGCCGGCGAAAGGTTACTTCCA

GGCGATCCTGCCGATTCTGCGTAAGTACGACATCCCGGTTATCTCTGATGAAGTTA
TCTGCGGCTTTGGTCGTACCGGTAATACTTGGGGTTGCGTTACCTATGACTTCACC
CCGGATGCGATCATCTCCAGCAAAAATCTGACCGCCGGTTTCTTTCCGGTTGGTG
CTGTGATTCTGGGTCCGGAACTGAGCAAACGCCTGGAAACGGCGATCGAAGCTAT
CGAAGAGTTCCCGCACGGCTTTACGGCCggcGGTCACCCGGTGGGTTGCGCTATC
GCTCTGAAAGCAATCGATGTTGTGATGAATGAGGGTCTGGCAGAGAACGTGCGCC
GCCTGGCACCGCGTTTTGAGGAGCGTCTGAAACACATTGCCGAACGTCCGAACAT
CGGTGAATATCGTGGCATCGGTTTTATGTGGGCACTGGAGGCTGTGAAAGACAAA
GCATCTAAAACCCCATTCGATGGTAATCTGTCTGTGAGCaaaCGTATCGCTAACACC
TGTcagGACCTGGGCCTGATCTGTAGCgCGCTGGGTCAGTCCGTTATCCTGTGCCC
GCCGTTCATCCTGACCGAGGCGCAAATGGATGAGATGTTTGACAAACTGGAGAAG
GCTCTGGACAAAGTCTTTGCGGAGGTGGCGTAA (SEQ ID NO. 11)

Amino acid sequence:

MNKPQSWEARAETYSLYGFTDMPSLHERGTVVVTHGEGPYIVDVNGRRYLDANSGLY
NMVAGFDHKGLIDAAKAQYERFPGYHSFFGRMSDQTVMLSEKLVEVSPFDSGRVFYT
NSGSEANDTMVKMLWFLHAAEGKPQKRKILTRNNAYHGVTAVSASMTGLPYNSVFGL
PLPGFVHLTCPHYWRYGEEGETEEQFVARLARELEETIQREGADTIAGFFAEPVMGAG
GVIPPAKGYFQAILPILRKYDIPVISDEVICGFGRTGNTWGCVTYDFTPDAIISSKNLTAG
FFPVGAVILGPELSKRLETAIEAIEEFPHGFTAGGHPVGCAIALKAIDVVMNEGLAENVR
RLAPRFEERLKHIAERPNIGEYRGIGFMWALEAVKDKASKTPFDGNLSVSKRIANTCQD
LGLICSALGQSVILCPPFILTEAQMDEMFDKLEKALDKVFAEVA (SEQ ID NO. 3)

**Example 34b: Vfat999**

DNA Sequence:

ATGAATAAACCACAAAGCTGGGAAGCGCGTGCTGAAACTTACTCTCTGTACGGCTT
CACTGATATGCCATCTCTGCACGAGCGTGGTACCGTGGTTGTCACCCACGGCGAG
GGCCCATACGTGGTGGACGTCAACGGTCGCCGTTACCTGGACGCAAACTCCGGC
CTGTACAATATGGTTGCCGGCTTCGACCACAAGGGTCTGATCGACGCAGCAAAGG
CCCAGTACGAACGCTTCCCGGGTTACCATAGCTTCTTCGGTCGTATGTCTGATCAA
ACTGTTATGCTGAGCGAGAAACTGGTAGAGGTGTCTCCATTCGACAGCGGTCGCG
TGTTCTATACTAACTCCGGCTCCGAGGCTAACGATACTATGGTGAAAATGCTGTGG
TTTCTGCACGCCGCAGAGGGCAAGCCGCAAAAACGCAAAATCCTGACTCGTCAAA
ACGCATACCACGGTGTAACTGCTGTTTCCGCTTCCATGACGGGTCTGCCGCACAA
CTCTGTATTCGGCCTGCCGCTGCCGGGTTTCGTTCACCTGACCTGTCCGCACTATT

GGCGTTACGGCGAAGAAGGTGAAACCGAAGAGCAGTTTGTTGCTCGTCTGGCCCG
CGAGCTGGAGGAAACTATCCAACGTGAAGGCGCGGACACGATTGCGGGCTTCTTT
GCTGAGCCGGTCATGGGCGCGGGCGGCGTAATCCCGCCGGCGAAAGGTTACTTC
CAGGCGATCCTGCCGATTCTGCGTAAGTACGACATCCCGGTTATCTCTGATGAAGT
TATCTGCGGCTTTGGTCGTACCGGTAATACTTGGGGTTGCGTTACCTATGACTTCA
CCCCGGATGCGATCATCTCCAGCAAAAATCTGACCGCCGGTTTCTTTCCGGTTGGT
GCTGTGATTCTGGGTCCGGAACTGAGCAAACGCCTGGAAACGGCGATCGAAGCTA
TCGAAGAGTTCCCGCACGGCTTTACGGCCGGCGGTCACCCGGTGGGTTGCGCTA
TCGCTCTGAAAGCAATCGATGTTGTGATGAATGAGGGTCTGGCAGAGAACGTGCG
CCGCCTGGCACCGCGTTTTGAGGAGCGTCTGAAACACATTGCCGAACGTCCGAAC
ATCGGTGAATATCGTGGCATCGGTTTTATGTGGGCACTGGAGGCTGTGAAAGACA
AAGCATCTAAAACCCCATTCGATGGTAATCTGTCTGTGAGCAAACGTATCGCTAAC
ACCTGTCAGGACCTGGGCCTGATCTGTAGCGCGCTGGGTCAGTCCGTTATCCTGA
GCCCGCCGTTCATCCTGACCGAGGCGCAAATGGATGAGATGTTTGACAAACTGGA
GAAGGCTCTGGACAAAGTCTTTGCGGAGGTGGCGTAA (SEQ ID NO. 12)

Amino acid sequence:

MNKPQSWEARAETYSLYGFTDMPSLHERGTVVVTHGEGPYVVDVNGRRYLDANSGL
YNMVAGFDHKGLIDAAKAQYERFPGYHSFFGRMSDQTVMLSEKLVEVSPFDSGRVFY
TNSGSEANDTMVKMLWFLHAAEGKPQKRKILTRQNAYHGVTAVSASMTGLPHNSVFG
LPLPGFVHLTCPHYWRYGEEGETEEQFVARLARELEETIQREGADTIAGFFAEPVMGA
GGVIPPAKGYFQAILPILRKYDIPVISDEVICGFGRTGNTWGCVTYDFTPDAIISSKNLTA
GFFPVGAVILGPELSKRLETAIEAIEEFPHGFTAGGHPVGCAIALKAIDVVMNEGLAENV
RRLAPRFEERLKHIAERPNIGEYRGIGFMWALEAVKDKASKTPFDGNLSVSKRIANTCQ
DLGLICSALGQSVILSPPFILTEAQMDEMFDKLEKALDKVFAEVA (SEQ ID NO. 4)

**Example 34c: Vfat1010**

DNA Sequence:

ATGAATAAACCACAAAGCTGGGAAGCGCGTGCTGAAACTTACTCTCTGTACGGCTT
CACTGATATGCCATCTCTGCACGAGCGTGGTACCGTGGTTGTCACCCACGGCGAG
GGCCCATACATCGTGGACGTCAACGGTCGCCGTTACCTGGACGCAAACTCCGGCC
TGTACAATATGGTTGCCGGCTTCGACCACAAGGGTCTGATCGACGCAGCAAAGGC
CCAGTACGAACGCTTCCCGGGTTACCATAGCTTCTTCGGTCGTATGTCTGATCAAA
CTGTTATGCTGAGCGAGAAACTGGTAGAGGTGTCTCCATTCGACAGCGGTCGCGT
GTTCTATACTAACTCCGGCTCCGAGGCTAACGATACTATGGTGAAAATGCTGTGGT

TTCTGCACGCCGCAGAGGGCAAGCCGCAAAAACGCAAAATCCTGACTCGTCAAAA
CGCATACCACGGTGTAACTGCTGTTTCCGCTTCCATGACGGGTATGCCGCACAAC
TCTGTATTCGGCCTGCCGCTGCCGGGTTTCGTTCACCTGACCTGTCCGCACTATTG
GCGTTACGGCGAAGAAGGTGAAACCGAAGAGCAGTTTGTTGCTCGTCTGGCCCGC
GAGCTGGAGGAAACTATCCAACGTGAAGGCGCGGACACGATTGCGGGCTTCTTTG
CTGAGCCGGTCATGGGCGCGGGCGGCGTAATCCCGCCGGCGAAAGGTTACTTCC
AGGCGATCCTGCCGATTCTGCGTAAGTACGACATCCCGGTTATCTCTGATGAAGTT
ATCTGCGGCTTTGGTCGTACCGGTAATACTTGGGGTTGCGTTACCTATGACTTCAC
CCCGGATGCGATCATCTCCAGCAAAAATCTGACCGCCGGTTTCTTTCCGGTTGGT
GCTGTGATTCTGGGTCCGGCACTGAGCAAACGCCTGGAAACGGCGATCGAAGCTA
TCGAAGAGTTCCCGCACGGCTTTACGGCCGGCGGTCACCCGGTGGGTTGCGCTA
TCGCTCTGAAAGCAATCGATGTTGTGATGAATGAGGGTCTGGCAGAGAACGTGCG
CCGCCTGGCACCGCGTTTTGAGGAGCGTCTGAAACACATTGCCGAACGTCCGAAC
ATCGGTGAATATCGTGGCATCGGTTTTATGTGGGCACTGGAGGCTGTGAAAGACA
AAGCATCTAAAACCCCATTCGATGGTAATCTGTCTGTGAGCAAACGTATCGCTAAC
ACCTGTCAGGACCTGGGCCTGATCTGTAGCGCGATGGGTCAGTCCGTTATCCTGA
GCCCGCCGTTCATCCTGACCGAGGCGCAAATGGATGAGATGTTTGACAAACTGGA
GAAGGCTCTGGACAAAGTCTTTGCGGAGGTGGCGTAA (SEQ ID NO. 13)

Amino acid sequence:

MNKPQSWEARAETYSLYGFTDMPSLHERGTVVVTHGEGPYIVDVNGRRYLDANSGLY
NMVAGFDHKGLIDAAKAQYERFPGYHSFFGRMSDQTVMLSEKLVEVSPFDSGRVFYT
NSGSEANDTMVKMLWFLHAAEGKPQKRKILTRQNAYHGVTAVSASMTGMPHNSVFG
LPLPGFVHLTCPHYWRYGEEGETEEQFVARLARELEETIQREGADTIAGFFAEPVMGA
GGVIPPAKGYFQAILPILRKYDIPVISDEVICGFGRTGNTWGCVTYDFTPDAIISSKNLTA
GFFPVGAVILGPALSKRLETAIEAIEEFPHGFTAGGHPVGCAIALKAIDVVMNEGLAENV
RRLAPRFEERLKHIAERPNIGEYRGIGFMWALEAVKDKASKTPFDGNLSVSKRIANTCQ
DLGLICSAMGQSVILSPPFILTEAQMDEMFDKLEKALDKVFAEVA (SEQ ID NO. 5)

**Example 34d: Vfat1020**

DNA Sequence:

ATGAATAAACCACAAAGCTGGGAAGCGCGTGCTGAAACTTACTCTCTGTACGGCTT CACTGATATGCCATCTCTGCACGAGCGTGGTACCGTGGTTGTCACCCACGGCGAG GGCCCATACGTGGTGGACGTCAACGGTCGCCGTTACCTGGACGCAAACTCCGGC CTGTACAATATGGTTGCCGGCTTCGACCACAAGGGTCTGATCGACGCAGCAAAGG CCCAGTACGAACGCTTCCCGGGTTACCATAGCTTCTTCGGTCGTATGTCTGATCAA ACTGTTATGCTGAGCGAGAAACTGGTAGAGGTGTCTCCATTCGACAGCGGTCGCG TGTTCTATACTAACTCCGGCTCCGAGGCTAACGATACTATGGTGAAAATGCTGTGG TTTCTGCACGCCGCAGAGGGCAAGCCGCAAAAACGCAAAATCCTGACTCGTCAAA ACGCATACCACGGTGTAACTGCTGTTTCCGCTTCCATGACGGGTCTGCCGCACAA CTCTGTATTCGGCCTGCCGCTGCCGGGTTTCGTTCACCTGGGTTGTCCGCACTATT GGCGTTACGGCGAAGAAGGTGAAACCGAAGAGCAGTTTGTTGCTCGTCTGGCCCG CGAGCTGGAGGAAACTATCCAACGTGAAGGCGCGGACACGATTGCGGGCTTCTTT GCTGAGCCGGTCATGGGCGCGGGCGGCGTAATCCCGCCGGCGAAAGGTTACTTC CAGGCGATCCTGCCGATTCTGCGTAAGTACGACATCCCGGTTATCTCTGATGAAGT TATCTGCGGCTTTGGTCGTACCGGTAATACTTGGGGTTGCGTTACCTATGACTTCA CCCCGGATGCGATCATCTCCAGCAAAAATCTGACCGCCGGTTTCTTTCCGGTTGGT GCTGTGATTCTGGGTCCGGAACTGAGCAAACGCCTGGAAACGGCGATCGAAGCTA TCGAAGAGTTCCCGCACGGCTTTACGGCCGGCGGTCACCCGGTGGGTTGCGCTA TCGCTCTGAAAGCAATCGATGTTGTGATGAATGAGGGTCTGGCAGAGAACGTGCG CCGCCTGGCACCGCGTTTTGAGGAGCGTCTGAAACACATTGCCGAACGTCCGAAC ATCGGTGAATATCGTGGCATCGGTTTTATGTGGGCACTGGAGGCTGTGAAAGACA AAGCATCTAAAACCCCATTCGATGGTAATCTGTCTGTGAGCAAACGTATCGCTAAC ACCTGTCAGGACCTGGGCCTGATCTGTAGCGCGATGGGTCAGTCCGTTATCCTGA GCCCGCCGTTCATCCTGACCGAGGCGCAAATGGATGAGATGTTTGACAAACTGGA GAAGGCTCTGGACAAAGTCTTTGCGGAGGTGGCGTAA (SEQ ID NO. 14)

Amino acid sequence:

MNKPQSWEARAETYSLYGFTDMPSLHERGTVVVTHGEGPYVVDVNGRRYLDANSGL YNMVAGFDHKGLIDAAKAQYERFPGYHSFFGRMSDQTVMLSEKLVEVSPFDSGRVFY TNSGSEANDTMVKMLWFLHAAEGKPQKRKILTRQNAYHGVTAVSASMTGLPHNSVFG LPLPGFVHLGCPHYWRYGEEGETEEQFVARLARELEETIQREGADTIAGFFAEPVMGA GGVIPPAKGYFQAILPILRKYDIPVISDEVICGFGRTGNTWGCVTYDFTPDAIISSKNLTA GFFPVGAVILGPELSKRLETAIEAIEEFPHGFTAGGHPVGCAIALKAIDVVMNEGLAENV RRLAPRFEERLKHIAERPNIGEYRGIGFMWALEAVKDKASKTPFDGNLSVSKRIANTCQ DLGLICAAMGQSVILSPPFILTEAQMDEMFDKLEKALDKVFAEVA (SEQ ID NO. 6)

**Example 34e: Vfat1030**

DNA Sequence:

ATGAATAAACCACAAAGCTGGGAAGCGCGTGCTGAAACTTACTCTCTGTACGGCTTCACTGATATGCCATCTCTGCACGAGCGTGGTACCGTGGTTGTCACCCACGGCGAGGGCCCATACATCGTGGACGTCCACGGTCGCCGTTACCTGGACGCAAACTCCGGCCTGTACAATATGGTTGCCGGCTTCGACCACAAGGGTCTGATCGACGCAGCAAAGGCCCAGTACGAACGCTTCCCGGGTTACCATAGCTTCTTCGGTCGTATGTCTGATCAAACTGTTATGCTGAGCGAGAAACTGGTAGAGGTGTCTCCATTCGACAGCGGTCGCGTGTTCTATACTAACTCCGGCTCCGAGGCTAACGATACTATGGTGAAAATGCTGTGGTTTCTGCACGCCGCAGAGGGCAAGCCGCAAAAACGCAAAATCCTGACTCGTCAAAACGCATACCACGGTGTAACTGCTGTTTCCGCTTCCATGACGGGTCTGCCGCACAACTCTGTATTCGGCCTGCCGCTGCCGGGTTTCGTTCACCTGAGCTGTCCGCACTATTGGCGTTACGGCGAAGAAGGTGAAACCGAAGAGCAGTTTGTTGCTCGTCTGGCCCGCGAGCTGGAGGAAACTATCCAACGTGAAGGCGCGGACACGATTGCGGGCTTCTTTGCTGAGCCGGTCATGGGCGCGGGCGGCGTAATCCCGCCGGCGAAAGGTTACTTCCAGGCGATCCTGCCGATTCTGCGTAAGTACGACATCCCGGTTATCTCTGATGAAGTTATCTGCGGCTTTGGTCGTACCGGTAATACTTGGGGTTGCGTTACCTATGACTTCACCCCGGATGCGATCATCTCCAGCAAAAATCTGACCGCCGGTTTCTTTCCGGTTGGTGCTGTGATTCTGGGTCCGGAACTGAGCAAACGCCTGGAAACGGCGATCGAAGCTATCGAAGAGTTCCCGCACGGCTTTACGGCCGGCGGTCACCCGGTGGGTTGCGCTATCGCTCTGAAAGCAATCGATGTTGTGATGAATGAGGGTCTGGCAGAGAACGTGCGCCGCCTGGCACCGCGTTTTGAGGAGCGTCTGAAACACATTGCCGAACGTCCGAACATCGGTGAATATCGTGGCATCGGTTTTATGTGGGCACTGGAGGCTGTGAAAGACAAAGCATCTAAAACCCCATTCGATGGTAATCTGTCTGTGAGCAAACGTATCGCTAACACCTGTCAGGACCTGGGCCTGATCTGTAGCGCGATGGGTCAGTCCGTTATCCTGAGCCCGCCGTTCATCCTGACCGAGGCGCAAATGGATGAGATGTTTGACAAACTGGAGAAGGCTCTGGACAAAGTCTTTGCGGAGGTGGCGTAA (SEQ ID NO. 15)

Amino acid sequence:

MNKPQSWEARAETYSLYGFTDMPSLHERGTVVVTHGEGPYIVDVHGRRYLDANSGLY NMVAGFDHKGLIDAAKAQYERFPGYHSFFGRMSDQTVMLSEKLVEVSPFDSGRVFYT NSGSEANDTMVKMLWFLHAAEGKPQKRKILTRQNAYHGVTAVSASMTGLPHNSVFGL PLPGFVHLSCPHYWRYGEEGETEEQFVARLARELEETIQREGADTIAGFFAEPVMGAG GVIPPAKGYFQAILPILRKYDIPVISDEVICGFGRTGNTWGCVTYDFTPDAIISSKNLTAG FFPVGAVILGPELSKRLETAIEAIEEFPHGFTAGGHPVGCAIALKAIDVVMNEGLAENVR RLAPRFEERLKHIAERPNIGEYRGIGFMWALEAVKDKASKTPFDGNLSVSKRIANTCQD LGLICSAMGQSVILSPPFILTEAQMDEMFDKLEKALDKVFAEVA (SEQ ID NO. 7)

**Example 35**

**Biotransformation of 3-isobutylidene-2-oxopentanedioic acid to Pregabalin**

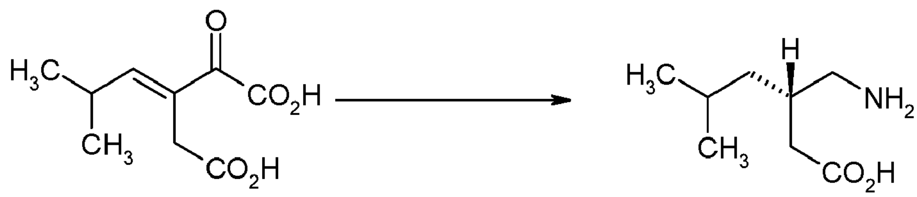

The genes for the *Pseudomonas putida* benzoylformate decarboxylase, *Enterobacter cloacae* pentaerythritol tetranitrate reductase, *Lactobacillus brevis* alcohol dehydrogenase, and *Vibrio fluvialis* ω-transaminase were cloned into expression vector pDSTRC52 (Pfizer Inc., USA) and put into the expression strain *E. coli* BDG62 (Pfizer Inc., USA). The culture was grown and enzyme production was induced as described in Example 22. Enzyme expression was determined by polyacrylamide gel electrophoresis using Novex gels and stains (Invitrogen Corporation Carlsbad, California).

Reactions (1.0mL) were carried out at 40°C in phosphate buffer (100mM, pH 6.4) with *P. putida* decarboxylase, *V. fluvialis* transaminase, *L. brevis* alcohol dehydrogenase, *E. cloacae* pentaerythritol tetranitrate reductase, and NADP (0.1mM) in one cell. 200μL 1 M 3-isobutylidene-2-oxopentanedioic acid, 100μL 1mM ThDp + 25mM MgSO4, 40μL 50mM PLP, 30μL isopropanol, 250μL 2M isopropylamine. Adjust pH to 6.4 for 24 hours, then adjust pH to 6.8 for additional 24 hours. An aliquot (0.5mL) of the reaction was treated with 1 M aqueous sodium bicarbonate (0.05mL) and Marfey's reagent (*N*-$\alpha$-(2,4-dinitro-5-fluorophenyl)alaninamide, 0.5mL of 5g/L solution in acetonitrile) at 40°C. After 1 h, the derivatization reactions were quenched with 0.05mL 1 N aqueous hydrochloric acid. The derivatized reaction samples were filtered and analyzed by UPLC (column: Agilent Eclipse Plus C18 column (100mm x 3.0mm, 1.8μm) eluted with 0.1% trifluoroacetic acid in water:acetonitrile (60:40, v/v) at 1.3mL/min. The column was maintained at 30°C and the effluent was monitored at 340nm and $ES^+$ mass spectroscopy. The reaction yielded a small amount of Pregabalin of which 82% was the desired S-isomer.

**Example 36**

**Biotransformation of 3-(2-methylpropyl)-2-oxopentanedioic acid to Pregabalin**

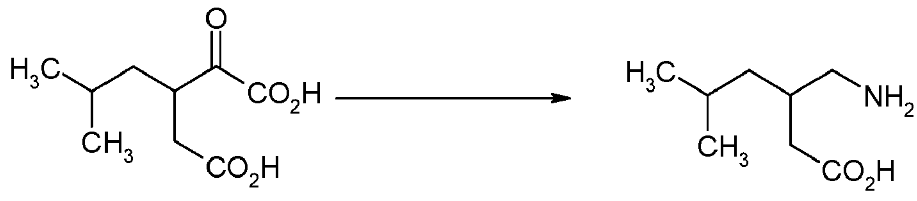

The genes for the *Pseudomonas putida* benzoylformate decarboxylase and *Vibrio fluvialis* ω-transaminase were cloned into expression vector pDSTRC52 (Pfizer Inc., USA) and put into the expression strain *E. coli* BDG62 (Pfizer Inc., USA). The culture was grown and enzyme production was induced as described in Example 22. Enzyme expression was determined by polyacrylamide gel electrophoresis using Novex gels and stains (Invitrogen Corporation Carlsbad, California).

Reactions (2.0mL) were carried out at 40°C in phosphate buffer (100mM, pH 6.4) with 500μL of culture (42.5mg dry cell weight) from a 24 hour fermentation tanks with *P. putida* decarboxylase and *V. fluvialis* ω-transaminase cloned in one plasmid, 400μL 0.5M 3-(2-methylpropyl)-2-oxopentanedioic acid, 200uL 10 x ThDP (final 0.1mM) and $MgSO_4$ (final 2.5mM), 80μL 50mM PLP (final 2mM), 300μL 2M isopropylamine (final 0.3M). Adjust pH to 6.4 and incubate at 45°C for 24 hours, then adjust pH to 6.8 for additional 24 hours. An aliquot (0.5mL) of the reaction was treated with 1 M aqueous sodium bicarbonate (0.05mL) and Marfey's reagent (*N*-$\alpha$-(2,4-dinitro-5-fluorophenyl)alaninamide, 0.5mL of 5g/L solution in acetonitrile) at 40°C. After 1h, the derivatization reactions were quenched with 0.05mL 1N aqueous hydrochloric acid. The derivatized reaction samples were filtered and analyzed by UPLC (column: Agilent Eclipse Plus C18 column (100mm x 3.0mm, 1.8μm) eluted with 0.1% trifluoroacetic acid in water:acetonitrile (60:40, v/v) at 1.3mL/min. The column was maintained at 30°C and the effluent was monitored at 340nm and $ES^+$ mass spectroscopy. The reaction yielded 100μg/mL of Pregabalin of which 65% was the desired S-isomer.

**Example 37**

**Preparation of (S)-Pregabalin via hydrolysis of 5-methoxy-4-(2-methylpropyl)-dihydrofuran-2(3H)-one and enzymatic transamination**

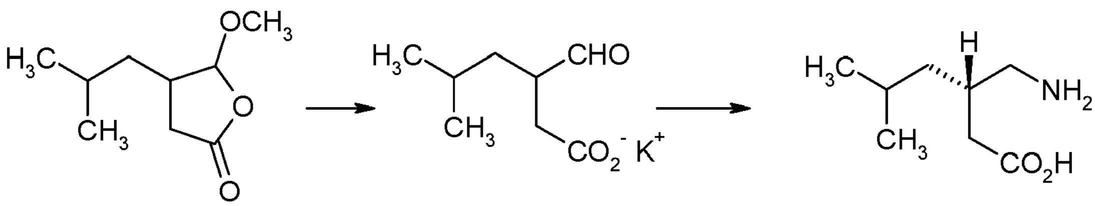

5-Methoxy-4-(2-methylpropyl)-dihydrofuran-2(3H)-one (2.58g, 15mmol, see example 13) was suspended in DIW (5.2g) and cooled in an ice/water bath. Aq KOH (50% w/w, 1.77g, 1.05eq) was added dropwise *via* syringe over 5mins. The reaction was removed from the ice/water bath and stirred at rt for 90mins. The pH was adjusted to 7.0 using formic acid. The reaction mixture was then used as feedstock in the subsequent transaminase reaction.

Transaminase enzyme solution (12.5g), PLP (35mg), DIW (15mL) and 4.0M isopropylamine.HCl aq soln (7.5mL, 30mmol) were charged to a 100mL flask and warmed to 45°C. The hydrolysis reaction was added in one portion followed by DIW (6 mL, used as a vessel rinse). The pH of the reaction was adjusted to 7.25 using neat isopropylamine and the reaction was stirred at 45°C until reaction completion. The reaction mixture was then heated to an internal temperature of 55°C and the pH adjusted to 4.0 using 95% formic acid. Darco carbon (125mg) was added and the mixture was allowed to cool to room temperature before cooling on ice/water for 20 minutes. The mixture was then filtered through Whatman paper no. 3. The filtrate was concentrated to 1/3 of its weight and then heated to 55°C. The pH of the solution was then adjusted to pH 7.5 using 50% KOH after which the solution was cooled to ambient and then to 0-5°C in an ice/water bath. Precipitation of product was observed in the cooldown. The slurry was filtered and washed with DIW/EtOH (10mL, 1:1, 0°C). The white precipitate was dried for 12 hours in a vacuum oven (45°C) to yield (S)-Pregabalin in 61% yield, 98.6% w/w purity and 99. 8% ee (preferred S-isomer).

**Example 38**

**Preparation of (S)-Pregabalin via hydrolysis of 5-butoxy-4-(2-methylpropyl)-dihydrofuran-2(3H)-one and enzymatic transamination.**

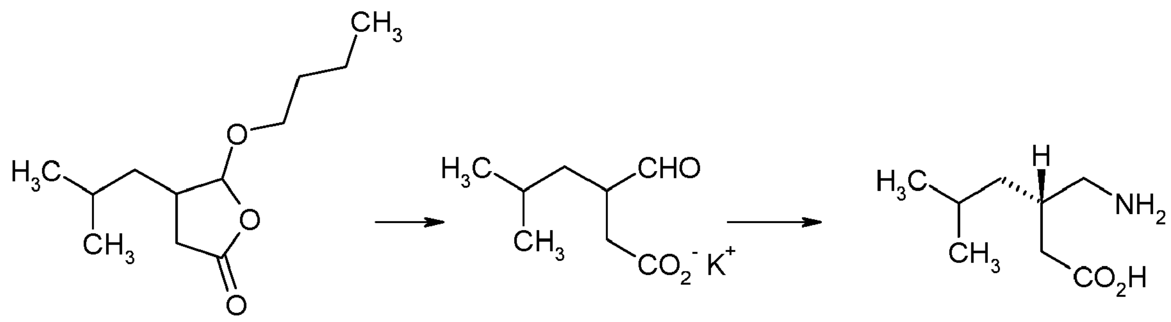

5-Butoxy-4-(2-methylpropyl)-dihydrofuran-2(3H)-one (3.21 g, 15mmol, see example 13C) was suspended in DIW (8.5g) and cooled in an ice/water bath. Aq KOH (50% w/w, 2.02g, 1.2eq) was added dropwise via syringe over 5mins. The reaction was removed from the ice/water bath and stirred at rt for 90mins. The reaction pH was then adjusted to pH 7.0 using formic acid, and the reaction mixture was then used as feedstock in the subsequent transaminase reaction.

Transaminase enzyme solution (12.5g), PLP (35mg), DIW (15mL) and 4.0M isopropylamine.HCl solution (7.5mL, 30mmol) were charged to a 100mL flask and warmed to 45°C. The hydrolysis reaction (see above) was added in one portion followed by DIW (6mL, used as a vessel rinse). The pH of the reaction was adjusted to 7.25 using neat isopropylamine and the reaction was stirred at 45°C.

The reaction mixture was heated to an internal temperature of 55°C and pH adjusted to 4.0 using 95% formic acid. Darco carbon (125mg) was added and the mixture was allowed to cool to room temperature before being cooled on ice/water for 20 minutes. The mixture was then filtered through Whatman paper no 3. The filtrate was concentrated to 1/3 of its weight then heated to 55°C. The pH of the solution was then adjusted to pH 7.5 using 50% KOH after which the solution was cooled to ambient and then to 0-5°C in an ice/water bath. Precipitation of product was observed in the cooldown. The slurry was filtered and washed with DIW/EtOH (10mL, 1:1, 0°C). The white precipitate was dried for 12 hours in a vacuum oven (45°C) to yield (S)-Pregabalin in 51% yield, 98.4% w/w purity and 99.9% e.e preferred S-isomer.

**Example 39**

**Preparation of (*S*)-Pregabalin from 5-hydroxy-4-(2-methylpropyl)-dihydrofuran-2(3H)-one via enzymatic transamination**

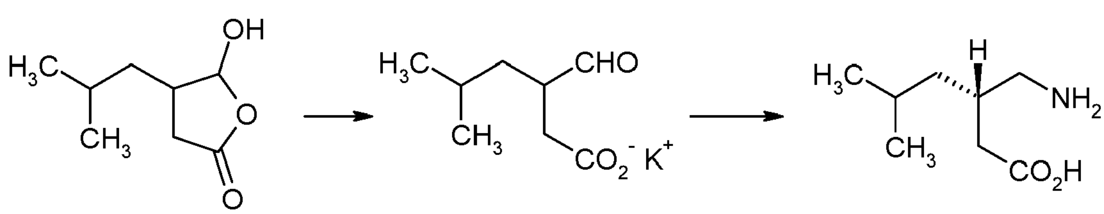

5-Hydroxy-4-(2-methylpropyl)-dihydrofuran-2(3H)-one (2.0g, 12.5mmol) was suspended in DIW (8.5g) and cooled in an ice/water bath. Potassium carbonate (0.863g, 6.3mmol) was added portion wise over the course of 5mins. The reaction was removed from the ice/water bath and stirred at rt for 90mins. The pH was adjusted to 7.0 using formic acid and the reaction mixture was then used as feedstock in the subsequent transaminase reaction.

Transaminase enzyme solution (10.4g), PLP (30mg), DIW (12.5mL) and aq 4.0M isopropylamine.HCl aq soln (6.3mL, 30mmol) were charged to a 100mL flask and warmed to 45°C. The hydrolysis reaction was added in one portion followed by DIW (5mL, used as a vessel rinse). The pH of the reaction was adjusted to 7.25 using neat isopropylamine and the reaction was stirred at 45°C. The reaction mixture was heated to an internal temperature of 55°C and adjusted

to pH 4.0 using 95% formic acid. Darco carbon (125mg) was added and the mixture was allowed to cool to room temperature before being cooled on ice/water for 20 minutes. The mixture was then filtered through Whatman paper no 3. The filtrate was concentrated to 1/3 of its weight then heated to 55°C. The pH of the solution was then adjusted to pH 7.5 using 50% KOH after which the solution was cooled to ambient and then to 0-5°C in an ice/water bath. Precipitation of product was observed in the cooldown. The slurry was filtered and washed with DIW/EtOH (10mL, 1:1, 0°C). The white precipitate was dried for 12 hours in a vacuum oven (45°C) to yield (S)-Pregabalin in 61% yield, 98.3% w/w purity and 99.9% e.e of the preferred S-isomer.

**Example 40**

**Preparation of (R/S)-3-aminomethyl-5-methylhexanoic acid via Reductive amination of 5-hydroxy-4-(2-methylprop-1-en-1-yl)furan-2(5*H*)-one**

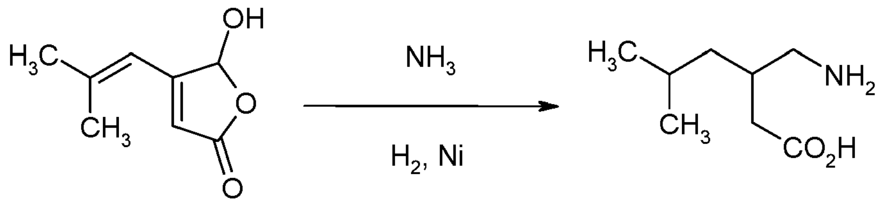


A 0.01M solution of 5-hydroxy-4-(2-methylprop-1-en-1-yl)furan-2(5H)-one in 30% aqueous ammonia solution was hydrogenated (10 bar, ambient temperature) for 48h in the presence of 10mol% Raney Nickel catalyst. The catalyst was filtered and the solution concentrated to leave a solid. The product was isolated by addition of hydrochloric acid to a methanol suspension and found to be pure 3-aminomethyl-5-methylhexanoic acid hydrochloride.

The use of palladium as a catalyst gave mixtures of 3-aminomethyl-5-methylhexanoic acid and the corresponding secondary amine, 3-[(2-carboxymethyl-4-methyl-pentylamino)-methyl]-5-methyl-hexanoic acid.

**Example 41**

**Conversion of R-3-aminomethyl-5-methylhexanoic acid to 5-hydroxy-4-(2-methylpropyl)-3,4-dihydro-5*H*-2-furanone using a transaminase**

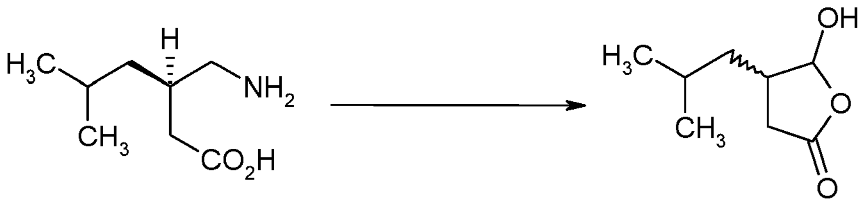

A solution of R-3-aminomethyl-5-methylhexanoic acid in D.I. water at pH 7.5/ 45°C is stirred with a transaminase enzyme lysate, or whole cell preparation containing pyridoxal phosphate (PLP) and acetone. The isopropylamine produced is removed via a nitrogen sweep. Analysis of the solution after 24h show 3-aminomethyl-5-methyl-hexanoic acid with a lower e.e. and the presence of compound 5-hydroxy-4-(2-methylpropyl)-3,4-dihydro-5*H*-2-furanone. The lowering of the chiral purity of the 5-hydroxy-4-(2-methylpropyl)-3,4-dihydro-5*H*-2-furanone is due to the selective conversion of (4*S*)-5-hydroxy-4-(2-methylpropyl)-3,4-dihydro-5*H*-2-furanone to S-pregabalin.

**Example 42**

**De-racemisation of *rac*-pregabalin using a transaminase**

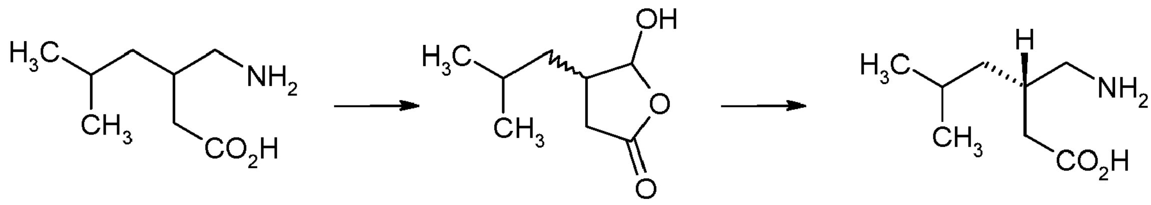

A solution of racemic pregabalin in DIW at pH 7.5 is treated with a suitable transaminase lysate. PLP is added and the reaction progressed for 12h at 45°C with a nitrogen sweep. Then, isopropylamine is added and the reaction is continued for a further 12h. The product is isolated as in Example 38 to afford enantiomerically enriched pregabalin.

It is also possible to effect the transformations exemplified in Examples 41 and 42 using a suitable amine oxidase/ imine reductase enzyme system with a co-factor such as NADP.

**SEQUENCE LISTING**

**<u>SEQ ID NO. 1</u>**

<u>Generic Vfat aa sequence</u>

MNKPQSWEARAETYSLYGFTDMPSLH**X**$^{27}$RGTVVVTHGEGPY**X**$^{41}$VDV**X**$^{45}$GRRYLDAN
SGLYNMVAGFDHKGLIDAAKAQYERFPGYHSFFGRMSDQTVMLSEKLVEVSPFDSGR
VFYTNSGSEANDTMVKMLWFLHAAEGKPQKRKILTR**X**$^{147}$NAYHGVTAVSASMTG**X**$^{163}$P
**X**$^{165}$NSVFGLPLPGFVHL**X**$^{180}$CPHYWRYGEEGETEEQFVARLARELEETIQREGADTIAG
FFAEPVMGAGGVIPPAKGYFQAILPILRKYDIPVISDEVICGFGRTGNTWGCVTYDFTPD
AIISSKNLTAGFFPVGAVILGPEL**X**$^{304}$KRLETAIEAIEEFPHGFTA**X**$^{324}$GHPVGCAIALKAID
VVMNEGLAENVRRLAPRFEERLKHIAERPNIGEYRGIGFMWALEAVKDKASKTPFDGN
LSVS**X**$^{401}$RIANTC**X**$^{408}$DLGLIC**X**$^{415}$**X**$^{416}$**X**$^{417}$GQSVIL**X**$^{424}$PPFILTEAQMDEMFDKLEKALD
KVFAEVA

**X**$^{27}$ is selected from glutamine (Q) and glutamic acid (E); **X**$^{41}$ is selected from isoleucine (I) and valine (V); **X**$^{45}$ is selected from asparigine (N) and histidine (H); **X**$^{147}$ is selected from asparigine (N) and glutamine (Q); **X**$^{163}$ is selected from leucine (L) and methionine (M); **X**$^{165}$ is selected from tyrosine (Y) and histidine (H); **X**$^{180}$ is selected from threonine (T); glycine (G) and serine (S); **X**$^{304}$ is selected from alanine (A) and serine (S); **X**$^{324}$ is selected from glycine (G) and serine (S); **X**$^{401}$ is selected from lysine (K) and glutamic acid (E); **X**$^{408}$ is selected from threonine (T) and glutamine (Q); **X**$^{415}$ is selected from serine (S) and alanine (A); **X**$^{416}$ is selected from proline (P) and alanine (A); **X**$^{417}$ is selected from leucine (L) and methionine (M); and **X**$^{424}$ is selected from cysteine (C) and serine (S).

**<u>SEQ ID NO. 2</u>**

<u>Vfat888 aa sequence</u>

MNKPQSWEARAETYSLYGFTDMPSLHQRGTVVVTHGEGPYIVDVNGRRYLDANSGLYNMVAGFDHKGLIDAAKAQYERFPGYHSFFGRMSDQTVMLSEKLVEVSPFDSGRVFYTNSGSEANDTMVKMLWFLHAAEGKPQKRKILTRNNAYHGVTAVSASMTGLPYNSVFGLPLPGFVHLTCPHYWRYGEEGETEEQFVARLARELEETIQREGADTIAGFFAEPVMGAGGVIPPAKGYFQAILPILRKYDIPVISDEVICGFGRTGNTWGCVTYDFTPDAIISSKNLTAGFFPVGAVILGPELAKRLETAIEAIEEFPHGFTASGHPVGCAIALKAIDVVMNEGLAENV

RRLAPRFEERLKHIAERPNIGEYRGIGFMWALEAVKDKASKTPFDGNLSVSERIANTCTDLGLICSPMGQSVILCPPFILTEAQMDEMFDKLEKALDKVFAEVA

**SEQ ID NO. 3**
Vfat906 aa sequence

MNKPQSWEARAETYSLYGFTDMPSLHERGTVVVTHGEGPYIVDVNGRRYLDANSGLYNMVAGFDHKGLIDAAKAQYERFPGYHSFFGRMSDQTVMLSEKLVEVSPFDSGRVFYTNSGSEANDTMVKMLWFLHAAEGKPQKRKILTRNNAYHGVTAVSASMTGLPYNSVFGLPLPGFVHLTCPHYWRYGEEGETEEQFVARLARELEETIQREGADTIAGFFAEPVMGAGGVIPPAKGYFQAILPILRKYDIPVISDEVICGFGRTGNTWGCVTYDFTPDAIISSKNLTAGFFPVGAVILGPELSKRLETAIEAIEEFPHGFTAGGHPVGCAIALKAIDVVMNEGLAENVRRLAPRFEERLKHIAERPNIGEYRGIGFMWALEAVKDKASKTPFDGNLSVSKRIANTCQDLGLICSALGQSVILCPPFILTEAQMDEMFDKLEKALDKVFAEVA

**SEQ ID NO. 4**
Vfat999 aa sequence

MNKPQSWEARAETYSLYGFTDMPSLHERGTVVVTHGEGPYVVDVNGRRYLDANSGLYNMVAGFDHKGLIDAAKAQYERFPGYHSFFGRMSDQTVMLSEKLVEVSPFDSGRVFYTNSGSEANDTMVKMLWFLHAAEGKPQKRKILTRQNAYHGVTAVSASMTGLPHNSVFGLPLPGFVHLTCPHYWRYGEEGETEEQFVARLARELEETIQREGADTIAGFFAEPVMGAGGVIPPAKGYFQAILPILRKYDIPVISDEVICGFGRTGNTWGCVTYDFTPDAIISSKNLTAGFFPVGAVILGPELSKRLETAIEAIEEFPHGFTAGGHPVGCAIALKAIDVVMNEGLAENVRRLAPRFEERLKHIAERPNIGEYRGIGFMWALEAVKDKASKTPFDGNLSVSKRIANTCQDLGLICSALGQSVILSPPFILTEAQMDEMFDKLEKALDKVFAEVA

**SEQ ID NO. 5**
Vfat1010 aa sequence

MNKPQSWEARAETYSLYGFTDMPSLHERGTVVVTHGEGPYIVDVNGRRYLDANSGLY NMVAGFDHKGLIDAAKAQYERFPGYHSFFGRMSDQTVMLSEKLVEVSPFDSGRVFYT NSGSEANDTMVKMLWFLHAAEGKPQKRKILTRQNAYHGVTAVSASMTGMPHNSVFG LPLPGFVHLTCPHYWRYGEEGETEEQFVARLARELEETIQREGADTIAGFFAEPVMGA GGVIPPAKGYFQAILPILRKYDIPVISDEVICGFGRTGNTWGCVTYDFTPDAIISSKNLTA GFFPVGAVILGPALSKRLETAIEAIEEFPHGFTAGGHPVGCAIALKAIDVVMNEGLAENV RRLAPRFEERLKHIAERPNIGEYRGIGFMWALEAVKDKASKTPFDGNLSVSKRIANTCQ DLGLICSAMGQSVILSPPFILTEAQMDEMFDKLEKALDKVFAEVA

**SEQ ID NO. 6**
Vfat1020 aa sequence

MNKPQSWEARAETYSLYGFTDMPSLHERGTVVVTHGEGPYVVDVNGRRYLDANSGL YNMVAGFDHKGLIDAAKAQYERFPGYHSFFGRMSDQTVMLSEKLVEVSPFDSGRVFY TNSGSEANDTMVKMLWFLHAAEGKPQKRKILTRQNAYHGVTAVSASMTGLPHNSVFG LPLPGFVHLGCPHYWRYGEEGETEEQFVARLARELEETIQREGADTIAGFFAEPVMGA GGVIPPAKGYFQAILPILRKYDIPVISDEVICGFGRTGNTWGCVTYDFTPDAIISSKNLTA GFFPVGAVILGPELSKRLETAIEAIEEFPHGFTAGGHPVGCAIALKAIDVVMNEGLAENV RRLAPRFEERLKHIAERPNIGEYRGIGFMWALEAVKDKASKTPFDGNLSVSKRIANTCQ DLGLICAAMGQSVILSPPFILTEAQMDEMFDKLEKALDKVFAEVA

**SEQ ID NO. 7**
Vfat1030 aa sequence

MNKPQSWEARAETYSLYGFTDMPSLHERGTVVVTHGEGPYIVDVHGRRYLDANSGLY NMVAGFDHKGLIDAAKAQYERFPGYHSFFGRMSDQTVMLSEKLVEVSPFDSGRVFYT NSGSEANDTMVKMLWFLHAAEGKPQKRKILTRQNAYHGVTAVSASMTGLPHNSVFGL PLPGFVHLSCPHYWRYGEEGETEEQFVARLARELEETIQREGADTIAGFFAEPVMGAG GVIPPAKGYFQAILPILRKYDIPVISDEVICGFGRTGNTWGCVTYDFTPDAIISSKNLTAG FFPVGAVILGPELSKRLETAIEAIEEFPHGFTAGGHPVGCAIALKAIDVVMNEGLAENVR RLAPRFEERLKHIAERPNIGEYRGIGFMWALEAVKDKASKTPFDGNLSVSKRIANTCQD LGLICSAMGQSVILSPPFILTEAQMDEMFDKLEKALDKVFAEVA

**SEQ ID NO. 8**
*Lycopersicon esculentum* (tomato) 12-Oxophytodienoate Reductase 1 protein sequence:

MENKVVEEKQVDKIPLMSPCKMGKFELCHRVVLAPLTRQRSYGYIPQPHAILHYSQRS
TNGGLLIGEATVISETGIGYKDVPGIWTKEQVEAWKPIVDAVHAKGGIFFCQIWHVGRV
SNKDFQPNGEDPISCTDRGLTPQIRSNGIDIAHFTRPRRLTTDEIPQIVNEFRVAARNAI
EAGFDGVEIHGAHGYLIDQFMKDQVNDRSDKYGGSLENRCRFALEIVEAVANEIGSDR
VGIRISPFAHYNEAGDTNPTALGLYMVESLNKYDLAYCHVVEPRMKTAWEKIECTESLV
PMRKAYKGTFIVAGGYDREDGNRALIEDRADLVAYGRLFISNPDLPKRFELNAPLNKYN
RDTFYTSDPIVGYTDYPFLETMT

**SEQ ID NO. 9**

*Lycopersicon esculentum* (tomato) 12-Oxophytodienoate Reductase 1 codon optimized sequence:

ATGGAAAACAAAGTTGTGGAAGAAAAACAGGTTGATAAAATCCCGCTGATGAGCCC
GTGTAAAATGGGTAAATTCGAGCTGTGTCATCGCGTTGTACTGGCACCGCTGACTC
GTCAGCGTTCTTATGGTTACATTCCGCAGCCGCACGCAATCCTGCATTACTCTCAG
CGCAGCACCAACGGTGGCCTGCTGATCGGTGAAGCAACCGTGATCAGCGAAACT
GGCATCGGTTACAAAGATGTGCCGGGTATCTGGACGAAAGAGCAGGTTGAGGCCT
GGAAACCGATCGTCGACGCGGTGCATGCCAAAGGTGGTATTTTCTTTTGTCAGATC
TGGCACGTTGGTCGTGTATCCAACAAAGATTTTCAGCCGAACGGCGAAGATCCGA
TTTCCTGTACTGACCGCGGCCTGACCCCGCAGATCCGTTCCAACGGCATTGACAT
TGCCCACTTCACCCGTCCACGTCGCCTGACTACTGACGAGATTCCGCAGATCGTG
AACGAGTTCCGCGTTGCAGCGCGTAATGCTATTGAAGCGGGTTTCGATGGCGTCG
AGATTCATGGTGCCCACGGTTACCTGATCGACCAATTCATGAAAGACCAAGTTAAC
GACCGCAGCGATAAGTATGGCGGTTCTCTGGAGAACCGTTGTCGCTTCGCGCTGG
AAATCGTTGAAGCAGTAGCCAACGAGATTGGCTCCGACCGTGTTGGTATCCGTATC
TCTCCATTCGCACACTACAACGAAGCGGGCGACACTAACCCGACCGCACTGGGCC
TGTATATGGTGGAGAGCCTGAATAAATACGACCTGGCGTATTGTCACGTGGTCGA
GCCGCGCATGAAAACCGCCTGGGAAAAGATTGAGTGCACCGAAAGCCTGGTGCC
GATGCGTAAAGCCTACAAAGGCACCTTCATCGTAGCTGGTGGCTACGACCGTGAA
GACGGTAACCGCGCTCTGATCGAAGACCGTGCCGACCTGGTTGCGTACGGTCGT
CTGTTCATCAGCAACCCAGACCTGCCGAAGCGTTTTGAACTGAACGCTCCGCTGA
ACAAATACAACCGTGACACTTTCTACACTTCCGACCCGATCGTTGGTTACACCGAT
TACCCGTTTCTGGAAACTATGACTTAATAA

**SEQ ID NO. 10**

Vfat 888 DNA SEQUENCE

ATGAATAAACCACAAAGCTGGGAAGCGCGTGCTGAAACTTACTCTCTGTACGGCTT
CACTGATATGCCATCTCTGCACCAGCGTGGTACCGTGGTTGTCACCCACGGCGAG
GGCCCATACATCGTGGACGTCAACGGTCGCCGTTACCTGGACGCAAACTCCGGCC
TGTACAATATGGTTGCCGGCTTCGACCACAAGGGTCTGATCGACGCAGCAAAGGC
CCAGTACGAACGCTTCCCGGGTTACCATAGCTTCTTCGGTCGTATGTCTGATCAAA
CTGTTATGCTGAGCGAGAAACTGGTAGAGGTGTCTCCATTCGACAGCGGTCGCGT
GTTCTATACTAACTCCGGCTCCGAGGCTAACGATACTATGGTGAAAATGCTGTGGT
TTCTGCACGCCGCAGAGGGCAAGCCGCAAAAACGCAAAATCCTGACTCGTAACAA
CGCATACCACGGTGTAACTGCTGTTTCCGCTTCCATGACGGGTCTGCCGTACAACT
CTGTATTCGGCCTGCCGCTGCCGGGTTTCGTTCACCTGACCTGTCCGCACTATTG
GCGTTACGGCGAAGAAGGTGAAACCGAAGAGCAGTTTGTTGCTCGTCTGGCCCGC
GAGCTGGAGGAAACTATCCAACGTGAAGGCGCGGACACGATTGCGGGCTTCTTTG
CTGAGCCGGTCATGGGCGCGGGCGGCGTAATCCCGCCGGCGAAAGGTTACTTCC
AGGCGATCCTGCCGATTCTGCGTAAGTACGACATCCCGGTTATCTCTGATGAAGTT
ATCTGCGGCTTTGGTCGTACCGGTAATACTTGGGGTTGCGTTACCTATGACTTCAC
CCCGGATGCGATCATCTCCAGCAAAAATCTGACCGCCGGTTTCTTTCCGGTTGGT
GCTGTGATTCTGGGTCCGGAACTGGCGAAACGCCTGGAAACGGCGATCGAAGCTA
TCGAAGAGTTCCCGCACGGCTTTACGGCCAGCGGTCACCCGGTGGGTTGCGCTAT
CGCTCTGAAAGCAATCGATGTTGTGATGAATGAGGGTCTGGCAGAGAACGTGCGC
CGCCTGGCACCGCGTTTTGAGGAGCGTCTGAAACACATTGCCGAACGTCCGAACA
TCGGTGAATATCGTGGCATCGGTTTTATGTGGGCACTGGAGGCTGTGAAAGACAA
AGCATCTAAAACCCCATTCGATGGTAATCTGTCTGTGAGCGAGCGTATCGCTAACA
CCTGTACCGACCTGGGCCTGATCTGTAGCCCGATGGGTCAGTCCGTTATCCTGTG
CCCGCCGTTCATCCTGACCGAGGCGCAAATGGATGAGATGTTTGACAAACTGGAG
AAGGCTCTGGACAAAGTCTTTGCGGAGGTGGCGTAA

**SEQ ID NO. 11**

Vfat 906 DNA SEQUENCE

ATGAATAAACCACAAAGCTGGGAAGCGCGTGCTGAAACTTACTCTCTGTACGGCTT
CACTGATATGCCATCTCTGCACgAGCGTGGTACCGTGGTTGTCACCCACGGCGAG
GGCCCATACATCGTGGACGTCAACGGTCGCCGTTACCTGGACGCAAACTCCGGCC
TGTACAATATGGTTGCCGGCTTCGACCACAAGGGTCTGATCGACGCAGCAAAGGC
CCAGTACGAACGCTTCCCGGGTTACCATAGCTTCTTCGGTCGTATGTCTGATCAAA
CTGTTATGCTGAGCGAGAAACTGGTAGAGGTGTCTCCATTCGACAGCGGTCGCGT
GTTCTATACTAACTCCGGCTCCGAGGCTAACGATACTATGGTGAAAATGCTGTGGT
TTCTGCACGCCGCAGAGGGCAAGCCGCAAAAACGCAAAATCCTGACTCGTaacAAC
GCATACCACGGTGTAACTGCTGTTTCCGCTTCCATGACGGGTCTGCCGTACAACTC
TGTATTCGGCCTGCCGCTGCCGGGTTTCGTTCACCTGACCTGTCCGCACTATTGG
CGTTACGGCGAAGAAGGTGAAACCGAAGAGCAGTTTGTTGCTCGTCTGGCCCGCG
AGCTGGAGGAAACTATCCAACGTGAAGGCGCGGACACGATTGCGGGCTTCTTTGC
TGAGCCGGTCATGGGCGCGGGCGGCGTAATCCCGCCGGCGAAAGGTTACTTCCA
GGCGATCCTGCCGATTCTGCGTAAGTACGACATCCCGGTTATCTCTGATGAAGTTA

TCTGCGGCTTTGGTCGTACCGGTAATACTTGGGGTTGCGTTACCTATGACTTCACC
CCGGATGCGATCATCTCCAGCAAAAATCTGACCGCCGGTTTCTTTCCGGTTGGTG
CTGTGATTCTGGGTCCGGAACTGAGCAAACGCCTGGAAACGGCGATCGAAGCTAT
CGAAGAGTTCCCGCACGGCTTTACGGCCggcGGTCACCCGGTGGGTTGCGCTATC
GCTCTGAAAGCAATCGATGTTGTGATGAATGAGGGTCTGGCAGAGAACGTGCGCC
GCCTGGCACCGCGTTTTGAGGAGCGTCTGAAACACATTGCCGAACGTCCGAACAT
CGGTGAATATCGTGGCATCGGTTTTATGTGGGCACTGGAGGCTGTGAAAGACAAA
GCATCTAAAACCCCATTCGATGGTAATCTGTCTGTGAGCaaaCGTATCGCTAACACC
TGTcagGACCTGGGCCTGATCTGTAGCgCGCTGGGTCAGTCCGTTATCCTGTGCCC
GCCGTTCATCCTGACCGAGGCGCAAATGGATGAGATGTTTGACAAACTGGAGAAG
GCTCTGGACAAAGTCTTTGCGGAGGTGGCGTAA

**SEQ ID NO. 12**
Vfat 999 DNA SEQUENCE

ATGAATAAACCACAAAGCTGGGAAGCGCGTGCTGAAACTTACTCTCTGTACGGCTT
CACTGATATGCCATCTCTGCACGAGCGTGGTACCGTGGTTGTCACCCACGGCGAG
GGCCCATACGTGGTGGACGTCAACGGTCGCCGTTACCTGGACGCAAACTCCGGC
CTGTACAATATGGTTGCCGGCTTCGACCACAAGGGTCTGATCGACGCAGCAAAGG
CCCAGTACGAACGCTTCCCGGGTTACCATAGCTTCTTCGGTCGTATGTCTGATCAA
ACTGTTATGCTGAGCGAGAAACTGGTAGAGGTGTCTCCATTCGACAGCGGTCGCG
TGTTCTATACTAACTCCGGCTCCGAGGCTAACGATACTATGGTGAAAATGCTGTGG
TTTCTGCACGCCGCAGAGGGCAAGCCGCAAAAACGCAAAATCCTGACTCGTCAAA
ACGCATACCACGGTGTAACTGCTGTTTCCGCTTCCATGACGGGTCTGCCGCACAA
CTCTGTATTCGGCCTGCCGCTGCCGGGTTTCGTTCACCTGACCTGTCCGCACTATT
GGCGTTACGGCGAAGAAGGTGAAACCGAAGAGCAGTTTGTTGCTCGTCTGGCCCG
CGAGCTGGAGGAAACTATCCAACGTGAAGGCGCGGACACGATTGCGGGCTTCTTT
GCTGAGCCGGTCATGGGCGCGGGCGGCGTAATCCCGCCGGCGAAAGGTTACTTC
CAGGCGATCCTGCCGATTCTGCGTAAGTACGACATCCCGGTTATCTCTGATGAAGT
TATCTGCGGCTTTGGTCGTACCGGTAATACTTGGGGTTGCGTTACCTATGACTTCA
CCCCGGATGCGATCATCTCCAGCAAAAATCTGACCGCCGGTTTCTTTCCGGTTGGT
GCTGTGATTCTGGGTCCGGAACTGAGCAAACGCCTGGAAACGGCGATCGAAGCTA
TCGAAGAGTTCCCGCACGGCTTTACGGCCGGCGGTCACCCGGTGGGTTGCGCTA
TCGCTCTGAAAGCAATCGATGTTGTGATGAATGAGGGTCTGGCAGAGAACGTGCG
CCGCCTGGCACCGCGTTTTGAGGAGCGTCTGAAACACATTGCCGAACGTCCGAAC
ATCGGTGAATATCGTGGCATCGGTTTTATGTGGGCACTGGAGGCTGTGAAAGACA

AAGCATCTAAAACCCCATTCGATGGTAATCTGTCTGTGAGCAAACGTATCGCTAAC
ACCTGTCAGGACCTGGGCCTGATCTGTAGCGCGCTGGGTCAGTCCGTTATCCTGA
GCCCGCCGTTCATCCTGACCGAGGCGCAAATGGATGAGATGTTTGACAAACTGGA
GAAGGCTCTGGACAAAGTCTTTGCGGAGGTGGCGTAA

**SEQ ID NO. 13**
Vfat 1010 DNA SEQUENCE

ATGAATAAACCACAAAGCTGGGAAGCGCGTGCTGAAACTTACTCTCTGTACGGCTT
CACTGATATGCCATCTCTGCACGAGCGTGGTACCGTGGTTGTCACCCACGGCGAG
GGCCCATACATCGTGGACGTCAACGGTCGCCGTTACCTGGACGCAAACTCCGGCC
TGTACAATATGGTTGCCGGCTTCGACCACAAGGGTCTGATCGACGCAGCAAAGGC
CCAGTACGAACGCTTCCCGGGTTACCATAGCTTCTTCGGTCGTATGTCTGATCAAA
CTGTTATGCTGAGCGAGAAACTGGTAGAGGTGTCTCCATTCGACAGCGGTCGCGT
GTTCTATACTAACTCCGGCTCCGAGGCTAACGATACTATGGTGAAAATGCTGTGGT
TTCTGCACGCCGCAGAGGGCAAGCCGCAAAAACGCAAAATCCTGACTCGTCAAAA
CGCATACCACGGTGTAACTGCTGTTTCCGCTTCCATGACGGGTATGCCGCACAAC
TCTGTATTCGGCCTGCCGCTGCCGGGTTTCGTTCACCTGACCTGTCCGCACTATTG
GCGTTACGGCGAAGAAGGTGAAACCGAAGAGCAGTTTGTTGCTCGTCTGGCCCGC
GAGCTGGAGGAAACTATCCAACGTGAAGGCGCGGACACGATTGCGGGCTTCTTTG
CTGAGCCGGTCATGGGCGCGGGCGGCGTAATCCCGCCGGCGAAAGGTTACTTCC
AGGCGATCCTGCCGATTCTGCGTAAGTACGACATCCCGGTTATCTCTGATGAAGTT
ATCTGCGGCTTTGGTCGTACCGGTAATACTTGGGGTTGCGTTACCTATGACTTCAC
CCCGGATGCGATCATCTCCAGCAAAAATCTGACCGCCGGTTTCTTTCCGGTTGGT
GCTGTGATTCTGGGTCCGGCACTGAGCAAACGCCTGGAAACGGCGATCGAAGCTA
TCGAAGAGTTCCCGCACGGCTTTACGGCCGGCGGTCACCCGGTGGGTTGCGCTA
TCGCTCTGAAAGCAATCGATGTTGTGATGAATGAGGGTCTGGCAGAGAACGTGCG
CCGCCTGGCACCGCGTTTTGAGGAGCGTCTGAAACACATTGCCGAACGTCCGAAC
ATCGGTGAATATCGTGGCATCGGTTTTATGTGGGCACTGGAGGCTGTGAAAGACA
AAGCATCTAAAACCCCATTCGATGGTAATCTGTCTGTGAGCAAACGTATCGCTAAC
ACCTGTCAGGACCTGGGCCTGATCTGTAGCGCGATGGGTCAGTCCGTTATCCTGA
GCCCGCCGTTCATCCTGACCGAGGCGCAAATGGATGAGATGTTTGACAAACTGGA
GAAGGCTCTGGACAAAGTCTTTGCGGAGGTGGCGTAA

**SEQ ID NO. 14**
Vfat 1020 DNA SEQUENCE

ATGAATAAACCACAAAGCTGGGAAGCGCGTGCTGAAACTTACTCTCTGTACGGCTT
CACTGATATGCCATCTCTGCACGAGCGTGGTACCGTGGTTGTCACCCACGGCGAG
GGCCCATACGTGGTGGACGTCAACGGTCGCCGTTACCTGGACGCAAACTCCGGC
CTGTACAATATGGTTGCCGGCTTCGACCACAAGGGTCTGATCGACGCAGCAAAGG
CCCAGTACGAACGCTTCCCGGGTTACCATAGCTTCTTCGGTCGTATGTCTGATCAA
ACTGTTATGCTGAGCGAGAAACTGGTAGAGGTGTCTCCATTCGACAGCGGTCGCG
TGTTCTATACTAACTCCGGCTCCGAGGCTAACGATACTATGGTGAAAATGCTGTGG
TTTCTGCACGCCGCAGAGGGCAAGCCGCAAAAACGCAAAATCCTGACTCGTCAAA
ACGCATACCACGGTGTAACTGCTGTTTCCGCTTCCATGACGGGTCTGCCGCACAA
CTCTGTATTCGGCCTGCCGCTGCCGGGTTTCGTTCACCTGGGTTGTCCGCACTATT
GGCGTTACGGCGAAGAAGGTGAAACCGAAGAGCAGTTTGTTGCTCGTCTGGCCCG
CGAGCTGGAGGAAACTATCCAACGTGAAGGCGCGGACACGATTGCGGGCTTCTTT
GCTGAGCCGGTCATGGGCGCGGGCGGCGTAATCCCGCCGGCGAAAGGTTACTTC
CAGGCGATCCTGCCGATTCTGCGTAAGTACGACATCCCGGTTATCTCTGATGAAGT
TATCTGCGGCTTTGGTCGTACCGGTAATACTTGGGGTTGCGTTACCTATGACTTCA
CCCCGGATGCGATCATCTCCAGCAAAAATCTGACCGCCGGTTTCTTTCCGGTTGGT
GCTGTGATTCTGGGTCCGGAACTGAGCAAACGCCTGGAAACGGCGATCGAAGCTA
TCGAAGAGTTCCCGCACGGCTTTACGGCCGGCGGTCACCCGGTGGGTTGCGCTA
TCGCTCTGAAAGCAATCGATGTTGTGATGAATGAGGGTCTGGCAGAGAACGTGCG
CCGCCTGGCACCGCGTTTTGAGGAGCGTCTGAAACACATTGCCGAACGTCCGAAC
ATCGGTGAATATCGTGGCATCGGTTTTATGTGGGCACTGGAGGCTGTGAAAGACA
AAGCATCTAAAACCCCATTCGATGGTAATCTGTCTGTGAGCAAACGTATCGCTAAC
ACCTGTCAGGACCTGGGCCTGATCTGTAGCGCGATGGGTCAGTCCGTTATCCTGA
GCCCGCCGTTCATCCTGACCGAGGCGCAAATGGATGAGATGTTTGACAAACTGGA
GAAGGCTCTGGACAAAGTCTTTGCGGAGGTGGCGTAA

**SEQ ID NO. 15**
Vfat 1030 DNA SEQUENCE

ATGAATAAACCACAAAGCTGGGAAGCGCGTGCTGAAACTTACTCTCTGTACGGCTT
CACTGATATGCCATCTCTGCACGAGCGTGGTACCGTGGTTGTCACCCACGGCGAG
GGCCCATACATCGTGGACGTCCACGGTCGCCGTTACCTGGACGCAAACTCCGGC
CTGTACAATATGGTTGCCGGCTTCGACCACAAGGGTCTGATCGACGCAGCAAAGG

CCCAGTACGAACGCTTCCCGGGTTACCATAGCTTCTTCGGTCGTATGTCTGATCAA
ACTGTTATGCTGAGCGAGAAACTGGTAGAGGTGTCTCCATTCGACAGCGGTCGCG
TGTTCTATACTAACTCCGGCTCCGAGGCTAACGATACTATGGTGAAAATGCTGTGG
TTTCTGCACGCCGCAGAGGGCAAGCCGCAAAAACGCAAAATCCTGACTCGTCAAA
ACGCATACCACGGTGTAACTGCTGTTTCCGCTTCCATGACGGGTCTGCCGCACAA
CTCTGTATTCGGCCTGCCGCTGCCGGGTTTCGTTCACCTGAGCTGTCCGCACTATT
GGCGTTACGGCGAAGAAGGTGAAACCGAAGAGCAGTTTGTTGCTCGTCTGGCCCG
CGAGCTGGAGGAAACTATCCAACGTGAAGGCGCGGACACGATTGCGGGCTTCTTT
GCTGAGCCGGTCATGGGCGCGGGCGGCGTAATCCCGCCGGCGAAAGGTTACTTC
CAGGCGATCCTGCCGATTCTGCGTAAGTACGACATCCCGGTTATCTCTGATGAAGT
TATCTGCGGCTTTGGTCGTACCGGTAATACTTGGGGTTGCGTTACCTATGACTTCA
CCCCGGATGCGATCATCTCCAGCAAAAATCTGACCGCCGGTTTCTTTCCGGTTGGT
GCTGTGATTCTGGGTCCGGAACTGAGCAAACGCCTGGAAACGGCGATCGAAGCTA
TCGAAGAGTTCCCGCACGGCTTTACGGCCGGCGGTCACCCGGTGGGTTGCGCTA
TCGCTCTGAAAGCAATCGATGTTGTGATGAATGAGGGTCTGGCAGAGAACGTGCG
CCGCCTGGCACCGCGTTTTGAGGAGCGTCTGAAACACATTGCCGAACGTCCGAAC
ATCGGTGAATATCGTGGCATCGGTTTTATGTGGGCACTGGAGGCTGTGAAAGACA
AAGCATCTAAAACCCCATTCGATGGTAATCTGTCTGTGAGCAAACGTATCGCTAAC
ACCTGTCAGGACCTGGGCCTGATCTGTAGCGCGATGGGTCAGTCCGTTATCCTGA
GCCCGCCGTTCATCCTGACCGAGGCGCAAATGGATGAGATGTTTGACAAACTGGA
GAAGGCTCTGGACAAAGTCTTTGCGGAGGTGGCGTAA

## Claims

1. A transaminase enzyme having an amino acid sequence that has at least 95% homology to the amino acid sequence

MNKPQSWEARAETYSLYGFTDMPSLH**X**$^{27}$RGTVVVTHGEGPY**X**$^{41}$VDV**X**$^{45}$ GRRYLDANSGLYNMVAGFDHKGLIDAAKAQYERFPGYHSFFGRMSDQT VMLSEKLVEVSPFDSGRVFYTNSGSEANDTMVKMLWFLHAAEGKPQKR KILTR**X**$^{147}$NAYHGVTAVSASMTG**X**$^{163}$P**X**$^{165}$NSVFGLPLPGFVHL**X**$^{180}$CPHY WRYGEEGETEEQFVARLARELEETIQREGADTIAGFFAEPVMGAGGVIP PAKGYFQAILPILRKYDIPVISDEVICGFGRTGNTWGCVTYDFTPDAIISSK NLTAGFFPVGAVILGPEL**X**$^{304}$KRLETAIEAIEEFPHGFTA**X**$^{324}$GHPVGCAIA LKAIDVVMNEGLAENVRRLAPRFEERLKHIAERPNIGEYRGIGFMWALEA VKDKASKTPFDGNLSVS**X**$^{401}$RIANTC**X**$^{408}$DLGLIC**X**$^{415}$**X**$^{416}$**X**$^{417}$GQSVIL**X**$^{424}$ PPFILTEAQMDEMFDKLEKALDKVFAEVA (SEQ ID NO. 1)

wherein

**X**$^{27}$ is selected from glutamine (Q) and glutamic acid (E);
**X**$^{41}$ is selected from isoleucine (I) and valine (V);
**X**$^{45}$ is selected from asparigine (N) and histidine (H);
**X**$^{147}$ is selected from asparigine (N) and glutamine (Q);
**X**$^{163}$ is selected from leucine (L) and methionine (M);
**X**$^{165}$ is selected from tyrosine (Y) and histidine (H);
**X**$^{180}$ is selected from threonine (T); glycine (G) and serine (S);
**X**$^{304}$ is selected from alanine (A) and serine (S);
**X**$^{324}$ is selected from glycine (G) and serine (S);
**X**$^{401}$ is selected from lysine (K) and glutamic acid (E);
**X**$^{408}$ is selected from threonine (T) and glutamine (Q);
**X**$^{415}$ is selected from serine (S) and alanine (A);
**X**$^{416}$ is selected from proline (P) and alanine (A);
**X**$^{417}$ is selected from leucine (L) and methionine (M); and
**X**$^{424}$ is selected from cysteine (C) and serine (S).

2. The transaminase enzyme according to claim 1 having the amino acid sequence of SEQ ID NO. 1.

3. The transaminase enzyme according to claim 1 or 2, wherein

**X**$^{27}$ is glutamic acid (E);
**X**$^{147}$ is glutamine (Q);
**X**$^{165}$ is histidine (H);
**X**$^{304}$ is serine (S);
**X**$^{324}$ is glycine (G);
**X**$^{401}$ is lysine (K);
**X**$^{408}$ is glutamine (Q);
**X**$^{416}$ is alanine (A);
**X**$^{417}$ is methionine (M); and
**X**$^{424}$ is serine (S).

**4.** The transaminase enzyme according to claim 2 having an amino acid sequence selected from:

MNKPQSWEARAETYSLYGFTDMPSLHQRGTVVVTHGEGPYIVDVN
GRRYLDANSGLYNMVAGFDHKGLIDAAKAQYERFPGYHSFFGRMS
DQTVMLSEKLVEVSPFDSGRVFYTNSGSEANDTMVKMLWFLHAAE
GKPQKRKILTRNNAYHGVTAVSASMTGLPYNSVFGLPLPGFVHLTC
PHYWRYGEEGETEEQFVARLARELEETIQREGADTIAGFFAEPVMG
AGGVIPPAKGYFQAILPILRKYDIPVISDEVICGFGRTGNTWGCVTYD
FTPDAIISSKNLTAGFFPVGAVILGPELAKRLETAIEAIEEFPHGFTAS
GHPVGCAIALKAIDVVMNEGLAENVRRLAPRFEERLKHIAERPNIGE
YRGIGFMWALEAVKDKASKTPFDGNLSVSERIANTCTDLGLICSPM
GQSVILCPPFILTEAQMDEMFDKLEKALDKVFAEVA (SEQ ID NO. 2);

MNKPQSWEARAETYSLYGFTDMPSLHERGTVVVTHGEGPYIVDVN
GRRYLDANSGLYNMVAGFDHKGLIDAAKAQYERFPGYHSFFGRMS
DQTVMLSEKLVEVSPFDSGRVFYTNSGSEANDTMVKMLWFLHAAE
GKPQKRKILTRNNAYHGVTAVSASMTGLPYNSVFGLPLPGFVHLTC
PHYWRYGEEGETEEQFVARLARELEETIQREGADTIAGFFAEPVMG
AGGVIPPAKGYFQAILPILRKYDIPVISDEVICGFGRTGNTWGCVTYD
FTPDAIISSKNLTAGFFPVGAVILGPELSKRLETAIEAIEEFPHGFTAG
GHPVGCAIALKAIDVVMNEGLAENVRRLAPRFEERLKHIAERPNIGE
YRGIGFMWALEAVKDKASKTPFDGNLSVSKRIANTCQDLGLICSAL
GQSVILCPPFILTEAQMDEMFDKLEKALDKVFAEVA (SEQ ID NO. 3);

MNKPQSWEARAETYSLYGFTDMPSLHERGTVVVTHGEGPYVVDV NGRRYLDANSGLYNMVAGFDHKGLIDAAKAQYERFPGYHSFFGRM SDQTVMLSEKLVEVSPFDSGRVFYTNSGSEANDTMVKMLWFLHAA EGKPQKRKILTRQNAYHGVTAVSASMTGLPHNSVFGLPLPGFVHLT CPHYWRYGEEGETEEQFVARLARELEETIQREGADTIAGFFAEPVM GAGGVIPPAKGYFQAILPILRKYDIPVISDEVICGFGRTGNTWGCVTY DFTPDAIISSKNLTAGFFPVGAVILGPELSKRLETAIEAIEEFPHGFTA GGHPVGCAIALKAIDVVMNEGLAENVRRLAPRFEERLKHIAERPNIG EYRGIGFMWALEAVKDKASKTPFDGNLSVSKRIANTCQDLGLICSAL GQSVILSPPFILTEAQMDEMFDKLEKALDKVFAEVA (SEQ ID NO. 4);

MNKPQSWEARAETYSLYGFTDMPSLHERGTVVVTHGEGPYIVDVN GRRYLDANSGLYNMVAGFDHKGLIDAAKAQYERFPGYHSFFGRMS DQTVMLSEKLVEVSPFDSGRVFYTNSGSEANDTMVKMLWFLHAAE GKPQKRKILTRQNAYHGVTAVSASMTGMPHNSVFGLPLPGFVHLT CPHYWRYGEEGETEEQFVARLARELEETIQREGADTIAGFFAEPVM GAGGVIPPAKGYFQAILPILRKYDIPVISDEVICGFGRTGNTWGCVTY DFTPDAIISSKNLTAGFFPVGAVILGPALSKRLETAIEAIEEFPHGFTA GGHPVGCAIALKAIDVVMNEGLAENVRRLAPRFEERLKHIAERPNIG EYRGIGFMWALEAVKDKASKTPFDGNLSVSKRIANTCQDLGLICSA MGQSVILSPPFILTEAQMDEMFDKLEKALDKVFAEVA (SEQ ID NO. 5);

MNKPQSWEARAETYSLYGFTDMPSLHERGTVVVTHGEGPYVVDV NGRRYLDANSGLYNMVAGFDHKGLIDAAKAQYERFPGYHSFFGRM

SDQTVMLSEKLVEVSPFDSGRVFYTNSGSEANDTMVKMLWFLHAA EGKPQKRKILTRQNAYHGVTAVSASMTGLPHNSVFGLPLPGFVHLG CPHYWRYGEEGETEEQFVARLARELEETIQREGADTIAGFFAEPVM GAGGVIPPAKGYFQAILPILRKYDIPVISDEVICGFGRTGNTWGCVTY DFTPDAIISSKNLTAGFFPVGAVILGPELSKRLETAIEAIEEFPHGFTA GGHPVGCAIALKAIDVVMNEGLAENVRRLAPRFEERLKHIAERPNIG EYRGIGFMWALEAVKDKASKTPFDGNLSVSKRIANTCQDLGLICAA MGQSVILSPPFILTEAQMDEMFDKLEKALDKVFAEVA(SEQ ID NO. 6); and

MNKPQSWEARAETYSLYGFTDMPSLHERGTVVVTHGEGPYIVDVH GRRYLDANSGLYNMVAGFDHKGLIDAAKAQYERFPGYHSFFGRMS DQTVMLSEKLVEVSPFDSGRVFYTNSGSEANDTMVKMLWFLHAAE GKPQKRKILTRQNAYHGVTAVSASMTGLPHNSVFGLPLPGFVHLSC PHYWRYGEEGETEEQFVARLARELEETIQREGADTIAGFFAEPVMG AGGVIPPAKGYFQAILPILRKYDIPVISDEVICGFGRTGNTWGCVTYD FTPDAIISSKNLTAGFFPVGAVILGPELSKRLETAIEAIEEFPHGFTAG GHPVGCAIALKAIDVVMNEGLAENVRRLAPRFEERLKHIAERPNIGE YRGIGFMWALEAVKDKASKTPFDGNLSVSKRIANTCQDLGLICSAM GQSVILSPPFILTEAQMDEMFDKLEKALDKVFAEVA (SEQ ID NO. 7).

**5.** A process for the manufacture of (S)-3-aminomethyl-5-methylhexanoic acid ((S)-**II**)

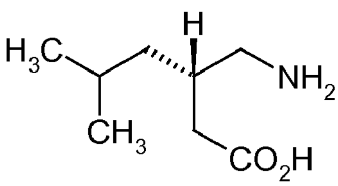

((*S*)-**II**)

or a pharmaceutically acceptable salt thereof, comprising the step of treating 5-hydroxy-4-(2-methylpropyl)-3,4-dihydro-5*H*-2-furanone (**I**[A]) and an amine with a transaminase enzyme according to any one of claims 1 to 4.

Europäisches Patentamt
European Patent Office
Office européen des brevets

# EUROPEAN SEARCH REPORT

Application Number
EP 17 16 2642

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2008/127646 A2 (BIOVERDANT INC [US]; NOTZ WOLFGANT REINHARD LUDWIG [US]; SCOTT ROBERT) 23 October 2008 (2008-10-23) * scheme 1, scheme 2; claims 1,11,12,13,14 * ----- | 1-5 | INV. C12N9/10 C07B53/00 C07C229/08 |
| X | WO 2010/081053 A2 (CODEXIS INC [US]; DHAWAN ISH KUMAR [US]; MILLER GREGORY [US]; ZHANG XI) 15 July 2010 (2010-07-15) * page 2, paragraph 0008; sequences 2, 14, 26 * ----- | 1-5 | |

TECHNICAL FIELDS SEARCHED (IPC)
C12N
C07B
C07C

The present search report has been drawn up for all claims

| Place of search | Date of completion of the search | Examiner |
|---|---|---|
| Munich | 1 June 2017 | Schuemacher, Anne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 17 16 2642

This annex lists the patent family members relating to the patent documents cited in the above-mentioned European search report. The members are as contained in the European Patent Office EDP file on The European Patent Office is in no way liable for these particulars which are merely given for the purpose of information.

01-06-2017

| Patent document cited in search report | Publication date | Patent family member(s) | Publication date |
|---|---|---|---|
| WO 2008127646 A2 | 23-10-2008 | NONE | |
| WO 2010081053 A2 | 15-07-2010 | CN 102341494 A | 01-02-2012 |
| | | EP 2385983 A2 | 16-11-2011 |
| | | IL 213950 A | 29-12-2016 |
| | | SG 172891 A1 | 29-08-2011 |
| | | US 2010209981 A1 | 19-08-2010 |
| | | US 2013266994 A1 | 10-10-2013 |
| | | US 2015210988 A1 | 30-07-2015 |
| | | US 2017073651 A1 | 16-03-2017 |
| | | WO 2010081053 A2 | 15-07-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5563175 A
- US 6001876 A
- US 2008004699 W
- WO 2008127646 A2
- IN 2010000140 W
- WO 2011086565 A
- GB 834100 A


### Non-patent literature cited in the description


- **KIENZLE, F. et al.** *Helv. Chim. Acta,* 1985, vol. 68 (5), 1133-39

SEQUENCE LISTING

<110> PFIZER IRELAND PHARMACEUTICALS
O'NEILL, PADRAIG MARY
DEBARGE, SEBASTIEN
ERDMAN, DAVID THOMAS
KUMAR, RAJESH
KARMILOWICZ, MICHAEL JOHN

<120> PROCESS

<130> PC71999A

<150> US 61/805786
<151> 2013-03-27

<160> 15

<170> PatentIn version 3.5

<210> 1
<211> 453
<212> PRT
<213> Artificial Sequence

<220>
<223> Modification of Vibrio fluvialis enzyme

<220>
<221> MISC_FEATURE
<222> (27)..(27)
<223> Xaa(27) is selected from glutamine (Gln) and glutamic acid (Glu)

<220>
<221> MISC_FEATURE
<222> (41)..(41)
<223> Xaa(41) is selected from isoleucine (Ile) and valine (Val)

<220>
<221> MISC_FEATURE
<222> (45)..(45)
<223> Xaa(45) is selected from asparigine (Asn) and histidine (His)

<220>
<221> MISC_FEATURE
<222> (147)..(147)
<223> Xaa(147) is selected from asparigine (Asn) and glutamine (Gln)

<220>
<221> MISC_FEATURE
<222> (163)..(163)
<223> Xaa(163) is selected from leucine (Leu) and methionine (Met)

<220>
<221> MISC_FEATURE
<222> (165)..(165)
<223> Xaa(165) is selected from tyrosine (Tyr) and histidine (His)

<220>
<221> MISC_FEATURE
<222> (180)..(180)
<223> Xaa(180) is selected from threonine (Thr); glycine (Gly) and

serine (Ser)

<220>
<221> MISC_FEATURE
<222> (304)..(304)
<223> Xaa(304) is selected from alanine (Ala) and serine (Ser)

<220>
<221> MISC_FEATURE
<222> (324)..(324)
<223> Xaa(324) is selected from glycine (Gly) and serine (Ser)

<220>
<221> MISC_FEATURE
<222> (401)..(401)
<223> Xaa(401) is selected from lysine (Lys) and glutamic acid (Glu)

<220>
<221> MISC_FEATURE
<222> (408)..(408)
<223> Xaa(408) is selected from threonine (Thr) and glutamine (Gln)

<220>
<221> MISC_FEATURE
<222> (415)..(415)
<223> Xaa(415) is selected from serine (Ser) and alanine (Ala)

<220>
<221> MISC_FEATURE
<222> (416)..(416)
<223> Xaa(416) is selected from proline (Pro) and alanine (Ala)

<220>
<221> MISC_FEATURE
<222> (417)..(417)
<223> Xaa(417) is selected from leucine (Leu) and methionine (Met)

<220>
<221> MISC_FEATURE
<222> (424)..(424)
<223> Xaa(424) is selected from cysteine (Cys) and serine (Ser)

<400> 1

```
Met Asn Lys Pro Gln Ser Trp Glu Ala Arg Ala Glu Thr Tyr Ser Leu
1               5                   10                  15

Tyr Gly Phe Thr Asp Met Pro Ser Leu His Xaa Arg Gly Thr Val Val
            20                  25                  30

Val Thr His Gly Glu Gly Pro Tyr Xaa Val Asp Val Xaa Gly Arg Arg
        35                  40                  45

Tyr Leu Asp Ala Asn Ser Gly Leu Tyr Asn Met Val Ala Gly Phe Asp
    50                  55                  60

His Lys Gly Leu Ile Asp Ala Ala Lys Ala Gln Tyr Glu Arg Phe Pro
65                  70                  75                  80
```

```
Gly Tyr His Ser Phe Phe Gly Arg Met Ser Asp Gln Thr Val Met Leu
                85                  90                  95

Ser Glu Lys Leu Val Glu Val Ser Pro Phe Asp Ser Gly Arg Val Phe
            100                 105                 110

Tyr Thr Asn Ser Gly Ser Glu Ala Asn Asp Thr Met Val Lys Met Leu
        115                 120                 125

Trp Phe Leu His Ala Ala Glu Gly Lys Pro Gln Lys Arg Lys Ile Leu
    130                 135                 140

Thr Arg Xaa Asn Ala Tyr His Gly Val Thr Ala Val Ser Ala Ser Met
145                 150                 155                 160

Thr Gly Xaa Pro Xaa Asn Ser Val Phe Gly Leu Pro Leu Pro Gly Phe
                165                 170                 175

Val His Leu Xaa Cys Pro His Tyr Trp Arg Tyr Gly Glu Glu Gly Glu
            180                 185                 190

Thr Glu Glu Gln Phe Val Ala Arg Leu Ala Arg Glu Leu Glu Glu Thr
        195                 200                 205

Ile Gln Arg Glu Gly Ala Asp Thr Ile Ala Gly Phe Phe Ala Glu Pro
    210                 215                 220

Val Met Gly Ala Gly Gly Val Ile Pro Pro Ala Lys Gly Tyr Phe Gln
225                 230                 235                 240

Ala Ile Leu Pro Ile Leu Arg Lys Tyr Asp Ile Pro Val Ile Ser Asp
                245                 250                 255

Glu Val Ile Cys Gly Phe Gly Arg Thr Gly Asn Thr Trp Gly Cys Val
            260                 265                 270

Thr Tyr Asp Phe Thr Pro Asp Ala Ile Ile Ser Ser Lys Asn Leu Thr
        275                 280                 285

Ala Gly Phe Phe Pro Val Gly Ala Val Ile Leu Gly Pro Glu Leu Xaa
    290                 295                 300

Lys Arg Leu Glu Thr Ala Ile Glu Ala Ile Glu Glu Phe Pro His Gly
305                 310                 315                 320

Phe Thr Ala Xaa Gly His Pro Val Gly Cys Ala Ile Ala Leu Lys Ala
                325                 330                 335
```

```
Ile Asp Val Val Met Asn Glu Gly Leu Ala Glu Asn Val Arg Arg Leu
            340                 345                 350

Ala Pro Arg Phe Glu Glu Arg Leu Lys His Ile Ala Glu Arg Pro Asn
        355                 360                 365

Ile Gly Glu Tyr Arg Gly Ile Gly Phe Met Trp Ala Leu Glu Ala Val
    370                 375                 380

Lys Asp Lys Ala Ser Lys Thr Pro Phe Asp Gly Asn Leu Ser Val Ser
385                 390                 395                 400

Xaa Arg Ile Ala Asn Thr Cys Xaa Asp Leu Gly Leu Ile Cys Xaa Xaa
                405                 410                 415

Xaa Gly Gln Ser Val Ile Leu Xaa Pro Pro Phe Ile Leu Thr Glu Ala
            420                 425                 430

Gln Met Asp Glu Met Phe Asp Lys Leu Glu Lys Ala Leu Asp Lys Val
        435                 440                 445

Phe Ala Glu Val Ala
    450


<210>  2
<211>  453
<212>  PRT
<213>  Artificial Sequence

<220>
<223>  Modification of Vibrio fluvialis enzyme

<400>  2

Met Asn Lys Pro Gln Ser Trp Glu Ala Arg Ala Glu Thr Tyr Ser Leu
1               5                   10                  15

Tyr Gly Phe Thr Asp Met Pro Ser Leu His Gln Arg Gly Thr Val Val
            20                  25                  30

Val Thr His Gly Glu Gly Pro Tyr Ile Val Asp Val Asn Gly Arg Arg
        35                  40                  45

Tyr Leu Asp Ala Asn Ser Gly Leu Tyr Asn Met Val Ala Gly Phe Asp
    50                  55                  60

His Lys Gly Leu Ile Asp Ala Ala Lys Ala Gln Tyr Glu Arg Phe Pro
65                  70                  75                  80
```

```
Gly Tyr His Ser Phe Phe Gly Arg Met Ser Asp Gln Thr Val Met Leu
                85                  90                  95

Ser Glu Lys Leu Val Glu Val Ser Pro Phe Asp Ser Gly Arg Val Phe
            100                 105                 110

Tyr Thr Asn Ser Gly Ser Glu Ala Asn Asp Thr Met Val Lys Met Leu
        115                 120                 125

Trp Phe Leu His Ala Ala Glu Gly Lys Pro Gln Lys Arg Lys Ile Leu
    130                 135                 140

Thr Arg Asn Asn Ala Tyr His Gly Val Thr Ala Val Ser Ala Ser Met
145                 150                 155                 160

Thr Gly Leu Pro Tyr Asn Ser Val Phe Gly Leu Pro Leu Pro Gly Phe
                165                 170                 175

Val His Leu Thr Cys Pro His Tyr Trp Arg Tyr Gly Glu Glu Gly Glu
            180                 185                 190

Thr Glu Glu Gln Phe Val Ala Arg Leu Ala Arg Glu Leu Glu Glu Thr
        195                 200                 205

Ile Gln Arg Glu Gly Ala Asp Thr Ile Ala Gly Phe Phe Ala Glu Pro
    210                 215                 220

Val Met Gly Ala Gly Gly Val Ile Pro Pro Ala Lys Gly Tyr Phe Gln
225                 230                 235                 240

Ala Ile Leu Pro Ile Leu Arg Lys Tyr Asp Ile Pro Val Ile Ser Asp
                245                 250                 255

Glu Val Ile Cys Gly Phe Gly Arg Thr Gly Asn Thr Trp Gly Cys Val
            260                 265                 270

Thr Tyr Asp Phe Thr Pro Asp Ala Ile Ile Ser Ser Lys Asn Leu Thr
        275                 280                 285

Ala Gly Phe Phe Pro Val Gly Ala Val Ile Leu Gly Pro Glu Leu Ala
    290                 295                 300

Lys Arg Leu Glu Thr Ala Ile Glu Ala Ile Glu Glu Phe Pro His Gly
305                 310                 315                 320

Phe Thr Ala Ser Gly His Pro Val Gly Cys Ala Ile Ala Leu Lys Ala
                325                 330                 335
```

```
Ile Asp Val Val Met Asn Glu Gly Leu Ala Glu Asn Val Arg Arg Leu
            340                 345                 350

Ala Pro Arg Phe Glu Glu Arg Leu Lys His Ile Ala Glu Arg Pro Asn
        355                 360                 365

Ile Gly Glu Tyr Arg Gly Ile Gly Phe Met Trp Ala Leu Glu Ala Val
    370                 375                 380

Lys Asp Lys Ala Ser Lys Thr Pro Phe Asp Gly Asn Leu Ser Val Ser
385                 390                 395                 400

Glu Arg Ile Ala Asn Thr Cys Thr Asp Leu Gly Leu Ile Cys Ser Pro
                405                 410                 415

Met Gly Gln Ser Val Ile Leu Cys Pro Pro Phe Ile Leu Thr Glu Ala
            420                 425                 430

Gln Met Asp Glu Met Phe Asp Lys Leu Glu Lys Ala Leu Asp Lys Val
        435                 440                 445

Phe Ala Glu Val Ala
    450
```

<210> 3
<211> 453
<212> PRT
<213> Artificial Sequence

<220>
<223> Modification of Vibrio fluvialis enzyme

<400> 3

```
Met Asn Lys Pro Gln Ser Trp Glu Ala Arg Ala Glu Thr Tyr Ser Leu
1               5                   10                  15

Tyr Gly Phe Thr Asp Met Pro Ser Leu His Glu Arg Gly Thr Val Val
            20                  25                  30

Val Thr His Gly Glu Gly Pro Tyr Ile Val Asp Val Asn Gly Arg Arg
        35                  40                  45

Tyr Leu Asp Ala Asn Ser Gly Leu Tyr Asn Met Val Ala Gly Phe Asp
    50                  55                  60

His Lys Gly Leu Ile Asp Ala Ala Lys Ala Gln Tyr Glu Arg Phe Pro
65                  70                  75                  80
```

```
Gly Tyr His Ser Phe Phe Gly Arg Met Ser Asp Gln Thr Val Met Leu
                85                  90                  95

Ser Glu Lys Leu Val Glu Val Ser Pro Phe Asp Ser Gly Arg Val Phe
            100                 105                 110

Tyr Thr Asn Ser Gly Ser Glu Ala Asn Asp Thr Met Val Lys Met Leu
        115                 120                 125

Trp Phe Leu His Ala Ala Glu Gly Lys Pro Gln Lys Arg Lys Ile Leu
    130                 135                 140

Thr Arg Asn Asn Ala Tyr His Gly Val Thr Ala Val Ser Ala Ser Met
145                 150                 155                 160

Thr Gly Leu Pro Tyr Asn Ser Val Phe Gly Leu Pro Leu Pro Gly Phe
                165                 170                 175

Val His Leu Thr Cys Pro His Tyr Trp Arg Tyr Gly Glu Glu Gly Glu
            180                 185                 190

Thr Glu Glu Gln Phe Val Ala Arg Leu Ala Arg Glu Leu Glu Glu Thr
        195                 200                 205

Ile Gln Arg Glu Gly Ala Asp Thr Ile Ala Gly Phe Phe Ala Glu Pro
    210                 215                 220

Val Met Gly Ala Gly Gly Val Ile Pro Pro Ala Lys Gly Tyr Phe Gln
225                 230                 235                 240

Ala Ile Leu Pro Ile Leu Arg Lys Tyr Asp Ile Pro Val Ile Ser Asp
                245                 250                 255

Glu Val Ile Cys Gly Phe Gly Arg Thr Gly Asn Thr Trp Gly Cys Val
            260                 265                 270

Thr Tyr Asp Phe Thr Pro Asp Ala Ile Ile Ser Ser Lys Asn Leu Thr
        275                 280                 285

Ala Gly Phe Phe Pro Val Gly Ala Val Ile Leu Gly Pro Glu Leu Ser
    290                 295                 300

Lys Arg Leu Glu Thr Ala Ile Glu Ala Ile Glu Glu Phe Pro His Gly
305                 310                 315                 320

Phe Thr Ala Gly Gly His Pro Val Gly Cys Ala Ile Ala Leu Lys Ala
                325                 330                 335
```

Ile Asp Val Val Met Asn Glu Gly Leu Ala Glu Asn Val Arg Arg Leu
340 345 350

Ala Pro Arg Phe Glu Glu Arg Leu Lys His Ile Ala Glu Arg Pro Asn
355 360 365

Ile Gly Glu Tyr Arg Gly Ile Gly Phe Met Trp Ala Leu Glu Ala Val
370 375 380

Lys Asp Lys Ala Ser Lys Thr Pro Phe Asp Gly Asn Leu Ser Val Ser
385 390 395 400

Lys Arg Ile Ala Asn Thr Cys Gln Asp Leu Gly Leu Ile Cys Ser Ala
405 410 415

Leu Gly Gln Ser Val Ile Leu Cys Pro Pro Phe Ile Leu Thr Glu Ala
420 425 430

Gln Met Asp Glu Met Phe Asp Lys Leu Glu Lys Ala Leu Asp Lys Val
435 440 445

Phe Ala Glu Val Ala
450

<210> 4
<211> 453
<212> PRT
<213> Artificial Sequence

<220>
<223> Modification of Vibrio fluvialis enzyme

<400> 4

Met Asn Lys Pro Gln Ser Trp Glu Ala Arg Ala Glu Thr Tyr Ser Leu
1 5 10 15

Tyr Gly Phe Thr Asp Met Pro Ser Leu His Glu Arg Gly Thr Val Val
20 25 30

Val Thr His Gly Glu Gly Pro Tyr Val Val Asp Val Asn Gly Arg Arg
35 40 45

Tyr Leu Asp Ala Asn Ser Gly Leu Tyr Asn Met Val Ala Gly Phe Asp
50 55 60

His Lys Gly Leu Ile Asp Ala Ala Lys Ala Gln Tyr Glu Arg Phe Pro
65 70 75 80

```
Gly Tyr His Ser Phe Phe Gly Arg Met Ser Asp Gln Thr Val Met Leu
                85                  90                  95

Ser Glu Lys Leu Val Glu Val Ser Pro Phe Asp Ser Gly Arg Val Phe
            100                 105                 110

Tyr Thr Asn Ser Gly Ser Glu Ala Asn Asp Thr Met Val Lys Met Leu
        115                 120                 125

Trp Phe Leu His Ala Ala Glu Gly Lys Pro Gln Lys Arg Lys Ile Leu
    130                 135                 140

Thr Arg Gln Asn Ala Tyr His Gly Val Thr Ala Val Ser Ala Ser Met
145                 150                 155                 160

Thr Gly Leu Pro His Asn Ser Val Phe Gly Leu Pro Leu Pro Gly Phe
                165                 170                 175

Val His Leu Thr Cys Pro His Tyr Trp Arg Tyr Gly Glu Glu Gly Glu
            180                 185                 190

Thr Glu Glu Gln Phe Val Ala Arg Leu Ala Arg Glu Leu Glu Glu Thr
        195                 200                 205

Ile Gln Arg Glu Gly Ala Asp Thr Ile Ala Gly Phe Phe Ala Glu Pro
    210                 215                 220

Val Met Gly Ala Gly Gly Val Ile Pro Pro Ala Lys Gly Tyr Phe Gln
225                 230                 235                 240

Ala Ile Leu Pro Ile Leu Arg Lys Tyr Asp Ile Pro Val Ile Ser Asp
                245                 250                 255

Glu Val Ile Cys Gly Phe Gly Arg Thr Gly Asn Thr Trp Gly Cys Val
            260                 265                 270

Thr Tyr Asp Phe Thr Pro Asp Ala Ile Ile Ser Ser Lys Asn Leu Thr
        275                 280                 285

Ala Gly Phe Phe Pro Val Gly Ala Val Ile Leu Gly Pro Glu Leu Ser
    290                 295                 300

Lys Arg Leu Glu Thr Ala Ile Glu Ala Ile Glu Glu Phe Pro His Gly
305                 310                 315                 320

Phe Thr Ala Gly Gly His Pro Val Gly Cys Ala Ile Ala Leu Lys Ala
                325                 330                 335
```

Ile Asp Val Val Met Asn Glu Gly Leu Ala Glu Asn Val Arg Arg Leu
340 345 350

Ala Pro Arg Phe Glu Glu Arg Leu Lys His Ile Ala Glu Arg Pro Asn
355 360 365

Ile Gly Glu Tyr Arg Gly Ile Gly Phe Met Trp Ala Leu Glu Ala Val
370 375 380

Lys Asp Lys Ala Ser Lys Thr Pro Phe Asp Gly Asn Leu Ser Val Ser
385 390 395 400

Lys Arg Ile Ala Asn Thr Cys Gln Asp Leu Gly Leu Ile Cys Ser Ala
405 410 415

Leu Gly Gln Ser Val Ile Leu Ser Pro Pro Phe Ile Leu Thr Glu Ala
420 425 430

Gln Met Asp Glu Met Phe Asp Lys Leu Glu Lys Ala Leu Asp Lys Val
435 440 445

Phe Ala Glu Val Ala
450

<210> 5
<211> 453
<212> PRT
<213> Artificial Sequence

<220>
<223> Modification of Vibrio fluvialis enzyme

<400> 5

Met Asn Lys Pro Gln Ser Trp Glu Ala Arg Ala Glu Thr Tyr Ser Leu
1 5 10 15

Tyr Gly Phe Thr Asp Met Pro Ser Leu His Glu Arg Gly Thr Val Val
20 25 30

Val Thr His Gly Glu Gly Pro Tyr Ile Val Asp Val Asn Gly Arg Arg
35 40 45

Tyr Leu Asp Ala Asn Ser Gly Leu Tyr Asn Met Val Ala Gly Phe Asp
50 55 60

His Lys Gly Leu Ile Asp Ala Ala Lys Ala Gln Tyr Glu Arg Phe Pro
65 70 75 80

```
Gly Tyr His Ser Phe Phe Gly Arg Met Ser Asp Gln Thr Val Met Leu
                85                  90                  95

Ser Glu Lys Leu Val Glu Val Ser Pro Phe Asp Ser Gly Arg Val Phe
            100                 105                 110

Tyr Thr Asn Ser Gly Ser Glu Ala Asn Asp Thr Met Val Lys Met Leu
        115                 120                 125

Trp Phe Leu His Ala Ala Glu Gly Lys Pro Gln Lys Arg Lys Ile Leu
    130                 135                 140

Thr Arg Gln Asn Ala Tyr His Gly Val Thr Ala Val Ser Ala Ser Met
145                 150                 155                 160

Thr Gly Met Pro His Asn Ser Val Phe Gly Leu Pro Leu Pro Gly Phe
                165                 170                 175

Val His Leu Thr Cys Pro His Tyr Trp Arg Tyr Gly Glu Glu Gly Glu
            180                 185                 190

Thr Glu Glu Gln Phe Val Ala Arg Leu Ala Arg Glu Leu Glu Glu Thr
        195                 200                 205

Ile Gln Arg Glu Gly Ala Asp Thr Ile Ala Gly Phe Phe Ala Glu Pro
    210                 215                 220

Val Met Gly Ala Gly Gly Val Ile Pro Pro Ala Lys Gly Tyr Phe Gln
225                 230                 235                 240

Ala Ile Leu Pro Ile Leu Arg Lys Tyr Asp Ile Pro Val Ile Ser Asp
                245                 250                 255

Glu Val Ile Cys Gly Phe Gly Arg Thr Gly Asn Thr Trp Gly Cys Val
            260                 265                 270

Thr Tyr Asp Phe Thr Pro Asp Ala Ile Ile Ser Ser Lys Asn Leu Thr
        275                 280                 285

Ala Gly Phe Phe Pro Val Gly Ala Val Ile Leu Gly Pro Ala Leu Ser
    290                 295                 300

Lys Arg Leu Glu Thr Ala Ile Glu Ala Ile Glu Glu Phe Pro His Gly
305                 310                 315                 320

Phe Thr Ala Gly Gly His Pro Val Gly Cys Ala Ile Ala Leu Lys Ala
                325                 330                 335
```

Ile Asp Val Val Met Asn Glu Gly Leu Ala Glu Asn Val Arg Arg Leu
340 345 350

Ala Pro Arg Phe Glu Glu Arg Leu Lys His Ile Ala Glu Arg Pro Asn
355 360 365

Ile Gly Glu Tyr Arg Gly Ile Gly Phe Met Trp Ala Leu Glu Ala Val
370 375 380

Lys Asp Lys Ala Ser Lys Thr Pro Phe Asp Gly Asn Leu Ser Val Ser
385 390 395 400

Lys Arg Ile Ala Asn Thr Cys Gln Asp Leu Gly Leu Ile Cys Ser Ala
405 410 415

Met Gly Gln Ser Val Ile Leu Ser Pro Pro Phe Ile Leu Thr Glu Ala
420 425 430

Gln Met Asp Glu Met Phe Asp Lys Leu Glu Lys Ala Leu Asp Lys Val
435 440 445

Phe Ala Glu Val Ala
450

<210> 6
<211> 453
<212> PRT
<213> Artificial Sequence

<220>
<223> Modification of Vibrio fluvialis enzyme

<400> 6

Met Asn Lys Pro Gln Ser Trp Glu Ala Arg Ala Glu Thr Tyr Ser Leu
1 5 10 15

Tyr Gly Phe Thr Asp Met Pro Ser Leu His Glu Arg Gly Thr Val Val
20 25 30

Val Thr His Gly Glu Gly Pro Tyr Val Val Asp Val Asn Gly Arg Arg
35 40 45

Tyr Leu Asp Ala Asn Ser Gly Leu Tyr Asn Met Val Ala Gly Phe Asp
50 55 60

His Lys Gly Leu Ile Asp Ala Ala Lys Ala Gln Tyr Glu Arg Phe Pro
65 70 75 80

```
Gly Tyr His Ser Phe Phe Gly Arg Met Ser Asp Gln Thr Val Met Leu
                85                  90                  95

Ser Glu Lys Leu Val Glu Val Ser Pro Phe Asp Ser Gly Arg Val Phe
            100                 105                 110

Tyr Thr Asn Ser Gly Ser Glu Ala Asn Asp Thr Met Val Lys Met Leu
        115                 120                 125

Trp Phe Leu His Ala Ala Glu Gly Lys Pro Gln Lys Arg Lys Ile Leu
    130                 135                 140

Thr Arg Gln Asn Ala Tyr His Gly Val Thr Ala Val Ser Ala Ser Met
145                 150                 155                 160

Thr Gly Leu Pro His Asn Ser Val Phe Gly Leu Pro Leu Pro Gly Phe
                165                 170                 175

Val His Leu Gly Cys Pro His Tyr Trp Arg Tyr Gly Glu Glu Gly Glu
            180                 185                 190

Thr Glu Glu Gln Phe Val Ala Arg Leu Ala Arg Glu Leu Glu Glu Thr
        195                 200                 205

Ile Gln Arg Glu Gly Ala Asp Thr Ile Ala Gly Phe Phe Ala Glu Pro
    210                 215                 220

Val Met Gly Ala Gly Gly Val Ile Pro Pro Ala Lys Gly Tyr Phe Gln
225                 230                 235                 240

Ala Ile Leu Pro Ile Leu Arg Lys Tyr Asp Ile Pro Val Ile Ser Asp
                245                 250                 255

Glu Val Ile Cys Gly Phe Gly Arg Thr Gly Asn Thr Trp Gly Cys Val
            260                 265                 270

Thr Tyr Asp Phe Thr Pro Asp Ala Ile Ile Ser Ser Lys Asn Leu Thr
        275                 280                 285

Ala Gly Phe Phe Pro Val Gly Ala Val Ile Leu Gly Pro Glu Leu Ser
    290                 295                 300

Lys Arg Leu Glu Thr Ala Ile Glu Ala Ile Glu Glu Phe Pro His Gly
305                 310                 315                 320

Phe Thr Ala Gly Gly His Pro Val Gly Cys Ala Ile Ala Leu Lys Ala
                325                 330                 335
```

```
Ile Asp Val Val Met Asn Glu Gly Leu Ala Glu Asn Val Arg Arg Leu
            340                 345                 350

Ala Pro Arg Phe Glu Glu Arg Leu Lys His Ile Ala Glu Arg Pro Asn
        355                 360                 365

Ile Gly Glu Tyr Arg Gly Ile Gly Phe Met Trp Ala Leu Glu Ala Val
    370                 375                 380

Lys Asp Lys Ala Ser Lys Thr Pro Phe Asp Gly Asn Leu Ser Val Ser
385                 390                 395                 400

Lys Arg Ile Ala Asn Thr Cys Gln Asp Leu Gly Leu Ile Cys Ala Ala
                405                 410                 415

Met Gly Gln Ser Val Ile Leu Ser Pro Pro Phe Ile Leu Thr Glu Ala
            420                 425                 430

Gln Met Asp Glu Met Phe Asp Lys Leu Glu Lys Ala Leu Asp Lys Val
        435                 440                 445

Phe Ala Glu Val Ala
    450


<210>  7
<211>  453
<212>  PRT
<213>  Artificial Sequence

<220>
<223>  Modification of Vibrio fluvialis enzyme

<400>  7

Met Asn Lys Pro Gln Ser Trp Glu Ala Arg Ala Glu Thr Tyr Ser Leu
1               5                   10                  15

Tyr Gly Phe Thr Asp Met Pro Ser Leu His Glu Arg Gly Thr Val Val
            20                  25                  30

Val Thr His Gly Glu Gly Pro Tyr Ile Val Asp Val His Gly Arg Arg
        35                  40                  45

Tyr Leu Asp Ala Asn Ser Gly Leu Tyr Asn Met Val Ala Gly Phe Asp
    50                  55                  60

His Lys Gly Leu Ile Asp Ala Ala Lys Ala Gln Tyr Glu Arg Phe Pro
65                  70                  75                  80
```

```
Gly Tyr His Ser Phe Phe Gly Arg Met Ser Asp Gln Thr Val Met Leu
                85                  90                  95

Ser Glu Lys Leu Val Glu Val Ser Pro Phe Asp Ser Gly Arg Val Phe
            100                 105                 110

Tyr Thr Asn Ser Gly Ser Glu Ala Asn Asp Thr Met Val Lys Met Leu
        115                 120                 125

Trp Phe Leu His Ala Ala Glu Gly Lys Pro Gln Lys Arg Lys Ile Leu
    130                 135                 140

Thr Arg Gln Asn Ala Tyr His Gly Val Thr Ala Val Ser Ala Ser Met
145                 150                 155                 160

Thr Gly Leu Pro His Asn Ser Val Phe Gly Leu Pro Leu Pro Gly Phe
                165                 170                 175

Val His Leu Ser Cys Pro His Tyr Trp Arg Tyr Gly Glu Glu Gly Glu
            180                 185                 190

Thr Glu Glu Gln Phe Val Ala Arg Leu Ala Arg Glu Leu Glu Glu Thr
        195                 200                 205

Ile Gln Arg Glu Gly Ala Asp Thr Ile Ala Gly Phe Phe Ala Glu Pro
    210                 215                 220

Val Met Gly Ala Gly Gly Val Ile Pro Pro Ala Lys Gly Tyr Phe Gln
225                 230                 235                 240

Ala Ile Leu Pro Ile Leu Arg Lys Tyr Asp Ile Pro Val Ile Ser Asp
                245                 250                 255

Glu Val Ile Cys Gly Phe Gly Arg Thr Gly Asn Thr Trp Gly Cys Val
            260                 265                 270

Thr Tyr Asp Phe Thr Pro Asp Ala Ile Ile Ser Ser Lys Asn Leu Thr
        275                 280                 285

Ala Gly Phe Phe Pro Val Gly Ala Val Ile Leu Gly Pro Glu Leu Ser
    290                 295                 300

Lys Arg Leu Glu Thr Ala Ile Glu Ala Ile Glu Glu Phe Pro His Gly
305                 310                 315                 320

Phe Thr Ala Gly Gly His Pro Val Gly Cys Ala Ile Ala Leu Lys Ala
                325                 330                 335
```

Ile Asp Val Val Met Asn Glu Gly Leu Ala Glu Asn Val Arg Arg Leu
340 345 350

Ala Pro Arg Phe Glu Glu Arg Leu Lys His Ile Ala Glu Arg Pro Asn
355 360 365

Ile Gly Glu Tyr Arg Gly Ile Gly Phe Met Trp Ala Leu Glu Ala Val
370 375 380

Lys Asp Lys Ala Ser Lys Thr Pro Phe Asp Gly Asn Leu Ser Val Ser
385 390 395 400

Lys Arg Ile Ala Asn Thr Cys Gln Asp Leu Gly Leu Ile Cys Ser Ala
405 410 415

Met Gly Gln Ser Val Ile Leu Ser Pro Pro Phe Ile Leu Thr Glu Ala
420 425 430

Gln Met Asp Glu Met Phe Asp Lys Leu Glu Lys Ala Leu Asp Lys Val
435 440 445

Phe Ala Glu Val Ala
450

<210> 8
<211> 376
<212> PRT
<213> Lycopersicon esculentum

<400> 8

Met Glu Asn Lys Val Val Glu Glu Lys Gln Val Asp Lys Ile Pro Leu
1 5 10 15

Met Ser Pro Cys Lys Met Gly Lys Phe Glu Leu Cys His Arg Val Val
20 25 30

Leu Ala Pro Leu Thr Arg Gln Arg Ser Tyr Gly Tyr Ile Pro Gln Pro
35 40 45

His Ala Ile Leu His Tyr Ser Gln Arg Ser Thr Asn Gly Gly Leu Leu
50 55 60

Ile Gly Glu Ala Thr Val Ile Ser Glu Thr Gly Ile Gly Tyr Lys Asp
65 70 75 80

Val Pro Gly Ile Trp Thr Lys Glu Gln Val Glu Ala Trp Lys Pro Ile
85 90 95

Val Asp Ala Val His Ala Lys Gly Gly Ile Phe Phe Cys Gln Ile Trp
100 105 110

His Val Gly Arg Val Ser Asn Lys Asp Phe Gln Pro Asn Gly Glu Asp
115 120 125

Pro Ile Ser Cys Thr Asp Arg Gly Leu Thr Pro Gln Ile Arg Ser Asn
130 135 140

Gly Ile Asp Ile Ala His Phe Thr Arg Pro Arg Arg Leu Thr Thr Asp
145 150 155 160

Glu Ile Pro Gln Ile Val Asn Glu Phe Arg Val Ala Ala Arg Asn Ala
165 170 175

Ile Glu Ala Gly Phe Asp Gly Val Glu Ile His Gly Ala His Gly Tyr
180 185 190

Leu Ile Asp Gln Phe Met Lys Asp Gln Val Asn Asp Arg Ser Asp Lys
195 200 205

Tyr Gly Gly Ser Leu Glu Asn Arg Cys Arg Phe Ala Leu Glu Ile Val
210 215 220

Glu Ala Val Ala Asn Glu Ile Gly Ser Asp Arg Val Gly Ile Arg Ile
225 230 235 240

Ser Pro Phe Ala His Tyr Asn Glu Ala Gly Asp Thr Asn Pro Thr Ala
245 250 255

Leu Gly Leu Tyr Met Val Glu Ser Leu Asn Lys Tyr Asp Leu Ala Tyr
260 265 270

Cys His Val Val Glu Pro Arg Met Lys Thr Ala Trp Glu Lys Ile Glu
275 280 285

Cys Thr Glu Ser Leu Val Pro Met Arg Lys Ala Tyr Lys Gly Thr Phe
290 295 300

Ile Val Ala Gly Gly Tyr Asp Arg Glu Asp Gly Asn Arg Ala Leu Ile
305 310 315 320

Glu Asp Arg Ala Asp Leu Val Ala Tyr Gly Arg Leu Phe Ile Ser Asn
325 330 335

Pro Asp Leu Pro Lys Arg Phe Glu Leu Asn Ala Pro Leu Asn Lys Tyr

340 345 350

Asn Arg Asp Thr Phe Tyr Thr Ser Asp Pro Ile Val Gly Tyr Thr Asp
355 360 365

Tyr Pro Phe Leu Glu Thr Met Thr
370 375

<210> 9
<211> 1134
<212> DNA
<213> Lycopersicon esculentum

<400> 9
atggaaaaca aagttgtgga agaaaaacag gttgataaaa tcccgctgat gagcccgtgt 60

aaaatgggta aattcgagct gtgtcatcgc gttgtactgg caccgctgac tcgtcagcgt 120

tcttatggtt acattccgca gccgcacgca atcctgcatt actctcagcg cagcaccaac 180

ggtggcctgc tgatcggtga agcaaccgtg atcagcgaaa ctggcatcgg ttacaaagat 240

gtgccgggta tctggacgaa agagcaggtt gaggcctgga aaccgatcgt cgacgcggtg 300

catgccaaag gtggtatttt cttttgtcag atctggcacg ttggtcgtgt atccaacaaa 360

gattttcagc cgaacggcga agatccgatt tcctgtactg accgcggcct gaccccgcag 420

atccgttcca acggcattga cattgcccac ttcacccgtc cacgtcgcct gactactgac 480

gagattccgc agatcgtgaa cgagttccgc gttgcagcgc gtaatgctat tgaagcgggt 540

ttcgatggcg tcgagattca tggtgcccac ggttacctga tcgaccaatt catgaaagac 600

caagttaacg accgcagcga taagtatggc ggttctctgg agaaccgttg tcgcttcgcg 660

ctggaaatcg ttgaagcagt agccaacgag attggctccg accgtgttgg tatccgtatc 720

tctccattcg cacactacaa cgaagcgggc gacactaacc cgaccgcact gggcctgtat 780

atggtggaga gcctgaataa atacgacctg gcgtattgtc acgtggtcga gccgcgcatg 840

aaaaccgcct gggaaaagat tgagtgcacc gaaagcctgg tgccgatgcg taaagcctac 900

aaaggcacct tcatcgtagc tggtggctac gaccgtgaag acggtaaccg cgctctgatc 960

gaagaccgtg ccgacctggt tgcgtacggt cgtctgttca tcagcaaccc agacctgccg 1020

aagcgttttg aactgaacgc tccgctgaac aaatacaacc gtgacacttt ctacacttcc 1080

gacccgatcg ttggttacac cgattacccg tttctggaaa ctatgactta ataa 1134

<210> 10
<211> 1362
<212> DNA
<213> Artificial Sequence

<220>
<223> Modification of Vibrio fluvialis enzyme

<400> 10
atgaataaac cacaaagctg ggaagcgcgt gctgaaactt actctctgta cggcttcact 60

gatatgccat ctctgcacca gcgtggtacc gtggttgtca cccacggcga gggcccatac 120

atcgtggacg tcaacggtcg ccgttacctg gacgcaaact ccggcctgta caatatggtt 180

gccggcttcg accacaaggg tctgatcgac gcagcaaagg cccagtacga acgcttcccg 240

ggttaccata gcttcttcgg tcgtatgtct gatcaaactg ttatgctgag cgagaaactg 300

gtagaggtgt ctccattcga cagcggtcgc gtgttctata ctaactccgg ctccgaggct 360

aacgatacta tggtgaaaat gctgtggttt ctgcacgccg cagagggcaa gccgcaaaaa 420

cgcaaaatcc tgactcgtaa caacgcatac cacggtgtaa ctgctgtttc cgcttccatg 480

acgggtctgc cgtacaactc tgtattcggc ctgccgctgc cgggtttcgt tcacctgacc 540

tgtccgcact attggcgtta cggcgaagaa ggtgaaaccg aagagcagtt tgttgctcgt 600

ctggcccgcg agctggagga aactatccaa cgtgaaggcg cggacacgat tgcgggcttc 660

tttgctgagc cggtcatggg cgcgggcggc gtaatcccgc cggcgaaagg ttacttccag 720

gcgatcctgc cgattctgcg taagtacgac atcccggtta tctctgatga agttatctgc 780

ggctttggtc gtaccggtaa tacttggggt tgcgttacct atgacttcac cccggatgcg 840

atcatctcca gcaaaaatct gaccgccggt ttctttccgg ttggtgctgt gattctgggt 900

ccggaactgg cgaaacgcct ggaaacggcg atcgaagcta tcgaagagtt cccgcacggc 960

tttacggcca gcggtcaccc ggtgggttgc gctatcgctc tgaaagcaat cgatgttgtg 1020

atgaatgagg gtctggcaga gaacgtgcgc cgcctggcac cgcgttttga ggagcgtctg 1080

aaacacattg ccgaacgtcc gaacatcggt gaatatcgtg gcatcggttt tatgtgggca 1140

ctggaggctg tgaaagacaa agcatctaaa accccattcg atggtaatct gtctgtgagc 1200

gagcgtatcg ctaacacctg taccgacctg ggcctgatct gtagcccgat gggtcagtcc 1260

gttatcctgt gcccgccgtt catcctgacc gaggcgcaaa tggatgagat gtttgacaaa 1320

ctggagaagg ctctggacaa agtctttgcg gaggtggcgt aa 1362

<210> 11
<211> 1362
<212> DNA
<213> Artificial Sequence

<220>
<223> Modification of Vibrio fluvialis enzyme

<400> 11
atgaataaac cacaaagctg ggaagcgcgt gctgaaactt actctctgta cggcttcact 60

gatatgccat ctctgcacga gcgtggtacc gtggttgtca cccacggcga gggcccatac 120

atcgtggacg tcaacggtcg ccgttacctg gacgcaaact ccggcctgta caatatggtt 180

gccggcttcg accacaaggg tctgatcgac gcagcaaagg cccagtacga acgcttcccg 240

ggttaccata gcttcttcgg tcgtatgtct gatcaaactg ttatgctgag cgagaaactg 300

gtagaggtgt ctccattcga cagcggtcgc gtgttctata ctaactccgg ctccgaggct 360

aacgatacta tggtgaaaat gctgtggttt ctgcacgccg cagagggcaa gccgcaaaaa 420

cgcaaaatcc tgactcgtaa caacgcatac cacggtgtaa ctgctgtttc cgcttccatg 480

acgggtctgc cgtacaactc tgtattcggc ctgccgctgc cgggtttcgt tcacctgacc 540

tgtccgcact attggcgtta cggcgaagaa ggtgaaaccg aagagcagtt tgttgctcgt 600

ctggcccgcg agctggagga aactatccaa cgtgaaggcg cggacacgat tgcgggcttc 660

tttgctgagc cggtcatggg cgcgggcggc gtaatcccgc cggcgaaagg ttacttccag 720

gcgatcctgc cgattctgcg taagtacgac atcccggtta tctctgatga agttatctgc 780

ggctttggtc gtaccggtaa tacttggggt tgcgttacct atgacttcac cccggatgcg 840

atcatctcca gcaaaaatct gaccgccggt ttctttccgg ttggtgctgt gattctgggt 900

ccggaactga gcaaacgcct ggaaacggcg atcgaagcta tcgaagagtt cccgcacggc 960

tttacggccg gcggtcaccc ggtgggttgc gctatcgctc tgaaagcaat cgatgttgtg 1020

atgaatgagg gtctggcaga gaacgtgcgc cgcctggcac cgcgttttga ggagcgtctg 1080

aaacacattg ccgaacgtcc gaacatcggt gaatatcgtg gcatcggttt tatgtgggca 1140

ctggaggctg tgaaagacaa agcatctaaa accccattcg atggtaatct gtctgtgagc 1200

aaacgtatcg ctaacacctg tcaggacctg ggcctgatct gtagcgcgct gggtcagtcc 1260

gttatcctgt gcccgccgtt catcctgacc gaggcgcaaa tggatgagat gtttgacaaa 1320

ctggagaagg ctctggacaa agtctttgcg gaggtggcgt aa 1362

<210> 12
<211> 1362
<212> DNA
<213> Artificial Sequence

<220>
<223> Modification of Vibrio fluvialis enzyme

<400> 12
atgaataaac cacaaagctg ggaagcgcgt gctgaaactt actctctgta cggcttcact 60

gatatgccat ctctgcacga gcgtggtacc gtggttgtca cccacggcga gggcccatac 120

gtggtggacg tcaacggtcg ccgttacctg gacgcaaact ccggcctgta caatatggtt 180

gccggcttcg accacaaggg tctgatcgac gcagcaaagg cccagtacga acgcttcccg 240

ggttaccata gcttcttcgg tcgtatgtct gatcaaactg ttatgctgag cgagaaactg 300

gtagaggtgt ctccattcga cagcggtcgc gtgttctata ctaactccgg ctccgaggct 360

aacgatacta tggtgaaaat gctgtggttt ctgcacgccg cagagggcaa gccgcaaaaa 420

cgcaaaatcc tgactcgtca aaacgcatac cacggtgtaa ctgctgtttc cgcttccatg 480

acgggtctgc cgcacaactc tgtattcggc ctgccgctgc cgggtttcgt tcacctgacc 540

tgtccgcact attggcgtta cggcgaagaa ggtgaaaccg aagagcagtt tgttgctcgt 600

ctggcccgcg agctggagga aactatccaa cgtgaaggcg cggacacgat tgcgggcttc 660

tttgctgagc cggtcatggg cgcgggcggc gtaatcccgc cggcgaaagg ttacttccag 720

gcgatcctgc cgattctgcg taagtacgac atcccggtta tctctgatga agttatctgc 780

ggctttggtc gtaccggtaa tacttggggt tgcgttacct atgacttcac cccggatgcg 840

atcatctcca gcaaaaatct gaccgccggt ttctttccgg ttggtgctgt gattctgggt 900

ccggaactga gcaaacgcct ggaaacggcg atcgaagcta tcgaagagtt cccgcacggc 960

tttacggccg gcggtcaccc ggtgggttgc gctatcgctc tgaaagcaat cgatgttgtg 1020

atgaatgagg gtctggcaga gaacgtgcgc cgcctggcac cgcgttttga ggagcgtctg 1080

aaacacattg ccgaacgtcc gaacatcggt gaatatcgtg gcatcggttt tatgtgggca 1140

ctggaggctg tgaaagacaa agcatctaaa accccattcg atggtaatct gtctgtgagc 1200

aaacgtatcg ctaacacctg tcaggacctg ggcctgatct gtagcgcgct gggtcagtcc 1260

gttatcctga gcccgccgtt catcctgacc gaggcgcaaa tggatgagat gtttgacaaa 1320

ctggagaagg ctctggacaa agtctttgcg gaggtggcgt aa 1362

<210> 13
<211> 1362
<212> DNA
<213> Artificial Sequence

<220>
<223> Modification of Vibrio fluvialis enzyme

<400> 13
atgaataaac cacaaagctg ggaagcgcgt gctgaaactt actctctgta cggcttcact 60

gatatgccat ctctgcacga gcgtggtacc gtggttgtca cccacggcga gggcccatac 120

atcgtggacg tcaacggtcg ccgttacctg gacgcaaact ccggcctgta caatatggtt 180

gccggcttcg accacaaggg tctgatcgac gcagcaaagg cccagtacga acgcttcccg 240

ggttaccata gcttcttcgg tcgtatgtct gatcaaactg ttatgctgag cgagaaactg 300

gtagaggtgt ctccattcga cagcggtcgc gtgttctata ctaactccgg ctccgaggct 360

aacgatacta tggtgaaaat gctgtggttt ctgcacgccg cagagggcaa gccgcaaaaa 420

cgcaaaatcc tgactcgtca aaacgcatac cacggtgtaa ctgctgtttc cgcttccatg 480

acgggtatgc cgcacaactc tgtattcggc ctgccgctgc cgggtttcgt tcacctgacc 540

tgtccgcact attggcgtta cggcgaagaa ggtgaaaccg aagagcagtt tgttgctcgt 600

```
ctggcccgcg agctggagga aactatccaa cgtgaaggcg cggacacgat tgcgggcttc      660
tttgctgagc cggtcatggg cgcgggcggc gtaatcccgc cggcgaaagg ttacttccag      720
gcgatcctgc cgattctgcg taagtacgac atcccggtta tctctgatga agttatctgc      780
ggctttggtc gtaccggtaa tacttggggt tgcgttacct atgacttcac cccggatgcg      840
atcatctcca gcaaaaatct gaccgccggt ttctttccgg ttggtgctgt gattctgggt      900
ccggcactga gcaaacgcct ggaaacggcg atcgaagcta tcgaagagtt cccgcacggc      960
tttacggccg gcggtcaccc ggtgggttgc gctatcgctc tgaaagcaat cgatgttgtg     1020
atgaatgagg gtctggcaga gaacgtgcgc cgcctggcac cgcgttttga ggagcgtctg     1080
aaacacattg ccgaacgtcc gaacatcggt gaatatcgtg gcatcggttt tatgtgggca     1140
ctggaggctg tgaaagacaa agcatctaaa accccattcg atggtaatct gtctgtgagc     1200
aaacgtatcg ctaacacctg tcaggacctg ggcctgatct gtagcgcgat gggtcagtcc     1260
gttatcctga gcccgccgtt catcctgacc gaggcgcaaa tggatgagat gtttgacaaa     1320
ctggagaagg ctctggacaa agtctttgcg gaggtggcgt aa                        1362
```

```
<210>  14
<211>  1362
<212>  DNA
<213>  Artificial Sequence

<220>
<223>  Modification of Vibrio fluvialis enzyme

<400>  14
atgaataaac cacaaagctg ggaagcgcgt gctgaaactt actctctgta cggcttcact       60
gatatgccat ctctgcacga gcgtggtacc gtggttgtca cccacggcga gggcccatac      120
gtggtggacg tcaacggtcg ccgttacctg gacgcaaact ccggcctgta caatatggtt      180
gccggcttcg accacaaggg tctgatcgac gcagcaaagg cccagtacga acgcttcccg      240
ggttaccata gcttcttcgg tcgtatgtct gatcaaactg ttatgctgag cgagaaactg      300
gtagaggtgt ctccattcga cagcggtcgc gtgttctata ctaactccgg ctccgaggct      360
aacgatacta tggtgaaaat gctgtggttt ctgcacgccg cagagggcaa gccgcaaaaa      420
cgcaaaatcc tgactcgtca aaacgcatac cacggtgtaa ctgctgtttc cgcttccatg      480
acgggtctgc cgcacaactc tgtattcggc ctgccgctgc cgggtttcgt tcacctgggt      540
tgtccgcact attggcgtta cggcgaagaa ggtgaaaccg aagagcagtt tgttgctcgt      600
ctggcccgcg agctggagga aactatccaa cgtgaaggcg cggacacgat tgcgggcttc      660
tttgctgagc cggtcatggg cgcgggcggc gtaatcccgc cggcgaaagg ttacttccag      720
gcgatcctgc cgattctgcg taagtacgac atcccggtta tctctgatga agttatctgc      780
```

ggctttggtc gtaccggtaa tacttggggt tgcgttacct atgacttcac cccggatgcg 840

atcatctcca gcaaaaatct gaccgccggt ttctttccgg ttggtgctgt gattctgggt 900

ccggaactga gcaaacgcct ggaaacggcg atcgaagcta tcgaagagtt cccgcacggc 960

tttacggccg gcggtcaccc ggtgggttgc gctatcgctc tgaaagcaat cgatgttgtg 1020

atgaatgagg gtctggcaga gaacgtgcgc cgcctggcac cgcgttttga ggagcgtctg 1080

aaacacattg ccgaacgtcc gaacatcggt gaatatcgtg gcatcggttt tatgtgggca 1140

ctggaggctg tgaaagacaa agcatctaaa accccattcg atggtaatct gtctgtgagc 1200

aaacgtatcg ctaacacctg tcaggacctg ggcctgatct gtagcgcgat gggtcagtcc 1260

gttatcctga gcccgccgtt catcctgacc gaggcgcaaa tggatgagat gtttgacaaa 1320

ctggagaagg ctctggacaa agtctttgcg gaggtggcgt aa 1362

<210> 15
<211> 1362
<212> DNA
<213> Artificial Sequence

<220>
<223> Modification of Vibrio fluvialis enzyme

<400> 15
atgaataaac cacaaagctg ggaagcgcgt gctgaaactt actctctgta cggcttcact 60

gatatgccat ctctgcacga gcgtggtacc gtggttgtca cccacggcga gggcccatac 120

atcgtggacg tccacggtcg ccgttacctg gacgcaaact ccggcctgta caatatggtt 180

gccggcttcg accacaaggg tctgatcgac gcagcaaagg cccagtacga acgcttcccg 240

ggttaccata gcttcttcgg tcgtatgtct gatcaaactg ttatgctgag cgagaaactg 300

gtagaggtgt ctccattcga cagcggtcgc gtgttctata ctaactccgg ctccgaggct 360

aacgatacta tggtgaaaat gctgtggttt ctgcacgccg cagagggcaa gccgcaaaaa 420

cgcaaaatcc tgactcgtca aaacgcatac cacggtgtaa ctgctgtttc cgcttccatg 480

acgggtctgc cgcacaactc tgtattcggc ctgccgctgc cgggtttcgt tcacctgagc 540

tgtccgcact attggcgtta cggcgaagaa ggtgaaaccg aagagcagtt tgttgctcgt 600

ctggcccgcg agctggagga aactatccaa cgtgaaggcg cggacacgat tgcgggcttc 660

tttgctgagc cggtcatggg cgcgggcggc gtaatcccgc cggcgaaagg ttacttccag 720

gcgatcctgc cgattctgcg taagtacgac atcccggtta tctctgatga agttatctgc 780

ggctttggtc gtaccggtaa tacttggggt tgcgttacct atgacttcac cccggatgcg 840

atcatctcca gcaaaaatct gaccgccggt ttctttccgg ttggtgctgt gattctgggt 900

ccggaactga gcaaacgcct ggaaacggcg atcgaagcta tcgaagagtt cccgcacggc 960

tttacggccg gcggtcaccc ggtgggttgc gctatcgctc tgaaagcaat cgatgttgtg 1020

```
atgaatgagg gtctggcaga gaacgtgcgc cgcctggcac cgcgttttga ggagcgtctg     1080
aaacacattg ccgaacgtcc gaacatcggt gaatatcgtg gcatcggttt tatgtgggca     1140
ctggaggctg tgaaagacaa agcatctaaa accccattcg atggtaatct gtctgtgagc     1200
aaacgtatcg ctaacacctg tcaggacctg ggcctgatct gtagcgcgat gggtcagtcc     1260
gttatcctga gcccgccgtt catcctgacc gaggcgcaaa tggatgagat gtttgacaaa     1320
ctggagaagg ctctggacaa agtctttgcg gaggtggcgt aa                        1362
```